# EUROPÄISCHE PATENTSCHRIFT

(11) **EP 1 824 968 B1**
(45) Veröffentlichungstag und Bekanntmachung des Hinweises auf die Patenterteilung: **05.01.2011**
(21) Anmeldenummer: 05807424.6
(22) Anmeldetag: 21.11.2005
(51) Int. Cl.: C12N 9/04, C12P 13/08

(54) **ALLELE DES GND-GENS AUS CORYNEFORMEN BAKTERIEN**
ALLELES OF THE GND-GENE FROM CORYNEFORM BACTERIA
ALLELES DU GENE GND DE BACTERIES CORYNEFORMES

(30) Priorität: 18.12.2004 DE 102004061015; 12.07.2005 DE 102005032426
(43) Veröffentlichungstag der Anmeldung: 29.08.2007
(73) Patentinhaber: Evonik Degussa GmbH, 45128 Essen (DE)
(72) Erfinder: THIERBACH, Georg, 33613 Bielefeld (DE); BATHE, Brigitte, 33154 Salzkotten (DE); SCHISCHKA, Natalie, 33649 Bielefeld (DE)
(86) Internationale Anmeldenummer: PCT/EP2005/012417
(87) Internationale Veröffentlichungsnummer: WO 2006/063660

(56) Entgegenhaltungen:
- EP-A- 1 462 516
- WO-A-01/71012
- NISHIO Y ET AL: "Comparative Complete Genome Sequence Analysis of the Amino Acid Replacements Responsible for the Thermostability of Corynebacterium efficiens" GENOME RESEARCH, COLD SPRING HARBOR LABORATORY PRESS, WOODBURY, NY, US, Bd. 13, 2003, Seiten 1572-1579, XP002995065 ISSN: 1088-9051 -& DATABASE UniProt 1. März 2003 (2003-03-01), XP002375536 Database accession no. Q8FTI1
- CERDEÑO-TÁRRAGA A M ET AL: "The complete genome sequence and analysis of Corynebacterium diphtheriae NCTC13129." NUCLEIC ACIDS RESEARCH. 15 NOV 2003, Bd. 31, Nr. 22, 15. November 2003 (2003-11-15), Seiten 6516-6523, XP002375534 ISSN: 1362-4962 -& DATABASE UniProt 5. Juli 2004 (2004-07-05), XP002375537 Database accession no. Q6NHC5

### SEQUENCE LISTING

<110> Degussa AG
<120> Allele des gnd-Gens aus coryneformen Bakterien
<130> 040341BT
<160> 22
<170> PatentIn version 3.3
<210> 1
   <211> 1476
   <212> DNA
   <213> Corynebacterium glutamicum
<220>
   <221> CDS
   <222> (1)..(1476)
   <223> gnd wild-type gene
<400> 1
   atg ccg tca agt acg atc aat aac atg act aat gga gat aat ctc gca      48
   Met Pro Ser Ser Thr Ile Asn Asn Met Thr Asn Gly Asp Asn Leu Ala
   1               5                   10                  15
   cag atc ggc gtt gta ggc cta gca gta atg ggc tca aac ctc gcc cgc      96
   Gln Ile Gly Val Val Gly Leu Ala Val Met Gly Ser Asn Leu Ala Arg
               20                  25                  30
   aac ttc gcc cgc aac ggc aac act gtc gct gtc tac aac cgc agc act     144
   Asn Phe Ala Arg Asn Gly Asn Thr Val Ala Val Tyr Asn Arg Ser Thr
           35                  40                  45
   gac aaa acc gac aag ctc atc gcc gat cac ggc tcc gaa ggc aac ttc     192
   Asp Lys Thr Asp Lys Leu Ile Ala Asp His Gly Ser Glu Gly Asn Phe
       50                  55                  60
   atc cct tct gca acc gtc gaa gag ttc gta gca tcc ctg gaa aag cca     240
   Ile Pro Ser Ala Thr Val Glu Glu Phe Val Ala Ser Leu Glu Lys Pro
   65                  70                  75                  80
   cgc cgc gcc atc atc atg gtt cag gct ggt aac gcc acc gac gca gtc     288
   Arg Arg Ala Ile Ile Met Val Gln Ala Gly Asn Ala Thr Asp Ala Val
                   85                  90                  95
   atc aac cag ctg gca gat gcc atg gac gaa ggc gac atc atc atc gac     336
   Ile Asn Gln Leu Ala Asp Ala Met Asp Glu Gly Asp Ile Ile Ile Asp
               100                 105                 110
   ggc ggc aac gcc ctc tac acc gac acc att cgt cgc gag aag gaa atc     384
   Gly Gly Asn Ala Leu Tyr Thr Asp Thr Ile Arg Arg Glu Lys Glu Ile
           115                 120                 125
   tcc gca cgc ggt ctc cac ttc gtc ggt gct ggt atc tcc ggc ggc gaa     432
   Ser Ala Arg Gly Leu His Phe Val Gly Ala Gly Ile Ser Gly Gly Glu
       130                 135                 140
   gaa ggc gca ctc aac ggc cca tcc atc atg cct ggt ggc cca gca aag     480
   Glu Gly Ala Leu Asn Gly Pro Ser Ile Met Pro Gly Gly Pro Ala Lys
   145                 150                 155                 160
   tcc tac gag tcc ctc gga cca ctg ctt gag tcc atc gct gcc aac gtt      528
   Ser Tyr Glu Ser Leu Gly Pro Leu Leu Glu Ser Ile Ala Ala Asn Val
                   165                 170                 175
   gac ggc acc cca tgt gtc acc cac atc ggc cca gac ggc gcc ggc cac      576
   Asp Gly Thr Pro Cys Val Thr His Ile Gly Pro Asp Gly Ala Gly His
               180                 185                 190
   ttc gtc aag atg gtc cac aac ggc atc gag tac gcc gac atg cag gtc      624
   Phe Val Lys Met Val His Asn Gly Ile Glu Tyr Ala Asp Met Gln Val
           195                 200                 205
   atc ggc gag gca tac cac ctt ctc cgc tac gca gca ggc atg cag cca      672
   Ile Gly Glu Ala Tyr His Leu Leu Arg Tyr Ala Ala Gly Met Gln Pro
       210                 215                 220
   gct gaa atc gct gag gtt ttc aag gaa tgg aac gca ggc gac ctg gat      720
   Ala Glu Ile Ala Glu Val Phe Lys Glu Trp Asn Ala Gly Asp Leu Asp
   225                 230                 235                 240
   tcc tac ctc atc gaa atc acc gca gag gtt ctc tcc cag gtg gat gct      768
   Ser Tyr Leu Ile Glu Ile Thr Ala Glu Val Leu Ser Gln Val Asp Ala
                   245                 250                 255
   gaa acc ggc aag cca cta atc gac gtc atc gtt gac gct gca ggt cag      816
   Glu Thr Gly Lys Pro Leu Ile Asp Val Ile Val Asp Ala Ala Gly Gln
               260                 265                 270
   aag ggc acc gga cgt tgg acc gtc aag gct gct ctt gat ctg ggt att      864
   Lys Gly Thr Gly Arg Trp Thr Val Lys Ala Ala Leu Asp Leu Gly Ile
           275                 280                 285
   gct acc acc ggc atc ggc gaa gct gtt ttc gca cgt gca ctc tcc ggc      912
   Ala Thr Thr Gly Ile Gly Glu Ala Val Phe Ala Arg Ala Leu Ser Gly
       290                 295                 300
   gca acc agc cag cgc gct gca gca cag ggc aac cta cct gca ggt gtc      960
   Ala Thr Ser Gln Arg Ala Ala Ala Gln Gly Asn Leu Pro Ala Gly Val
   305                 310                 315                 320
   ctc acc gat ctg gaa gca ctt ggc gtg gac aag gca cag ttc gtc gaa     1008
   Leu Thr Asp Leu Glu Ala Leu Gly Val Asp Lys Ala Gln Phe Val Glu
                   325                 330                 335
   gac gtt cgc cgt gca ctg tac gca tcc aag ctt gtt gct tac gca cag     1056
   Asp Val Arg Arg Ala Leu Tyr Ala Ser Lys Leu Val Ala Tyr Ala Gln
               340                 345                 350
   ggc ttc gac gag atc aag gct ggc tcc gac gag aac aac tgg gac gtt     1104
   Gly Phe Asp Glu Ile Lys Ala Gly Ser Asp Glu Asn Asn Trp Asp Val
           355                 360                 365
   gac cct cgc gac ctc gct acc atc tgg cgc ggc ggc tgc atc att cgc     1152
   Asp Pro Arg Asp Leu Ala Thr Ile Trp Arg Gly Gly Cys Ile Ile Arg
       370                 375                 380
   gct aag ttc ctc aac cgc atc gtc gaa gca tac gat gca aac gct gaa     1200
   Ala Lys Phe Leu Asn Arg Ile Val Glu Ala Tyr Asp Ala Asn Ala Glu
   385                 390                 395                 400
   ctt gag tcc ctg ctg ctc gat cct tac ttc aag agc gag ctc ggc gac     1248
   Leu Glu Ser Leu Leu Leu Asp Pro Tyr Phe Lys Ser Glu Leu Gly Asp
                     405                 410                 415
   ctc atc gat tca tgg cgt cgc gtg att gtc acc gcc acc cag ctt ggc      1296
   Leu Ile Asp Ser Trp Arg Arg Val Ile Val Thr Ala Thr Gln Leu Gly
               420                 425                 430
   ctg cca atc cca gtg ttc gct tcc tcc ctg tcc tac tac gac agc ctg      1344
   Leu Pro Ile Pro Val Phe Ala Ser Ser Leu Ser Tyr Tyr Asp Ser Leu
           435                 440                 445
   cgt gca gag cgt ctg cca gca gcc ctg atc caa gga cag cgc gac ttc      1392
   Arg Ala Glu Arg Leu Pro Ala Ala Leu Ile Gln Gly Gln Arg Asp Phe
       450                 455                 460
   ttc ggt gcg cac acc tac aag cgc atc gac aag gat ggc tcc ttc cac      1440
   Phe Gly Ala His Thr Tyr Lys Arg Ile Asp Lys Asp Gly Ser Phe His
   465                 470                 475                 480
   acc gag tgg tcc ggc gac cgc tcc gag gtt gaa gct                      1476
   Thr Glu Trp Ser Gly Asp Arg Ser Glu Val Glu Ala
                   485                 490
<210> 2
   <211> 492
   <212> PRT
   <213> Corynebacterium glutamicum
<400> 2
   Met Pro Ser Ser Thr Ile Asn Asn Met Thr Asn Gly Asp Asn Leu Ala
   1               5                   10                  15
   Gln Ile Gly Val Val Gly Leu Ala Val Met Gly Ser Asn Leu Ala Arg
               20                  25                  30
   Asn Phe Ala Arg Asn Gly Asn Thr Val Ala Val Tyr Asn Arg Ser Thr
           35                  40                  45
   Asp Lys Thr Asp Lys Leu Ile Ala Asp His Gly Ser Glu Gly Asn Phe
       50                  55                  60
   Ile Pro Ser Ala Thr Val Glu Glu Phe Val Ala Ser Leu Glu Lys Pro
   65                  70                  75                  80
   Arg Arg Ala Ile Ile Met Val Gln Ala Gly Asn Ala Thr Asp Ala Val
                   85                  90                  95
   Ile Asn Gln Leu Ala Asp Ala Met Asp Glu Gly Asp Ile Ile Ile Asp
               100                 105                 110
   Gly Gly Asn Ala Leu Tyr Thr Asp Thr Ile Arg Arg Glu Lys Glu Ile
           115                 120                 125
   Ser Ala Arg Gly Leu His Phe Val Gly Ala Gly Ile Ser Gly Gly Glu
   130 135 140
   Glu Gly Ala Leu Asn Gly Pro Ser Ile Met Pro Gly Gly Pro Ala Lys
   145 150 155 160
   Ser Tyr Glu Ser Leu Gly Pro Leu Leu Glu Ser Ile Ala Ala Asn Val
   165 170 175
   Asp Gly Thr Pro Cys Val Thr His Ile Gly Pro Asp Gly Ala Gly His
   180 185 190
   Phe Val Lys Met Val His Asn Gly Ile Glu Tyr Ala Asp Met Gln Val
   195 200 205
   Ile Gly Glu Ala Tyr His Leu Leu Arg Tyr Ala Ala Gly Met Gln Pro
   210 215 220
   Ala Glu Ile Ala Glu Val Phe Lys Glu Trp Asn Ala Gly Asp Leu Asp
   225 230 235 240
   Ser Tyr Leu Ile Glu Ile Thr Ala Glu Val Leu Ser Gln Val Asp Ala
   245 250 255
   Glu Thr Gly Lys Pro Leu Ile Asp Val Ile Val Asp Ala Ala Gly Gln
   260 265 270
   Lys Gly Thr Gly Arg Trp Thr Val Lys Ala Ala Leu Asp Leu Gly Ile
   275 280 285
   Ala Thr Thr Gly Ile Gly Glu Ala Val Phe Ala Arg Ala Leu Ser Gly
   290 295 300
   Ala Thr Ser Gln Arg Ala Ala Ala Gln Gly Asn Leu Pro Ala Gly Val
   305 310 315 320
   Leu Thr Asp Leu Glu Ala Leu Gly Val Asp Lys Ala Gln Phe Val Glu
   325 330 335
   Asp Val Arg Arg Ala Leu Tyr Ala Ser Lys Leu Val Ala Tyr Ala Gln
   340 345 350
   Gly Phe Asp Glu Ile Lys Ala Gly Ser Asp Glu Asn Asn Trp Asp Val
   355 360 365
   Asp Pro Arg Asp Leu Ala Thr Ile Trp Arg Gly Gly Cys Ile Ile Arg
   370 375 380
   Ala Lys Phe Leu Asn Arg Ile Val Glu Ala Tyr Asp Ala Asn Ala Glu
   385                 390                 395                 400
   Leu Glu Ser Leu Leu Leu Asp Pro Tyr Phe Lys Ser Glu Leu Gly Asp
                   405                 410                 415
   Leu Ile Asp Ser Trp Arg Arg Val Ile Val Thr Ala Thr Gln Leu Gly
               420                 425                 430
   Leu Pro Ile Pro Val Phe Ala Ser Ser Leu Ser Tyr Tyr Asp Ser Leu
           435                 440                 445
   Arg Ala Glu Arg Leu Pro Ala Ala Leu Ile Gln Gly Gln Arg Asp Phe
       450                 455                 460
   Phe Gly Ala His Thr Tyr Lys Arg Ile Asp Lys Asp Gly Ser Phe His
   465                 470                 475                 480
   Thr Glu Trp Ser Gly Asp Arg Ser Glu Val Glu Ala
                   485                 490
<210> 3
   <211> 1866
   <212> DNA
   <213> Corynebacterium glutamicum
<220>
   <221> CDS
   <222> (227)..(1702)
   <223> gnd wild-type gene with upstream- (1-226) and downstream-region (1703-1866)
<400> 3
   ccataattgg cgttgttgag tgcttcaagt tcgtctgtgg ttaaagctct ggtggcggca      60
   agttctgcaa gcgaaagcag atcttggggt tgatcatcgc gggaagtcat aattaattac     120
   tctagtcggc ctaaaatggt tggattttca ccttctgtga cctggtaaaa tcgccactac     180
   ccccaaatgg tcacaccttt taggccgatt ttgctgacac cgggct atg ccg tca        235
                                                      Met Pro Ser
                                                      1
   agt acg atc aat aac atg act aat gga gat aat ctc gca cag atc ggc       283
   Ser Thr Ile Asn Asn Met Thr Asn Gly Asp Asn Leu Ala Gln Ile Gly
       5                   10                  15
   gtt gta ggc cta gca gta atg ggc tca aac ctc gcc cgc aac ttc gcc       331
   Val Val Gly Leu Ala Val Met Gly Ser Asn Leu Ala Arg Asn Phe Ala
   20                  25                  30                  35
   cgc aac ggc aac act gtc gct gtc tac aac cgc agc act gac aaa acc       379
   Arg Asn Gly Asn Thr Val Ala Val Tyr Asn Arg Ser Thr Asp Lys Thr
                   40                  45                  50
   gac aag ctc atc gcc gat cac ggc tcc gaa ggc aac ttc atc cct tct      427
   Asp Lys Leu Ile Ala Asp His Gly Ser Glu Gly Asn Phe Ile Pro Ser
                   55                  60                  65
   gca acc gtc gaa gag ttc gta gca tcc ctg gaa aag cca cgc cgc gcc      475
   Ala Thr Val Glu Glu Phe Val Ala Ser Leu Glu Lys Pro Arg Arg Ala
               70                  75                  80
   atc atc atg gtt cag gct ggt aac gcc acc gac gca gtc atc aac cag      523
   Ile Ile Met Val Gln Ala Gly Asn Ala Thr Asp Ala Val Ile Asn Gln
       85                  90                  95
   ctg gca gat gcc atg gac gaa ggc gac atc atc atc gac ggc ggc aac      571
   Leu Ala Asp Ala Met Asp Glu Gly Asp Ile Ile Ile Asp Gly Gly Asn
   100                 105                 110                 115
   gcc ctc tac acc gac acc att cgt cgc gag aag gaa atc tcc gca cgc      619
   Ala Leu Tyr Thr Asp Thr Ile Arg Arg Glu Lys Glu Ile Ser Ala Arg
                   120                 125                 130
   ggt ctc cac ttc gtc ggt gct ggt atc tcc ggc ggc gaa gaa ggc gca      667
   Gly Leu His Phe Val Gly Ala Gly Ile Ser Gly Gly Glu Glu Gly Ala
               135                 140                 145
   ctc aac ggc cca tcc atc atg cct ggt ggc cca gca aag tcc tac gag      715
   Leu Asn Gly Pro Ser Ile Met Pro Gly Gly Pro Ala Lys Ser Tyr Glu
           150                 155                 160
   tcc ctc gga cca ctg ctt gag tcc atc gct gcc aac gtt gac ggc acc      763
   Ser Leu Gly Pro Leu Leu Glu Ser Ile Ala Ala Asn Val Asp Gly Thr
       165                 170                 175
   cca tgt gtc acc cac atc ggc cca gac ggc gcc ggc cac ttc gtc aag      811
   Pro Cys Val Thr His Ile Gly Pro Asp Gly Ala Gly His Phe Val Lys
   180                 185                 190                 195
   atg gtc cac aac ggc atc gag tac gcc gac atg cag gtc atc ggc gag      859
   Met Val His Asn Gly Ile Glu Tyr Ala Asp Met Gln Val Ile Gly Glu
                   200                 205                 210
   gca tac cac ctt ctc cgc tac gca gca ggc atg cag cca gct gaa atc      907
   Ala Tyr His Leu Leu Arg Tyr Ala Ala Gly Met Gln Pro Ala Glu Ile
               215                 220                 225
   gct gag gtt ttc aag gaa tgg aac gca ggc gac ctg gat tcc tac ctc      955
   Ala Glu Val Phe Lys Glu Trp Asn Ala Gly Asp Leu Asp Ser Tyr Leu
           230                 235                 240
   atc gaa atc acc gca gag gtt ctc tcc cag gtg gat gct gaa acc ggc     1003
   Ile Glu Ile Thr Ala Glu Val Leu Ser Gln Val Asp Ala Glu Thr Gly
       245                 250                 255
   aag cca cta atc gac gtc atc gtt gac gct gca ggt cag aag ggc acc     1051
   Lys Pro Leu Ile Asp Val Ile Val Asp Ala Ala Gly Gln Lys Gly Thr
   260                 265                 270                 275
   gga cgt tgg acc gtc aag gct gct ctt gat ctg ggt att gct acc acc     1099
   Gly Arg Trp Thr Val Lys Ala Ala Leu Asp Leu Gly Ile Ala Thr Thr
                   280                 285                 290
   ggc atc ggc gaa gct gtt ttc gca cgt gca ctc tcc ggc gca acc agc     1147
   Gly Ile Gly Glu Ala Val Phe Ala Arg Ala Leu Ser Gly Ala Thr Ser
               295                 300                 305
   cag cgc gct gca gca cag ggc aac cta cct gca ggt gtc ctc acc gat     1195
   Gln Arg Ala Ala Ala Gln Gly Asn Leu Pro Ala Gly Val Leu Thr Asp
           310                 315                 320
   ctg gaa gca ctt ggc gtg gac aag gca cag ttc gtc gaa gac gtt cgc     1243
   Leu Glu Ala Leu Gly Val Asp Lys Ala Gln Phe Val Glu Asp Val Arg
       325                 330                 335
   cgt gca ctg tac gca tcc aag ctt gtt gct tac gca cag ggc ttc gac     1291
   Arg Ala Leu Tyr Ala Ser Lys Leu Val Ala Tyr Ala Gln Gly Phe Asp
   340                 345                 350                 355
   gag atc aag gct ggc tcc gac gag aac aac tgg gac gtt gac cct cgc     1339
   Glu Ile Lys Ala Gly Ser Asp Glu Asn Asn Trp Asp Val Asp Pro Arg
                   360                 365                 370
   gac ctc gct acc atc tgg cgc ggc ggc tgc atc att cgc gct aag ttc     1387
   Asp Leu Ala Thr Ile Trp Arg Gly Gly Cys Ile Ile Arg Ala Lys Phe
               375                 380                 385
   ctc aac cgc atc gtc gaa gca tac gat gca aac gct gaa ctt gag tcc     1435
   Leu Asn Arg Ile Val Glu Ala Tyr Asp Ala Asn Ala Glu Leu Glu Ser
           390                 395                 400
   ctg ctg ctc gat cct tac ttc aag agc gag ctc ggc gac ctc atc gat     1483
   Leu Leu Leu Asp Pro Tyr Phe Lys Ser Glu Leu Gly Asp Leu Ile Asp
       405                 410                 415
   tca tgg cgt cgc gtg att gtc acc gcc acc cag ctt ggc ctg cca atc     1531
   Ser Trp Arg Arg Val Ile Val Thr Ala Thr Gln Leu Gly Leu Pro Ile
   420                 425                 430                 435
   cca gtg ttc gct tcc tcc ctg tcc tac tac gac agc ctg cgt gca gag     1579
   Pro Val Phe Ala Ser Ser Leu Ser Tyr Tyr Asp Ser Leu Arg Ala Glu
                   440                 445                 450
   cgt ctg cca gca gcc ctg atc caa gga cag cgc gac ttc ttc ggt gcg     1627
   Arg Leu Pro Ala Ala Leu Ile Gln Gly Gln Arg Asp Phe Phe Gly Ala
               455                 460                 465
   cac acc tac aag cgc atc gac aag gat ggc tcc ttc cac acc gag tgg     1675
   His Thr Tyr Lys Arg Ile Asp Lys Asp Gly Ser Phe His Thr Glu Trp
           470                 475                 480
   tcc ggc gac cgc tcc gag gtt gaa gct taaaggctct ccttttaaca           1722
   Ser Gly Asp Arg Ser Glu Val Glu Ala
       485                 490
   caacgccaaa acccctcaca gtcaccttag attgtgaggg gtttttcgcg tgctgccagg   1782
   gattcgccgg aggtgggcgt cgataagcaa aaatctttta attgctttta cccatggctc   1842
   tgcccttgtt ccaataacct tgcg                                          1866
<210> 4
   <211> 492
   <212> PRT
   <213> Corynebacterium glutamicum
<400> 4
   Met Pro Ser Ser Thr Ile Asn Asn Met Thr Asn Gly Asp Asn Leu Ala
   1 5 10 15
   Gln Ile Gly Val Val Gly Leu Ala Val Met Gly Ser Asn Leu Ala Arg
   20 25 30
   Asn Phe Ala Arg Asn Gly Asn Thr Val Ala Val Tyr Asn Arg Ser Thr
   35 40 45
   Asp Lys Thr Asp Lys Leu Ile Ala Asp His Gly Ser Glu Gly Asn Phe
   50 55 60
   Ile Pro Ser Ala Thr Val Glu Glu Phe Val Ala Ser Leu Glu Lys Pro
   65 70 75 80
   Arg Arg Ala Ile Ile Met Val Gln Ala Gly Asn Ala Thr Asp Ala Val
   85 90 95
   Ile Asn Gln Leu Ala Asp Ala Met Asp Glu Gly Asp Ile Ile Ile Asp
   100 105 110
   Gly Gly Asn Ala Leu Tyr Thr Asp Thr Ile Arg Arg Glu Lys Glu Ile
   115 120 125
   Ser Ala Arg Gly Leu His Phe Val Gly Ala Gly Ile Ser Gly Gly Glu
   130 135 140
   Glu Gly Ala Leu Asn Gly Pro Ser Ile Met Pro Gly Gly Pro Ala Lys
   145 150 155 160
   Ser Tyr Glu Ser Leu Gly Pro Leu Leu Glu Ser Ile Ala Ala Asn Val
   165 170 175
   Asp Gly Thr Pro Cys Val Thr His Ile Gly Pro Asp Gly Ala Gly His
   180 185 190
   Phe Val Lys Met Val His Asn Gly Ile Glu Tyr Ala Asp Met Gln Val
   195 200 205
   Ile Gly Glu Ala Tyr His Leu Leu Arg Tyr Ala Ala Gly Met Gln Pro
   210 215 220
   Ala Glu Ile Ala Glu Val Phe Lys Glu Trp Asn Ala Gly Asp Leu Asp
   225 230 235 240
   Ser Tyr Leu Ile Glu Ile Thr Ala Glu Val Leu Ser Gln Val Asp Ala
   245 250 255
   Glu Thr Gly Lys Pro Leu Ile Asp Val Ile Val Asp Ala Ala Gly Gln
               260                 265                 270
   Lys Gly Thr Gly Arg Trp Thr Val Lys Ala Ala Leu Asp Leu Gly Ile
           275                 280                 285
   Ala Thr Thr Gly Ile Gly Glu Ala Val Phe Ala Arg Ala Leu Ser Gly
       290                 295                 300
   Ala Thr Ser Gln Arg Ala Ala Ala Gln Gly Asn Leu Pro Ala Gly Val
   305                 310                 315                 320
   Leu Thr Asp Leu Glu Ala Leu Gly Val Asp Lys Ala Gln Phe Val Glu
                   325                 330                 335
   Asp Val Arg Arg Ala Leu Tyr Ala Ser Lys Leu Val Ala Tyr Ala Gln
               340                 345                 350
   Gly Phe Asp Glu Ile Lys Ala Gly Ser Asp Glu Asn Asn Trp Asp Val
           355                 360                 365
   Asp Pro Arg Asp Leu Ala Thr Ile Trp Arg Gly Gly Cys Ile Ile Arg
       370                 375                 380
   Ala Lys Phe Leu Asn Arg Ile Val Glu Ala Tyr Asp Ala Asn Ala Glu
   385                 390                 395                 400
   Leu Glu Ser Leu Leu Leu Asp Pro Tyr Phe Lys Ser Glu Leu Gly Asp
                   405                 410                 415
   Leu Ile Asp Ser Trp Arg Arg Val Ile Val Thr Ala Thr Gln Leu Gly
               420                 425                 430
   Leu Pro Ile Pro Val Phe Ala Ser Ser Leu Ser Tyr Tyr Asp Ser Leu
           435                 440                 445
   Arg Ala Glu Arg Leu Pro Ala Ala Leu Ile Gln Gly Gln Arg Asp Phe
       450                 455                 460
   Phe Gly Ala His Thr Tyr Lys Arg Ile Asp Lys Asp Gly Ser Phe His
   465                 470                 475                 480
   Thr Glu Trp Ser Gly Asp Arg Ser Glu Val Glu Ala
                   485                 490
<210> 5
   <211> 1476
   <212> DNA
   <213> Corynebacterium glutamicum
<220>
<221> CDS
   <222> (1)..(1476)
   <223> gnd-allele
<220>
   <221> misc_feature
   <222> (985)..(985)
   <223> G-A Transition
<400> 5
   atg ccg tca agt acg atc aat aac atg act aat gga gat aat ctc gca       48
   Met Pro Ser Ser Thr Ile Asn Asn Met Thr Asn Gly Asp Asn Leu Ala
   1               5                   10                  15
   cag atc ggc gtt gta ggc cta gca gta atg ggc tca aac ctc gcc cgc       96
   Gln Ile Gly Val Val Gly Leu Ala Val Met Gly Ser Asn Leu Ala Arg
               20                  25                  30
   aac ttc gcc cgc aac ggc aac act gtc gct gtc tac aac cgc agc act      144
   Asn Phe Ala Arg Asn Gly Asn Thr Val Ala Val Tyr Asn Arg Ser Thr
           35                  40                  45
   gac aaa acc gac aag ctc atc gcc gat cac ggc tcc gaa ggc aac ttc      192
   Asp Lys Thr Asp Lys Leu Ile Ala Asp His Gly Ser Glu Gly Asn Phe
       50                  55                  60
   atc cct tct gca acc gtc gaa gag ttc gta gca tcc ctg gaa aag cca      240
   Ile Pro Ser Ala Thr Val Glu Glu Phe Val Ala Ser Leu Glu Lys Pro
   65                  70                  75                  80
   cgc cgc gcc atc atc atg gtt cag gct ggt aac gcc acc gac gca gtc      288
   Arg Arg Ala Ile Ile Met Val Gln Ala Gly Asn Ala Thr Asp Ala Val
                   85                  90                  95
   atc aac cag ctg gca gat gcc atg gac gaa ggc gac atc atc atc gac      336
   Ile Asn Gln Leu Ala Asp Ala Met Asp Glu Gly Asp Ile Ile Ile Asp
               100                 105                 110
   ggc ggc aac gcc ctc tac acc gac acc att cgt cgc gag aag gaa atc      384
   Gly Gly Asn Ala Leu Tyr Thr Asp Thr Ile Arg Arg Glu Lys Glu Ile
           115                 120                 125
   tcc gca cgc ggt ctc cac ttc gtc ggt gct ggt atc tcc ggc ggc gaa      432
   Ser Ala Arg Gly Leu His Phe Val Gly Ala Gly Ile Ser Gly Gly Glu
       130                 135                 140
   gaa ggc gca ctc aac ggc cca tcc atc atg cct ggt ggc cca gca aag      480
   Glu Gly Ala Leu Asn Gly Pro Ser Ile Met Pro Gly Gly Pro Ala Lys
   145                 150                 155                 160
   tcc tac gag tcc ctc gga cca ctg ctt gag tcc atc gct gcc aac gtt      528
   Ser Tyr Glu Ser Leu Gly Pro Leu Leu Glu Ser Ile Ala Ala Asn Val
                   165                 170                 175
   gac ggc acc cca tgt gtc acc cac atc ggc cca gac ggc gcc ggc cac      576
   Asp Gly Thr Pro Cys Val Thr His Ile Gly Pro Asp Gly Ala Gly His
               180                 185                 190
   ttc gtc aag atg gtc cac aac ggc atc gag tac gcc gac atg cag gtc      624
   Phe Val Lys Met Val His Asn Gly Ile Glu Tyr Ala Asp Met Gln Val
           195                 200                 205
   atc ggc gag gca tac cac ctt ctc cgc tac gca gca ggc atg cag cca      672
   Ile Gly Glu Ala Tyr His Leu Leu Arg Tyr Ala Ala Gly Met Gln Pro
       210                 215                 220
   gct gaa atc gct gag gtt ttc aag gaa tgg aac gca ggc gac ctg gat      720
   Ala Glu Ile Ala Glu Val Phe Lys Glu Trp Asn Ala Gly Asp Leu Asp
   225                 230                 235                 240
   tcc tac ctc atc gaa atc acc gca gag gtt ctc tcc cag gtg gat gct      768
   Ser Tyr Leu Ile Glu Ile Thr Ala Glu Val Leu Ser Gln Val Asp Ala
                   245                 250                 255
   gaa acc ggc aag cca cta atc gac gtc atc gtt gac gct gca ggt cag      816
   Glu Thr Gly Lys Pro Leu Ile Asp Val Ile Val Asp Ala Ala Gly Gln
               260                 265                 270
   aag ggc acc gga cgt tgg acc gtc aag gct gct ctt gat ctg ggt att      864
   Lys Gly Thr Gly Arg Trp Thr Val Lys Ala Ala Leu Asp Leu Gly Ile
           275                 280                 285
   gct acc acc ggc atc ggc gaa gct gtt ttc gca cgt gca ctc tcc ggc      912
   Ala Thr Thr Gly Ile Gly Glu Ala Val Phe Ala Arg Ala Leu Ser Gly
       290                 295                 300
   gca acc agc cag cgc gct gca gca cag ggc aac cta cct gca ggt gtc      960
   Ala Thr Ser Gln Arg Ala Ala Ala Gln Gly Asn Leu Pro Ala Gly Val
   305                 310                 315                 320
   ctc acc gat ctg gaa gca ctt ggc atg gac aag gca cag ttc gtc gaa     1008
   Leu Thr Asp Leu Glu Ala Leu Gly Met Asp Lys Ala Gln Phe Val Glu
                   325                 330                 335
   gac gtt cgc cgt gca ctg tac gca tcc aag ctt gtt gct tac gca cag     1056
   Asp Val Arg Arg Ala Leu Tyr Ala Ser Lys Leu Val Ala Tyr Ala Gln
               340                 345                 350
   ggc ttc gac gag atc aag gct ggc tcc gac gag aac aac tgg gac gtt     1104
   Gly Phe Asp Glu Ile Lys Ala Gly Ser Asp Glu Asn Asn Trp Asp Val
           355                 360                 365
   gac cct cgc gac ctc gct acc atc tgg cgc ggc ggc tgc atc att cgc     1152
   Asp Pro Arg Asp Leu Ala Thr Ile Trp Arg Gly Gly Cys Ile Ile Arg
       370                 375                 380
   gct aag ttc ctc aac cgc atc gtc gaa gca tac gat gca aac gct gaa     1200
   Ala Lys Phe Leu Asn Arg Ile Val Glu Ala Tyr Asp Ala Asn Ala Glu
   385                 390                 395                 400
   ctt gag tcc ctg ctg ctc gat cct tac ttc aag agc gag ctc ggc gac     1248
   Leu Glu Ser Leu Leu Leu Asp Pro Tyr Phe Lys Ser Glu Leu Gly Asp
                   405                 410                 415
   ctc atc gat tca tgg cgt cgc gtg att gtc acc gcc acc cag ctt ggc     1296
   Leu Ile Asp Ser Trp Arg Arg Val Ile Val Thr Ala Thr Gln Leu Gly
               420                 425                 430
   ctg cca atc cca gtg ttc gct tcc tcc ctg tcc tac tac gac agc ctg     1344
   Leu Pro Ile Pro Val Phe Ala Ser Ser Leu Ser Tyr Tyr Asp Ser Leu
           435                 440                 445
   cgt gca gag cgt ctg cca gca gcc ctg atc caa gga cag cgc gac ttc      1392
   Arg Ala Glu Arg Leu Pro Ala Ala Leu Ile Gln Gly Gln Arg Asp Phe
       450                 455                 460
   ttc ggt gcg cac acc tac aag cgc atc gac aag gat ggc tcc ttc cac      1440
   Phe Gly Ala His Thr Tyr Lys Arg Ile Asp Lys Asp Gly Ser Phe His
   465                 470                 475                 480
   acc gag tgg tcc ggc gac cgc tcc gag gtt gaa gct                      1476
   Thr Glu Trp Ser Gly Asp Arg Ser Glu Val Glu Ala
                   485                 490
<210> 6
   <211> 492
   <212> PRT
   <213> Corynebacterium glutamicum
<400> 6
   Met Pro Ser Ser Thr Ile Asn Asn Met Thr Asn Gly Asp Asn Leu Ala
   1               5                   10                  15
   Gln Ile Gly Val Val Gly Leu Ala Val Met Gly Ser Asn Leu Ala Arg
               20                  25                  30
   Asn Phe Ala Arg Asn Gly Asn Thr Val Ala Val Tyr Asn Arg Ser Thr
           35                  40                  45
   Asp Lys Thr Asp Lys Leu Ile Ala Asp His Gly Ser Glu Gly Asn Phe
       50                  55                  60
   Ile Pro Ser Ala Thr Val Glu Glu Phe Val Ala Ser Leu Glu Lys Pro
   65                  70                  75                  80
   Arg Arg Ala Ile Ile Met Val Gln Ala Gly Asn Ala Thr Asp Ala Val
                   85                  90                  95
   Ile Asn Gln Leu Ala Asp Ala Met Asp Glu Gly Asp Ile Ile Ile Asp
               100                 105                 110
   Gly Gly Asn Ala Leu Tyr Thr Asp Thr Ile Arg Arg Glu Lys Glu Ile
           115                 120                 125
   Ser Ala Arg Gly Leu His Phe Val Gly Ala Gly Ile Ser Gly Gly Glu
       130                 135                 140
   Glu Gly Ala Leu Asn Gly Pro Ser Ile Met Pro Gly Gly Pro Ala Lys
   145                 150                 155                 160
   Ser Tyr Glu Ser Leu Gly Pro Leu Leu Glu Ser Ile Ala Ala Asn Val
                   165                 170                 175
   Asp Gly Thr Pro Cys Val Thr His Ile Gly Pro Asp Gly Ala Gly His
               180                 185                 190
   Phe Val Lys Met Val His Asn Gly Ile Glu Tyr Ala Asp Met Gln Val
           195                 200                 205
   Ile Gly Glu Ala Tyr His Leu Leu Arg Tyr Ala Ala Gly Met Gln Pro
       210                 215                 220
   Ala Glu Ile Ala Glu Val Phe Lys Glu Trp Asn Ala Gly Asp Leu Asp
   225                 230                 235                 240
   Ser Tyr Leu Ile Glu Ile Thr Ala Glu Val Leu Ser Gln Val Asp Ala
                   245                 250                 255
   Glu Thr Gly Lys Pro Leu Ile Asp Val Ile Val Asp Ala Ala Gly Gln
               260                 265                 270
   Lys Gly Thr Gly Arg Trp Thr Val Lys Ala Ala Leu Asp Leu Gly Ile
           275                 280                 285
   Ala Thr Thr Gly Ile Gly Glu Ala Val Phe Ala Arg Ala Leu Ser Gly
       290                 295                 300
   Ala Thr Ser Gln Arg Ala Ala Ala Gln Gly Asn Leu Pro Ala Gly Val
   305                 310                 315                 320
   Leu Thr Asp Leu Glu Ala Leu Gly Met Asp Lys Ala Gln Phe Val Glu
                   325                 330                 335
   Asp Val Arg Arg Ala Leu Tyr Ala Ser Lys Leu Val Ala Tyr Ala Gln
               340                 345                 350
   Gly Phe Asp Glu Ile Lys Ala Gly Ser Asp Glu Asn Asn Trp Asp Val
           355                 360                 365
   Asp Pro Arg Asp Leu Ala Thr Ile Trp Arg Gly Gly Cys Ile Ile Arg
       370                 375                 380
   Ala Lys Phe Leu Asn Arg Ile Val Glu Ala Tyr Asp Ala Asn Ala Glu
   385                 390                 395                 400
   Leu Glu Ser Leu Leu Leu Asp Pro Tyr Phe Lys Ser Glu Leu Gly Asp
                   405                 410                 415
   Leu Ile Asp Ser Trp Arg Arg Val Ile Val Thr Ala Thr Gln Leu Gly
   420 425 430
   Leu Pro Ile Pro Val Phe Ala Ser Ser Leu Ser Tyr Tyr Asp Ser Leu
   435 440 445
   Arg Ala Glu Arg Leu Pro Ala Ala Leu Ile Gln Gly Gln Arg Asp Phe
   450 455 460
   Phe Gly Ala His Thr Tyr Lys Arg Ile Asp Lys Asp Gly Ser Phe His
   465 470 475 480
   Thr Glu Trp Ser Gly Asp Arg Ser Glu Val Glu Ala
   485 490
<210> 7
   <211> 20
   <212> DNA
   <213> Corynebacterium glutamicum
<220>
   <221> misc_feature
   <222> (1)..(20)
   <223> Primer gnd-A1
<400> 7
   ccataattgg cgttgttgag 20
<210> 8
   <211> 20
   <212> DNA
   <213> Corynebacterium glutamicum
<220>
   <221> misc_feature
   <222> (1)..(20)
   <223> Primer gnd-E1
<400> 8
   cgcaaggtta ttggaacaag 20
<210> 9
   <211> 18
   <212> DNA
   <213> Corynebacterium glutamicum
<220>
   <221> misc_feature
   <222> (1)..(18)
   <223> Primer gndFS-1
<400> 9
   gctatgccgt caagtacg 18
<210> 10
   <211> 20
   <212> DNA
   <213> Corynebacterium glutamicum
<220>
   <221> misc_feature
   <222> (1)..(20)
   <223> Primer gndFS-E
<400> 10
   ccctcacaat ctaaggtgac 20
<210> 11
   <211> 99
   <212> DNA
   <213> Corynebacterium glutamicum
<220>
   <221> CDS
   <222> (1)..(99)
   <223> Leseraster
<220>
   <221> mutation
   <222> (49)..(51)
   <223> Position 49 bis 51 entspricht Position 985 bis 987 von SEQ ID NO:1 oder SEQ ID NO:5
<220>
   <221> misc_feature
   <222> (49)..(51)
   <223> n is a, c, g, or t
<400> 11
   cag ggc aac cta cct gca ggt gtc ctc acc gat ctg gaa gca ctt ggc        48
   Gln Gly Asn Leu Pro Ala Gly Val Leu Thr Asp Leu Glu Ala Leu Gly
   1               5                   10                  15
   nnn gac aag gca cag ttc gtc gaa gac gtt cgc cgt gca ctg tac gca        96
   Xaa Asp Lys Ala Gln Phe Val Glu Asp Val Arg Arg Ala Leu Tyr Ala
               20                  25                  30
   tcc                                                                    99
   Ser
<210> 12
   <211> 33
   <212> PRT
   <213> Corynebacterium glutamicum
<220>
   <221> misc_feature
   <222> (17)..(17)
   <223> The 'Xaa' at location 17 stands for Lys, Asn, Arg, Ser, Thr, Ile, Met, Glu, Asp, Gly, Ala, Val, Gln, His, Pro, Leu, Tyr, Trp, Cys, or Phe.
<400> 12
   Gln Gly Asn Leu Pro Ala Gly Val Leu Thr Asp Leu Glu Ala Leu Gly
   1 5 10 15
   Xaa Asp Lys Ala Gln Phe Val Glu Asp Val Arg Arg Ala Leu Tyr Ala
   20 25 30
   Ser
<210> 13
   <211> 99
   <212> DNA
   <213> Corynebacterium glutamicum
<220>
   <221> CDS
   <222> (1)..(99)
   <223> Leseraster
<220>
   <221> mutation
   <222> (49)..(51)
   <223> Position 49 bis 51 entspricht Position 985 bis 987 von SEQ ID NO:5
<400> 13
   cag ggc aac cta cct gca ggt gtc ctc acc gat ctg gaa gca ctt ggc 48
   Gln Gly Asn Leu Pro Ala Gly Val Leu Thr Asp Leu Glu Ala Leu Gly
   1 5 10 15
   atg gac aag gca cag ttc gtc gaa gac gtt cgc cgt gca ctg tac gca 96
   Met Asp Lys Ala Gln Phe Val Glu Asp Val Arg Arg Ala Leu Tyr Ala
   20 25 30
   tcc 99
   Ser
<210> 14
   <211> 33
   <212> PRT
   <213> Corynebacterium glutamicum
<400> 14
   Gln Gly Asn Leu Pro Ala Gly Val Leu Thr Asp Leu Glu Ala Leu Gly
   1 5 10 15
   Met Asp Lys Ala Gln Phe Val Glu Asp Val Arg Arg Ala Leu Tyr Ala
   20 25 30
   Ser
<210> 15
   <211> 1263
   <212> DNA
   <213> Corynebacterium glutamicum
<220>
   <221> CDS
   <222> (1)..(1263)
   <223> lysC-Wildtypgen
<400> 15
   gtg gcc ctg gtc gta cag aaa tat ggc ggt tcc tcg ctt gag agt gcg      48
   Met Ala Leu Val Val Gln Lys Tyr Gly Gly Ser Ser Leu Glu Ser Ala
   1               5                   10                  15
   gaa cgc att aga aac gtc gct gaa cgg atc gtt gcc acc aag aag gct      96
   Glu Arg Ile Arg Asn Val Ala Glu Arg Ile Val Ala Thr Lys Lys Ala
               20                  25                  30
   gga aat gat gtc gtg gtt gtc tgc tcc gca atg gga gac acc acg gat     144
   Gly Asn Asp Val Val Val Val Cys Ser Ala Met Gly Asp Thr Thr Asp
           35                  40                  45
   gaa ctt cta gaa ctt gca gcg gca gtg aat ccc gtt ccg cca gct cgt     192
   Glu Leu Leu Glu Leu Ala Ala Ala Val Asn Pro Val Pro Pro Ala Arg
       50                  55                  60
   gaa atg gat atg ctc ctg act gct ggt gag cgt att tct aac gct ctc     240
   Glu Met Asp Met Leu Leu Thr Ala Gly Glu Arg Ile Ser Asn Ala Leu
   65                  70                  75                  80
   gtc gcc atg gct att gag tcc ctt ggc gca gaa gcc caa tct ttc acg     288
   Val Ala Met Ala Ile Glu Ser Leu Gly Ala Glu Ala Gln Ser Phe Thr
                   85                  90                  95
   ggc tct cag gct ggt gtg ctc acc acc gag cgc cac gga aac gca cgc     336
   Gly Ser Gln Ala Gly Val Leu Thr Thr Glu Arg His Gly Asn Ala Arg
               100                 105                 110
   att gtt gat gtc act cca ggt cgt gtg cgt gaa gca ctc gat gag ggc     384
   Ile Val Asp Val Thr Pro Gly Arg Val Arg Glu Ala Leu Asp Glu Gly
           115                 120                 125
   aag atc tgc att gtt gct ggt ttc cag ggt gtt aat aaa gaa acc cgc     432
   Lys Ile Cys Ile Val Ala Gly Phe Gln Gly Val Asn Lys Glu Thr Arg
       130                 135                 140
   gat gtc acc acg ttg ggt cgt ggt ggt tct gac acc act gca gtt gcg     480
   Asp Val Thr Thr Leu Gly Arg Gly Gly Ser Asp Thr Thr Ala Val Ala
   145                 150                 155                 160
   ttg gca gct gct ttg aac gct gat gtg tgt gag att tac tcg gac gtt     528
   Leu Ala Ala Ala Leu Asn Ala Asp Val Cys Glu Ile Tyr Ser Asp Val
                   165                 170                 175
   gac ggt gtg tat acc gct gac ccg cgc atc gtt cct aat gca cag aag     576
   Asp Gly Val Tyr Thr Ala Asp Pro Arg Ile Val Pro Asn Ala Gln Lys
               180                 185                 190
   ctg gaa aag ctc agc ttc gaa gaa atg ctg gaa ctt gct gct gtt ggc     624
   Leu Glu Lys Leu Ser Phe Glu Glu Met Leu Glu Leu Ala Ala Val Gly
           195                 200                 205
   tcc aag att ttg gtg ctg cgc agt gtt gaa tac gct cgt gca ttc aat      672
   Ser Lys Ile Leu Val Leu Arg Ser Val Glu Tyr Ala Arg Ala Phe Asn
       210                 215                 220
   gtg cca ctt cgc gta cgc tcg tct tat agt aat gat ccc ggc act ttg      720
   Val Pro Leu Arg Val Arg Ser Ser Tyr Ser Asn Asp Pro Gly Thr Leu
   225                 230                 235                 240
   att gcc ggc tct atg gag gat att cct gtg gaa gaa gca gtc ctt acc      768
   Ile Ala Gly Ser Met Glu Asp Ile Pro Val Glu Glu Ala Val Leu Thr
                   245                 250                 255
   ggt gtc gca acc gac aag tcc gaa gcc aaa gta acc gtt ctg ggt att      816
   Gly Val Ala Thr Asp Lys Ser Glu Ala Lys Val Thr Val Leu Gly Ile
               260                 265                 270
   tcc gat aag cca ggc gag gct gcg aag gtt ttc cgt gcg ttg gct gat      864
   Ser Asp Lys Pro Gly Glu Ala Ala Lys Val Phe Arg Ala Leu Ala Asp
           275                 280                 285
   gca gaa atc aac att gac atg gtt ctg cag aac gtc tct tct gta gaa      912
   Ala Glu Ile Asn Ile Asp Met Val Leu Gln Asn Val Ser Ser Val Glu
       290                 295                 300
   gac ggc acc acc gac atc acc ttc acc tgc cct cgt tcc gac ggc cgc      960
   Asp Gly Thr Thr Asp Ile Thr Phe Thr Cys Pro Arg Ser Asp Gly Arg
   305                 310                 315                 320
   cgc gcg atg gag atc ttg aag aag ctt cag gtt cag ggc aac tgg acc     1008
   Arg Ala Met Glu Ile Leu Lys Lys Leu Gln Val Gln Gly Asn Trp Thr
                   325                 330                 335
   aat gtg ctt tac gac gac cag gtc ggc aaa gtc tcc ctc gtg ggt gct     1056
   Asn Val Leu Tyr Asp Asp Gln Val Gly Lys Val Ser Leu Val Gly Ala
               340                 345                 350
   ggc atg aag tct cac cca ggt gtt acc gca gag ttc atg gaa gct ctg     1104
   Gly Met Lys Ser His Pro Gly Val Thr Ala Glu Phe Met Glu Ala Leu
           355                 360                 365
   cgc gat gtc aac gtg aac atc gaa ttg att tcc acc tct gag att cgt     1152
   Arg Asp Val Asn Val Asn Ile Glu Leu Ile Ser Thr Ser Glu Ile Arg
       370                 375                 380
   att tcc gtg ctg atc cgt gaa gat gat ctg gat gct gct gca cgt gca     1200
   Ile Ser Val Leu Ile Arg Glu Asp Asp Leu Asp Ala Ala Ala Arg Ala
   385                 390                 395                 400
   ttg cat gag cag ttc cag ctg ggc ggc gaa gac gaa gcc gtc gtt tat     1248
   Leu His Glu Gln Phe Gln Leu Gly Gly Glu Asp Glu Ala Val Val Tyr
                   405                 410                 415
   gca ggc acc gga cgc                                                 1263
   Ala Gly Thr Gly Arg
                   420
<210> 16
   <211> 421
   <212> PRT
   <213> Corynebacterium glutamicum
<400> 16
   Met Ala Leu Val Val Gln Lys Tyr Gly Gly Ser Ser Leu Glu Ser Ala
   1               5                   10                  15
   Glu Arg Ile Arg Asn Val Ala Glu Arg Ile Val Ala Thr Lys Lys Ala
               20                  25                  30
   Gly Asn Asp Val Val Val Val Cys Ser Ala Met Gly Asp Thr Thr Asp
           35                  40                  45
   Glu Leu Leu Glu Leu Ala Ala Ala Val Asn Pro Val Pro Pro Ala Arg
       50                  55                  60
   Glu Met Asp Met Leu Leu Thr Ala Gly Glu Arg Ile Ser Asn Ala Leu
   65                  70                  75                  80
   Val Ala Met Ala Ile Glu Ser Leu Gly Ala Glu Ala Gln Ser Phe Thr
                   85                  90                  95
   Gly Ser Gln Ala Gly Val Leu Thr Thr Glu Arg His Gly Asn Ala Arg
               100                 105                 110
   Ile Val Asp Val Thr Pro Gly Arg Val Arg Glu Ala Leu Asp Glu Gly
           115                 120                 125
   Lys Ile Cys Ile Val Ala Gly Phe Gln Gly Val Asn Lys Glu Thr Arg
       130                 135                 140
   Asp Val Thr Thr Leu Gly Arg Gly Gly Ser Asp Thr Thr Ala Val Ala
   145                 150                 155                 160
   Leu Ala Ala Ala Leu Asn Ala Asp Val Cys Glu Ile Tyr Ser Asp Val
                   165                 170                 175
   Asp Gly Val Tyr Thr Ala Asp Pro Arg Ile Val Pro Asn Ala Gln Lys
               180                 185                 190
   Leu Glu Lys Leu Ser Phe Glu Glu Met Leu Glu Leu Ala Ala Val Gly
           195                 200                 205
   Ser Lys Ile Leu Val Leu Arg Ser Val Glu Tyr Ala Arg Ala Phe Asn
       210                 215                 220
   Val Pro Leu Arg Val Arg Ser Ser Tyr Ser Asn Asp Pro Gly Thr Leu
   225                 230                 235                 240
   Ile Ala Gly Ser Met Glu Asp Ile Pro Val Glu Glu Ala Val Leu Thr
                   245                 250                 255
   Gly Val Ala Thr Asp Lys Ser Glu Ala Lys Val Thr Val Leu Gly Ile
               260                 265                 270
   Ser Asp Lys Pro Gly Glu Ala Ala Lys Val Phe Arg Ala Leu Ala Asp
           275                 280                 285
   Ala Glu Ile Asn Ile Asp Met Val Leu Gln Asn Val Ser Ser Val Glu
       290                 295                 300
   Asp Gly Thr Thr Asp Ile Thr Phe Thr Cys Pro Arg Ser Asp Gly Arg
   305                 310                 315                 320
   Arg Ala Met Glu Ile Leu Lys Lys Leu Gln Val Gln Gly Asn Trp Thr
                   325                 330                 335
   Asn Val Leu Tyr Asp Asp Gln Val Gly Lys Val Ser Leu Val Gly Ala
               340                 345                 350
   Gly Met Lys Ser His Pro Gly Val Thr Ala Glu Phe Met Glu Ala Leu
           355                 360                 365
   Arg Asp Val Asn Val Asn Ile Glu Leu Ile Ser Thr Ser Glu Ile Arg
       370                 375                 380
   Ile Ser Val Leu Ile Arg Glu Asp Asp Leu Asp Ala Ala Ala Arg Ala
   385                 390                 395                 400
   Leu His Glu Gln Phe Gln Leu Gly Gly Glu Asp Glu Ala Val Val Tyr
                   405                 410                 415
   Ala Gly Thr Gly Arg
               420
<210> 17
   <211> 1479
   <212> DNA
   <213> Corynebacterium glutamicum
<220>
   <221> CDS
   <222> (1)..(1476)
<220>
   <221> mutation
   <222> (510)..(510)
   <223> Guanin Adenin Transition
<220>
   <221> mutation
   <222> (612)..(612)
   <223> Cytosin Guanin Transversion
<220>
   <221> mutation
   <222> (786)..(786)
   <223> Adenin Guanin Transition
<220>
   <221> mutation
   <222> (813)..(813)
   <223> Thymin Cytosin Transition
<220>
   <221> mutation
   <222> (985)..(985)
   <223> Cytosin Adenin Transversion
<220>
   <221> mutation
   <222> (1470)..(1470)
   <223> Thymin Guanin Transversion
<400> 17
   atg ccg tca agt acg atc aat aac atg act aat gga gat aat ctc gca      48
   Met Pro Ser Ser Thr Ile Asn Asn Met Thr Asn Gly Asp Asn Leu Ala
   1               5                   10                  15
   cag atc ggc gtt gta ggc cta gca gta atg ggc tca aac ctc gcc cgc      96
   Gln Ile Gly Val Val Gly Leu Ala Val Met Gly Ser Asn Leu Ala Arg
               20                  25                  30
   aac ttc gcc cgc aac ggc aac act gtc gct gtc tac aac cgc agc act      144
   Asn Phe Ala Arg Asn Gly Asn Thr Val Ala Val Tyr Asn Arg Ser Thr
           35                  40                  45
   gac aaa acc gac aag ctc atc gcc gat cac ggc tcc gaa ggc aac ttc      192
   Asp Lys Thr Asp Lys Leu Ile Ala Asp His Gly Ser Glu Gly Asn Phe
       50                  55                  60
   atc cct tct gca acc gtc gaa gag ttc gta gca tcc ctg gaa aag cca      240
   Ile Pro Ser Ala Thr Val Glu Glu Phe Val Ala Ser Leu Glu Lys Pro
   65                  70                  75                  80
   cgc cgc gcc atc atc atg gtt cag gct ggt aac gcc acc gac gca gtc      288
   Arg Arg Ala Ile Ile Met Val Gln Ala Gly Asn Ala Thr Asp Ala Val
                   85                  90                  95
   atc aac cag ctg gca gat gcc atg gac gaa ggc gac atc atc atc gac      336
   Ile Asn Gln Leu Ala Asp Ala Met Asp Glu Gly Asp Ile Ile Ile Asp
               100                 105                 110
   ggc ggc aac gcc ctc tac acc gac acc att cgt cgc gag aag gaa atc      384
   Gly Gly Asn Ala Leu Tyr Thr Asp Thr Ile Arg Arg Glu Lys Glu Ile
           115                 120                 125
   tcc gca cgc ggt ctc cac ttc gtc ggt gct ggt atc tcc ggc ggc gaa      432
   Ser Ala Arg Gly Leu His Phe Val Gly Ala Gly Ile Ser Gly Gly Glu
       130                 135                 140
   gaa ggc gca ctc aac ggc cca tcc atc atg cct ggt ggc cca gca aag      480
   Glu Gly Ala Leu Asn Gly Pro Ser Ile Met Pro Gly Gly Pro Ala Lys
   145                 150                 155                 160
   tcc tac gag tcc ctc gga cca ctg ctt gaa tcc atc gct gcc aac gtt      528
   Ser Tyr Glu Ser Leu Gly Pro Leu Leu Glu Ser Ile Ala Ala Asn Val
                   165                 170                 175
   gac ggc acc cca tgt gtc acc cac atc ggc cca gac ggc gcc ggc cac      576
   Asp Gly Thr Pro Cys Val Thr His Ile Gly Pro Asp Gly Ala Gly His
               180                 185                 190
   ttc gtc aag atg gtc cac aac ggc atc gag tac gcg gac atg cag gtc      624
   Phe Val Lys Met Val His Asn Gly Ile Glu Tyr Ala Asp Met Gln Val
           195                 200                 205
   atc ggc gag gca tac cac ctt ctc cgc tac gca gca ggc atg cag cca      672
   Ile Gly Glu Ala Tyr His Leu Leu Arg Tyr Ala Ala Gly Met Gln Pro
       210                 215                 220
   gct gaa atc gct gag gtt ttc aag gaa tgg aac gca ggc gac ctg gat      720
   Ala Glu Ile Ala Glu Val Phe Lys Glu Trp Asn Ala Gly Asp Leu Asp
   225                 230                 235                 240
   tcc tac ctc atc gaa atc acc gca gag gtt ctc tcc cag gtg gat gct      768
   Ser Tyr Leu Ile Glu Ile Thr Ala Glu Val Leu Ser Gln Val Asp Ala
                   245                 250                 255
   gaa acc ggc aag cca ctg atc gac gtc atc gtt gac gct gca ggc cag      816
   Glu Thr Gly Lys Pro Leu Ile Asp Val Ile Val Asp Ala Ala Gly Gln
               260                 265                 270
   aag ggc acc gga cgt tgg acc gtc aag gct gct ctt gat ctg ggt att      864
   Lys Gly Thr Gly Arg Trp Thr Val Lys Ala Ala Leu Asp Leu Gly Ile
           275                 280                 285
   gct acc acc ggc atc ggc gaa gct gtt ttc gca cgt gca ctc tcc ggc      912
   Ala Thr Thr Gly Ile Gly Glu Ala Val Phe Ala Arg Ala Leu Ser Gly
       290                 295                 300
   gca acc agc cag cgc gct gca gca cag ggc aac cta cct gca ggt gtc      960
   Ala Thr Ser Gln Arg Ala Ala Ala Gln Gly Asn Leu Pro Ala Gly Val
   305                 310                 315                 320
   ctc acc gat ctg gaa gca ctt ggc atg gac aag gca cag ttc gtc gaa     1008
   Leu Thr Asp Leu Glu Ala Leu Gly Met Asp Lys Ala Gln Phe Val Glu
                   325                 330                 335
   gac gtt cgc cgt gca ctg tac gca tcc aag ctt gtt gct tac gca cag     1056
   Asp Val Arg Arg Ala Leu Tyr Ala Ser Lys Leu Val Ala Tyr Ala Gln
               340                 345                 350
   ggc ttc gac gag atc aag gct ggc tcc gac gag aac aac tgg gac gtt     1104
   Gly Phe Asp Glu Ile Lys Ala Gly Ser Asp Glu Asn Asn Trp Asp Val
           355                 360                 365
   gac cct cgc gac ctc gct acc atc tgg cgc ggc ggc tgc atc att cgc     1152
   Asp Pro Arg Asp Leu Ala Thr Ile Trp Arg Gly Gly Cys Ile Ile Arg
       370                 375                 380
   gct aag ttc ctc aac cgc atc gtc gaa gca tac gat gca aac gct gaa     1200
   Ala Lys Phe Leu Asn Arg Ile Val Glu Ala Tyr Asp Ala Asn Ala Glu
   385                 390                 395                 400
   ctt gag tcc ctg ctg ctc gat cct tac ttc aag agc gag ctc ggc gac      1248
   Leu Glu Ser Leu Leu Leu Asp Pro Tyr Phe Lys Ser Glu Leu Gly Asp
                   405                     410                     415
   ctc atc gat tca tgg cgt cgc gtg att gtc acc gcc acc cag ctt ggc      1296
   Leu Ile Asp Ser Trp Arg Arg Val Ile Val Thr Ala Thr Gln Leu Gly
               420                     425                     430
   ctg cca atc cca gtg ttc gct tcc tcc ctg tcc tac tac gac agc ctg      1344
   Leu Pro Ile Pro Val Phe Ala Ser Ser Leu Ser Tyr Tyr Asp Ser Leu
           435                     440                     445
   cgt gca gag cgt ctg cca gca gcc ctg atc caa gga cag cgc gac ttc      1392
   Arg Ala Glu Arg Leu Pro Ala Ala Leu Ile Gln Gly Gln Arg Asp Phe
       450                     455                     460
   ttc ggt gcg cac acc tac aag cgc atc gac aag gat ggc tcc ttc cac      1440
   Phe Gly Ala His Thr Tyr Lys Arg Ile Asp Lys Asp Gly Ser Phe His
   465                     470                     475                     480
   acc gag tgg tcc ggc gac cgc tcc gag gtg gaa gct taa                  1479
   Thr Glu Trp Ser Gly Asp Arg Ser Glu Val Glu Ala
                   485                     490
<210> 18
   <211> 492
   <212> PRT
   <213> Corynebacterium glutamicum
<400> 18
   Met Pro Ser Ser Thr Ile Asn Asn Met Thr Asn Gly Asp Asn Leu Ala
   1               5                   10                  15
   Gln Ile Gly Val Val Gly Leu Ala Val Met Gly Ser Asn Leu Ala Arg
               20                  25                  30
   Asn Phe Ala Arg Asn Gly Asn Thr Val Ala Val Tyr Asn Arg Ser Thr
           35                  40                  45
   Asp Lys Thr Asp Lys Leu Ile Ala Asp His Gly Ser Glu Gly Asn Phe
       50                  55                  60
   Ile Pro Ser Ala Thr Val Glu Glu Phe Val Ala Ser Leu Glu Lys Pro
   65                  70                  75                  80
   Arg Arg Ala Ile Ile Met Val Gln Ala Gly Asn Ala Thr Asp Ala Val
                   85                  90                  95
   Ile Asn Gln Leu Ala Asp Ala Met Asp Glu Gly Asp Ile Ile Ile Asp
               100                 105                 110
   Gly Gly Asn Ala Leu Tyr Thr Asp Thr Ile Arg Arg Glu Lys Glu Ile
           115                 120                 125
   Ser Ala Arg Gly Leu His Phe Val Gly Ala Gly Ile Ser Gly Gly Glu
   130 135 140
   Glu Gly Ala Leu Asn Gly Pro Ser Ile Met Pro Gly Gly Pro Ala Lys
   145 150 155 160
   Ser Tyr Glu Ser Leu Gly Pro Leu Leu Glu Ser Ile Ala Ala Asn Val
   165 170 175
   Asp Gly Thr Pro Cys Val Thr His Ile Gly Pro Asp Gly Ala Gly His
   180 185 190
   Phe Val Lys Met Val His Asn Gly Ile Glu Tyr Ala Asp Met Gln Val
   195 200 205
   Ile Gly Glu Ala Tyr His Leu Leu Arg Tyr Ala Ala Gly Met Gln Pro
   210 215 220
   Ala Glu Ile Ala Glu Val Phe Lys Glu Trp Asn Ala Gly Asp Leu Asp
   225 230 235 240
   Ser Tyr Leu Ile Glu Ile Thr Ala Glu Val Leu Ser Gln Val Asp Ala
   245 250 255
   Glu Thr Gly Lys Pro Leu Ile Asp Val Ile Val Asp Ala Ala Gly Gln
   260 265 270
   Lys Gly Thr Gly Arg Trp Thr Val Lys Ala Ala Leu Asp Leu Gly Ile
   275 280 285
   Ala Thr Thr Gly Ile Gly Glu Ala Val Phe Ala Arg Ala Leu Ser Gly
   290 295 300
   Ala Thr Ser Gln Arg Ala Ala Ala Gln Gly Asn Leu Pro Ala Gly Val
   305 310 315 320
   Leu Thr Asp Leu Glu Ala Leu Gly Met Asp Lys Ala Gln Phe Val Glu
   325 330 335
   Asp Val Arg Arg Ala Leu Tyr Ala Ser Lys Leu Val Ala Tyr Ala Gln
   340 345 350
   Gly Phe Asp Glu Ile Lys Ala Gly Ser Asp Glu Asn Asn Trp Asp Val
   355 360 365
   Asp Pro Arg Asp Leu Ala Thr Ile Trp Arg Gly Gly Cys Ile Ile Arg
   370 375 380
   Ala Lys Phe Leu Asn Arg Ile Val Glu Ala Tyr Asp Ala Asn Ala Glu
   385 390 395 400
   Leu Glu Ser Leu Leu Leu Asp Pro Tyr Phe Lys Ser Glu Leu Gly Asp
   405 410 415
   Leu Ile Asp Ser Trp Arg Arg Val Ile Val Thr Ala Thr Gln Leu Gly
   420 425 430
   Leu Pro Ile Pro Val Phe Ala Ser Ser Leu Ser Tyr Tyr Asp Ser Leu
   435 440 445
   Arg Ala Glu Arg Leu Pro Ala Ala Leu Ile Gln Gly Gln Arg Asp Phe
   450 455 460
   Phe Gly Ala His Thr Tyr Lys Arg Ile Asp Lys Asp Gly Ser Phe His
   465 470 475 480
   Thr Glu Trp Ser Gly Asp Arg Ser Glu Val Glu Ala
   485 490
<210> 19
   <211> 1851
   <212> DNA
   <213> Corynebacterium glutamicum
<220>
   <221> CDS
   <222> (227)..(1702)
   <223> gnd allele with upstream- (1-226) and downstream-region (1703-1866)
<220>
   <221> mutation
   <222> (736)..(736)
   <223> Guanin Adenin Transition
<220>
   <221> mutation
   <222> (838)..(838)
   <223> Cytosin Guanin Transversion
<220>
   <221> mutation
   <222> (1012)..(1012)
   <223> Adenin Guanin Transition
<220>
   <221> mutation
   <222> (1039)..(1039)
   <223> Thymin Cytosin Transition
<220>
   <221> mutation
   <222> (1211)..(1211)
   <223> Cytosin Adenin Transversion
<220>
   <221> mutation
   <222> (1696)..(1696)
   <223> Thymin Guanin Transversion
<400> 19
   ccataattgg cgttgttgag tgcttcaagt tcgtctgtgg ttaaagctct ggtggcggca      60
   agttctgcaa gcgaaagcag atcttggggt tgatcatcgc gggaagtcat aattaattac     120
   tctagtcggc ctaaaatggt tggattttca ccttctgtga cctggtaaaa tcgccactac     180
   ccccaaatgg tcacaccttt taggccgatt ttgctgacac cgggct atg ccg tca        235
                                                      Met Pro Ser
                                                      1
   agt acg atc aat aac atg act aat gga gat aat ctc gca cag atc ggc       283
   Ser Thr Ile Asn Asn Met Thr Asn Gly Asp Asn Leu Ala Gln Ile Gly
       5                   10                  15
   gtt gta ggc cta gca gta atg ggc tca aac ctc gcc cgc aac ttc gcc       331
   Val Val Gly Leu Ala Val Met Gly Ser Asn Leu Ala Arg Asn Phe Ala
   20                  25                  30                  35
   cgc aac ggc aac act gtc gct gtc tac aac cgc agc act gac aaa acc       379
   Arg Asn Gly Asn Thr Val Ala Val Tyr Asn Arg Ser Thr Asp Lys Thr
                   40                  45                  50
   gac aag ctc atc gcc gat cac ggc tcc gaa ggc aac ttc atc cct tct       427
   Asp Lys Leu Ile Ala Asp His Gly Ser Glu Gly Asn Phe Ile Pro Ser
               55                  60                  65
   gca acc gtc gaa gag ttc gta gca tcc ctg gaa aag cca cgc cgc gcc       475
   Ala Thr Val Glu Glu Phe Val Ala Ser Leu Glu Lys Pro Arg Arg Ala
           70                  75                  80
   atc atc atg gtt cag gct ggt aac gcc acc gac gca gtc atc aac cag       523
   Ile Ile Met Val Gln Ala Gly Asn Ala Thr Asp Ala Val Ile Asn Gln
       85                  90                  95
   ctg gca gat gcc atg gac gaa ggc gac atc atc atc gac ggc ggc aac       571
   Leu Ala Asp Ala Met Asp Glu Gly Asp Ile Ile Ile Asp Gly Gly Asn
   100                 105                 110                 115
   gcc ctc tac acc gac acc att cgt cgc gag aag gaa atc tcc gca cgc       619
   Ala Leu Tyr Thr Asp Thr Ile Arg Arg Glu Lys Glu Ile Ser Ala Arg
                   120                 125                 130
   ggt ctc cac ttc gtc ggt gct ggt atc tcc ggc ggc gaa gaa ggc gca       667
   Gly Leu His Phe Val Gly Ala Gly Ile Ser Gly Gly Glu Glu Gly Ala
               135                 140                 145
   ctc aac ggc cca tcc atc atg cct ggt ggc cca gca aag tcc tac gag       715
   Leu Asn Gly Pro Ser Ile Met Pro Gly Gly Pro Ala Lys Ser Tyr Glu
           150                 155                 160
   tcc ctc gga cca ctg ctt gaa tcc atc gct gcc aac gtt gac ggc acc       763
   Ser Leu Gly Pro Leu Leu Glu Ser Ile Ala Ala Asn Val Asp Gly Thr
           165                 170                 175
   cca tgt gtc acc cac atc ggc cca gac ggc gcc ggc cac ttc gtc aag      811
   Pro Cys Val Thr His Ile Gly Pro Asp Gly Ala Gly His Phe Val Lys
   180                 185                 190                 195
   atg gtc cac aac ggc atc gag tac gcg gac atg cag gtc atc ggc gag      859
   Met Val His Asn Gly Ile Glu Tyr Ala Asp Met Gln Val Ile Gly Glu
                   200                 205                 210
   gca tac cac ctt ctc cgc tac gca gca ggc atg cag cca gct gaa atc      907
   Ala Tyr His Leu Leu Arg Tyr Ala Ala Gly Met Gln Pro Ala Glu Ile
               215                 220                 225
   gct gag gtt ttc aag gaa tgg aac gca ggc gac ctg gat tcc tac ctc      955
   Ala Glu Val Phe Lys Glu Trp Asn Ala Gly Asp Leu Asp Ser Tyr Leu
           230                 235                 240
   atc gaa atc acc gca gag gtt ctc tcc cag gtg gat gct gaa acc ggc     1003
   Ile Glu Ile Thr Ala Glu Val Leu Ser Gln Val Asp Ala Glu Thr Gly
       245                 250                 255
   aag cca ctg atc gac gtc atc gtt gac gct gca ggc cag aag ggc acc     1051
   Lys Pro Leu Ile Asp Val Ile Val Asp Ala Ala Gly Gln Lys Gly Thr
   260                 265                 270                 275
   gga cgt tgg acc gtc aag gct gct ctt gat ctg ggt att gct acc acc     1099
   Gly Arg Trp Thr Val Lys Ala Ala Leu Asp Leu Gly Ile Ala Thr Thr
                   280                 285                 290
   ggc atc ggc gaa gct gtt ttc gca cgt gca ctc tcc ggc gca acc agc     1147
   Gly Ile Gly Glu Ala Val Phe Ala Arg Ala Leu Ser Gly Ala Thr Ser
               295                 300                 305
   cag cgc gct gca gca cag ggc aac cta cct gca ggt gtc ctc acc gat     1195
   Gln Arg Ala Ala Ala Gln Gly Asn Leu Pro Ala Gly Val Leu Thr Asp
           310                 315                 320
   ctg gaa gca ctt ggc atg gac aag gca cag ttc gtc gaa gac gtt cgc     1243
   Leu Glu Ala Leu Gly Met Asp Lys Ala Gln Phe Val Glu Asp Val Arg
       325                 330                 335
   cgt gca ctg tac gca tcc aag ctt gtt gct tac gca cag ggc ttc gac     1291
   Arg Ala Leu Tyr Ala Ser Lys Leu Val Ala Tyr Ala Gln Gly Phe Asp
   340                 345                 350                 355
   gag atc aag gct ggc tcc gac gag aac aac tgg gac gtt gac cct cgc     1339
   Glu Ile Lys Ala Gly Ser Asp Glu Asn Asn Trp Asp Val Asp Pro Arg
                   360                 365                 370
   gac ctc gct acc atc tgg cgc ggc ggc tgc atc att cgc gct aag ttc     1387
   Asp Leu Ala Thr Ile Trp Arg Gly Gly Cys Ile Ile Arg Ala Lys Phe
               375                 380                 385
   ctc aac cgc atc gtc gaa gca tac gat gca aac gct gaa ctt gag tcc     1435
   Leu Asn Arg Ile Val Glu Ala Tyr Asp Ala Asn Ala Glu Leu Glu Ser
           390                 395                 400
   ctg ctg ctc gat cct tac ttc aag agc gag ctc ggc gac ctc atc gat     1483
   Leu Leu Leu Asp Pro Tyr Phe Lys Ser Glu Leu Gly Asp Leu Ile Asp
       405                 410                 415
   tca tgg cgt cgc gtg att gtc acc gcc acc cag ctt ggc ctg cca atc     1531
   Ser Trp Arg Arg Val Ile Val Thr Ala Thr Gln Leu Gly Leu Pro Ile
   420                 425                 430                 435
   cca gtg ttc gct tcc tcc ctg tcc tac tac gac agc ctg cgt gca gag      1579
   Pro Val Phe Ala Ser Ser Leu Ser Tyr Tyr Asp Ser Leu Arg Ala Glu
                   440                 445                 450
   cgt ctg cca gca gcc ctg atc caa gga cag cgc gac ttc ttc ggt gcg      1627
   Arg Leu Pro Ala Ala Leu Ile Gln Gly Gln Arg Asp Phe Phe Gly Ala
               455                 460                 465
   cac acc tac aag cgc atc gac aag gat ggc tcc ttc cac acc gag tgg      1675
   His Thr Tyr Lys Arg Ile Asp Lys Asp Gly Ser Phe His Thr Glu Trp
           470                 475                 480
   tcc ggc gac cgc tcc gag gtg gaa gct taacacaacg ccaaaacccc            1722
   Ser Gly Asp Arg Ser Glu Val Glu Ala
       485                 490
   tcacagtcac cttagattgt gaggggtttt tcgcgtgctg ccagggattc gccggaggtg    1782
   ggcgtcgata agcaaaaatc ttttaattgc ttttacccat ggctctgccc ttgttccaat    1842
   aaccttgcg                                                            1851
<210> 20
   <211> 492
   <212> PRT
   <213> Corynebacterium glutamicum
<400> 20
   Met Pro Ser Ser Thr Ile Asn Asn Met Thr Asn Gly Asp Asn Leu Ala
   1               5                   10                  15
   Gln Ile Gly Val Val Gly Leu Ala Val Met Gly Ser Asn Leu Ala Arg
               20                  25                  30
   Asn Phe Ala Arg Asn Gly Asn Thr Val Ala Val Tyr Asn Arg Ser Thr
           35                  40                  45
   Asp Lys Thr Asp Lys Leu Ile Ala Asp His Gly Ser Glu Gly Asn Phe
       50                  55                  60
   Ile Pro Ser Ala Thr Val Glu Glu Phe Val Ala Ser Leu Glu Lys Pro
   65                  70                  75                  80
   Arg Arg Ala Ile Ile Met Val Gln Ala Gly Asn Ala Thr Asp Ala Val
                   85                  90                  95
   Ile Asn Gln Leu Ala Asp Ala Met Asp Glu Gly Asp Ile Ile Ile Asp
               100                 105                 110
   Gly Gly Asn Ala Leu Tyr Thr Asp Thr Ile Arg Arg Glu Lys Glu Ile
           115                 120                 125
   Ser Ala Arg Gly Leu His Phe Val Gly Ala Gly Ile Ser Gly Gly Glu
   130 135 140
   Glu Gly Ala Leu Asn Gly Pro Ser Ile Met Pro Gly Gly Pro Ala Lys
   145 150 155 160
   Ser Tyr Glu Ser Leu Gly Pro Leu Leu Glu Ser Ile Ala Ala Asn Val
   165 170 175
   Asp Gly Thr Pro Cys Val Thr His Ile Gly Pro Asp Gly Ala Gly His
   180 185 190
   Phe Val Lys Met Val His Asn Gly Ile Glu Tyr Ala Asp Met Gln Val
   195 200 205
   Ile Gly Glu Ala Tyr His Leu Leu Arg Tyr Ala Ala Gly Met Gln Pro
   210 215 220
   Ala Glu Ile Ala Glu Val Phe Lys Glu Trp Asn Ala Gly Asp Leu Asp
   225 230 235 240
   Ser Tyr Leu Ile Glu Ile Thr Ala Glu Val Leu Ser Gln Val Asp Ala
   245 250 255
   Glu Thr Gly Lys Pro Leu Ile Asp Val Ile Val Asp Ala Ala Gly Gln
   260 265 270
   Lys Gly Thr Gly Arg Trp Thr Val Lys Ala Ala Leu Asp Leu Gly Ile
   275 280 285
   Ala Thr Thr Gly Ile Gly Glu Ala Val Phe Ala Arg Ala Leu Ser Gly
   290 295 300
   Ala Thr Ser Gln Arg Ala Ala Ala Gln Gly Asn Leu Pro Ala Gly Val
   305 310 315 320
   Leu Thr Asp Leu Glu Ala Leu Gly Met Asp Lys Ala Gln Phe Val Glu
   325 330 335
   Asp Val Arg Arg Ala Leu Tyr Ala Ser Lys Leu Val Ala Tyr Ala Gln
   340 345 350
   Gly Phe Asp Glu Ile Lys Ala Gly Ser Asp Glu Asn Asn Trp Asp Val
   355 360 365
   Asp Pro Arg Asp Leu Ala Thr Ile Trp Arg Gly Gly Cys Ile Ile Arg
               370                 375                 380
   Ala Lys Phe Leu Asn Arg Ile Val Glu Ala Tyr Asp Ala Asn Ala Glu
   385                 390                 395                 400
   Leu Glu Ser Leu Leu Leu Asp Pro Tyr Phe Lys Ser Glu Leu Gly Asp
                   405                 410                 415
   Leu Ile Asp Ser Trp Arg Arg Val Ile Val Thr Ala Thr Gln Leu Gly
               420                 425                 430
   Leu Pro Ile Pro Val Phe Ala Ser Ser Leu Ser Tyr Tyr Asp Ser Leu
           435                 440                 445
   Arg Ala Glu Arg Leu Pro Ala Ala Leu Ile Gln Gly Gln Arg Asp Phe
       450                 455                 460
   Phe Gly Ala His Thr Tyr Lys Arg Ile Asp Lys Asp Gly Ser Phe His
   465                 470                 475                 480
   Thr Glu Trp Ser Gly Asp Arg Ser Glu Val Glu Ala
                   485                 490
<210> 21
   <211> 30
   <212> DNA
   <213> Artificial Sequence
<220>
   <223> Primer gnd-int1-eco
<400> 21
   ctaggaattc ttcgtcggtg ctggtatctc 30
<210> 22
   <211> 30
   <212> DNA
   <213> Artificial Sequence
<220>
   <223> Primer gnd-int2-eco
<400> 22
   ctaggaattc gtcgatgcgc ttgtaggtgt 30

## Beschreibung

Gegenstand der Erfindung sind Mutanten und Allele des gnd-Gens coryneformer Bakterien kodierend für Varianten der 6-Phosphogluconat Dehydrogenase (EC: 1.1.1.44) und Verfahren zur Herstellung von Aminosäuren, insbesondere L-Lysin und Tryptophan, unter Verwendung von Bakterien, die diese Allele enthalten.

### Stand der Technik

Aminosäuren finden in der Humanmedizin, in der pharmazeutischen Industrie, in der Lebensmittelindustrie und ganz besonders in der Tierernährung Anwendung.

Es ist bekannt, dass Aminosäuren durch Fermentation von Stämmen coryneformer Bakterien, insbesondere Corynebacterium glutamicum, hergestellt werden. Wegen der großen Bedeutung wird ständig an der Verbesserung der Herstellverfahren gearbeitet. Verfahrensverbesserungen können fermentationstechnische Maßnahmen wie zum Beispiel Rührung und Versorgung mit Sauerstoff, oder die Zusammensetzung der Nährmedien, wie zum Beispiel die Zuckerkonzentration während der Fermentation, oder die Aufarbeitung zur Produktform durch zum Beispiel Ionenaustauschchromatographie oder die intrinsischen Leistungseigenschaften des Mikroorganismus selbst betreffen.

Zur Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite oder auxotroph für regulatorisch bedeutsame Metabolite sind und die Aminosäuren produzieren. Ein bekannter Antimetabolit ist das Lysinanalogon S-(2-Aminoethyl)-L-Cystein (AEC).

Seit einigen Jahren werden ebenfalls Methoden der rekombinanten DNA-Technik zur Stammverbesserung von L-Aminosäure produzierenden Stämmen von Corynebacterium eingesetzt, indem man einzelne Aminosäure-Biosynthesegene amplifiziert und die Auswirkung auf die Aminosäure-Produktion untersucht. Eine zusammenfassende Darstellung über verschiedenste Aspekte der Genetik, des Stoffwechsels und der Biotechnologie von Corynebacterium glutamicum findet sich bei Pühler (chief ed.) im Journal of Biotechnology 104 (1-3), 1-338, 2003.

Die Nukleotidsequenz des für die 6-Phosphogluconat Dehydrogenase kodierenden gnd-Gens eines als Brevibacterium flavum Stamm ML-223 (FERM BP-1497) bezeichneten coryneformen Bakteriums ist in der JP-A-9-224662 beschrieben. Die Nukleotidsequenz des gnd-Gens von Corynebacterium glutamicum kann der Patentanmeldung EP-A-1108790 als Sequenz Nr. 1605, Sequenz Nr. 7063 und Sequenz Nr. 7064 entnommen werden.

Weitere Nukleotidsequenzen zum gnd-Gen sind ebenfalls in der Datenbank des National Center for Biotechnology Information (NCBI) der National Library of Medicine (Bethesda, MD, USA) unter den Zugangsnummern (Accession Number) AX253243, AX121689, AX065125, AX065127, AX127147 und AX127148 hinterlegt.

Physiologische Untersuchungen zur Bedeutung der 6-Phosphogluconat Dehydrogenase wurden unter anderem von Sugimoto und Shiio (Agricultural and Biological Chemistry 51, 1257-1263) und Moritz et al. (European Journal of Biochemistry 267, 3442-3452 (2000)) veröffentlicht.

In der Patentanmeldung WO 01/71012 wird die förderliche Wirkung der Überexpression des gnd-Gens auf die Produktion und Herstellung verschiedener Aminosäuren mit Stämmen coryneformer Bakterien wie L-Lysin, L-Threonin, L-Isoleucin und L-Tryptophan mit Stämmen coryneformer Bakterien beschrieben.

Die Nukleotidsequenz, die für die 6-Phosphogluconat Dehydrogenase von Corynebacterium glutamicum kodiert, ist in der DNA Data Bank of Japan (DDBJ, Mishima, Japan http://gib.genes.nig.ac.jp/single/index.php?spid=Cglu_ATCC1 3032) unter der Genbezeichnung Cg11452 abgelegt. Die Kodierregion ist im Gegenstrang der dargestellten Genomsequenz von Nukleotid 1530338 bis Nukleotid 1531816 lokalisiert. Zur Lokalisierung der Nukleotidsequenz kann ebenfalls die Datenbank des National Center for Biotechnology Information (NCBI, Bethesda, MD, USA)herangezogen werden. Unter der Zugangsnummer (Accession Nummer) AX127148 ist die Kodierregion der 6-Phosphogluconat Dehydrogenase von Nukleotid 30341 bis Nukleotid 31816 im Gegenstrang der dort angegebenen Nukleotidsequenz zu finden.

Der besseren Übersichtlichkeit halber ist die Nukleotidsequenz des für die 6-Phosphogluconat Dehydrogenase aus Corynebacterium glutamicum kodierenden gnd-Gens ("Wildtyp-Gen") gemäß den Angaben der DDJB- und NCBI-Datenbank in SEQ ID NO:1 und die sich daraus ergebende Aminosäuresequenz der kodierten 6-Phosphogluconat Dehydrogenase in SEQ ID NO:2 und 4 dargestellt. In SEQ ID NO:3 sind stromaufwärts (upstream) und und stromabwärts (downstream) gelegene Nukleotidsequenzen mit angegeben.

### Aufgabe der Erfindung

Die Erfinder haben sich zur Aufgabe gestellt, neue Maßnahmen zur verbesserten Herstellung von Aminosäuren, insbesondere L-Lysin und L-Tryptophan, bereitzustellen.

### Beschreibung der Erfindung

Gegenstand der Erfindung sind erzeugte beziehungsweise isolierte Mutanten coryneformer Bakterien, die bevorzugt Aminosäuren ausscheiden, und welche ein Gen bzw. Allel enthalten, das für ein Polypeptid mit 6-Phosphogluconat Dehydrogenase Aktivität kodiert, **dadurch gekennzeichnet, dass** das Polypeptid eine Aminosäuresequenz umfasst, bei der an der Position 329 der Aminosäuresequenz SEQ ID NO:2 jede proteinogene Aminosäure ausgenommen L-Valin enthalten ist. Der Austausch von L-Valin gegen L-Methionin wird bevorzugt.

Bei den coryneformen Bakterien wird die Gattung Corynebacterium bevorzugt. Besonders bevorzugt sind Aminosäure-ausscheidende Stämme, die auf folgenden Arten beruhen:
Corynebacterium efficiens, wie zum Beispiel der Stamm DSM44549,
Corynebacterium glutamicum, wie zum Beispiel der Stamm ATCC13032,
Corynebacterium thermoaminogenes wie zum Beispiel der Stamm FERM BP-1539, und
Corynebacterium ammoniagenes, wie zum Beispiel der Stamm ATCC6871,
wobei die Art Corynbacterium glutamicum ganz besonders bevorzugt wird.

Einige Vertreter der Art Corynebacterium glutamicum sind im Stand der Technik auch unter anderen Artbezeicnungen bekannt. Hierzu gehören beispielsweise:
Corynebacterium acetoacidophilum ATCC13870
Corynebacterium lilium DSM20137
Corynebacterium melassecola ATCC17965
Brevibacterium flavum ATCC14067
Brevibacterium lactofermentum ATCC13869 und
Brevibacterium divaricatum ATCC14020

Bekannte Vertreter Aminosäure ausscheidender Stämme coryneformer Bakterien sind beispielsweise
die L-Lysin produzierenden Stämme
Corynebacterium glutamicum DM58-1/pDM6 (= DSM4697) beschrieben in EP 0 358 940
Corynebacterium glutamicum MH20 (= DSM5714) beschrieben in EP 0 435 132
Corynebacterium glutamicum AHP-3 (= FermBP-7382) beschrieben in EP 1 108 790
Corynebacterium thermoaminogenes AJ12521 (= FERM BP-3304) beschrieben in US 5,250,423
oder die L-Tryptophan produzierenden Stämme
Corynebacterium glutamicum K76 (=FermBP-1847) beschrieben in US 5,563,052
Corynebacterium glutamicum BPS13 (=FermBP-1777) beschrieben in US 5,605,818
Corynebacterium glutamicum FermBP-3055 beschrieben in US 5,235,940

Angaben zur taxonomischen Einordnung von Stämmen dieser Gruppe von Bakterien findet man unter anderem bei Seiler (Journal of General Microbiology 129, 1433-1477 (1983), Kämpfer und Kroppenstedt (Canadian Journal of Microbiology 42, 989-1005 (1996)), Liebl et al (International Journal of Systematic Bacteriology 41, 255-260 (1991) und in der US-A-5, 250, 434.

Stämme mit der Bezeichnung "ATCC" können von der American Type Culture Collection (Manassas, VA, USA) bezogen werden. Stämme mit der Bezeichnung "DSM" können von der Deutschen Sammlung von Mikroorganismen und Zellkulturen (DSMZ, Braunschweig, Deutschland) bezogen werden. Stämme mit der Bezeichnung "FERM" können vom National Institute of Advanced Industrial Science and Technology (AIST Tsukuba Central 6, 1-1-1 Higashi, Tsukuba Ibaraki, Japan) bezogen werden. Die genannten Stämme von Corynebacterium thermoaminogenes (FERM BP-1539, FERM BP-1540, FERM BP-1541 und FERM BP-1542) sind in der US-A 5,250,434 beschrieben.

Unter proteinogenen Aminosäuren versteht man die Aminosäuren, die in natürlichen Proteinen, das heißt in Proteinen von Mikroorganismen, Pflanzen, Tieren und Menschen vorkommen. Hierzu gehören insbesondere L-Aminosäuren, ausgewählt aus der Gruppe L-Asparaginsäure, L-Asparagin, L-Threonin, L-Serin, L-Glutaminsäure, L-Glutamin, Glycin, L-Alanin, L-Cystein, L-Valin, L-Methionin, L-Isoleucin, L-Leucin, L-Tyrosin, L-Phenylalanin, L-Histidin, L-Lysin, L-Tryptophan, L-Prolin und L-Arginin. Zu den Aminosäuren zählt auch L-Homoserin.

Die Begriffe Protein und Polypeptid sind gegenseitig austauschbar.

Die erfindungsgemäßen Mutanten scheiden bevorzugt die genannten proteinogen Aminosäuren, insbesondere L-Lysin aus. Der Begriff Aminosäuren umfasst auch deren Salze wie beispielsweise das Lysin-Monohydrochlorid oder Lysin-sulfat im Falle der Aminosäure L-Lysin.

Gegenstand der Erfindung sind weiterhin Mutanten coryneformer Bakterien, die ein gnd-Allel enthalten, das für ein Polypeptid mit 6-Phosphogluconat Dehydrogenase Enzymaktivität kodiert, das die Aminsäuresequenz von SEQ ID NO: 2 umfasst, wobei an Position 329 jede proteinogene Aminosäure ausgenommen L-Valin enthalten ist. Der Austausch L-Valin gegen L-Methionin wird bevorzugt.

Gegenstand der Beschreibung sind weiterhin Mutanten coryneformer Bakterien, die ein gnd-Allel enthalten, das für ein Polypeptid mit 6-Phosphogluconat Dehydrogenase Enzymaktivität kodiert, das an der Position 329 der Aminosäuresequenz von SEQ ID NO:2 entsprechenden Position jede proteinogene Aminosäure ausgenommen L-Valin, bevorzugt L-Methionin enthält, wobei das Gen eine Nukleotidsequenz umfasst, die mit der Nukleotidsequenz eines Polynukleotids identisch ist, das durch eine Polymerasekettenreaktion (PCR) unter Verwendung eines Primerpaars erhältlich ist, deren Nukleotidsequenzen jeweils mindestens 15 aufeinanderfolgende Nukleotide umfassen, die aus der Nukleotidsequenz zwischen Position 1 und 226 von SEQ ID NO:3 und aus der komplementären Nukleotidsequenz zwischen Position 1866 und 1703 von SEQ ID NO:3 ausgewählt werden. Beispiele für derartige geeignete Primerpaare sind in SEQ ID NO:7 und SEQ ID NO:8 und in SEQ ID NO:9 und SEQ ID NO:10 dargestellt. Als Ausgangsmaterial ("template"-DNA)) wird chromosomale DNA coryneformer Bakterien bevorzugt, die insbesondere mit einem Mutagen behandelt worden sind. Besonders bevorzugt wird die chromosomale DNA der Gattung Corynebacterium und ganz besonders bevorzugt die der Art Corynebacterium glutamicum.

Gegenstand der Beschreibung sind weiterhin Mutanten coryneformer Bakterien, die ein gnd-Allel enthalten, das für ein Polypeptid mit 6-Phosphogluconat Dehydrogenase Enzymaktivität kodiert, das eine Aminsäuresequenz mit einer Länge entsprechend 492 L-Aminosäuren umfasst, wobei an Position 329 jede proteinogene Aminosäure ausgenommen L-Valin, bevorzugt L-Methionin, enthalten ist.

Gegenstand der Beschreibung sind weiterhin Mutanten coryneformer Bakterien, die ein gnd-Allel enthalten, das für ein Polypeptid mit 6-Phosphogluconat Dehydrogenase Enzymaktivität kodiert, das an Position 324 bis 334 der Aminosäuresquenz die Aminosäuresequenz entsprechend Position 324 bis 334 von SEQ ID NO:6 enthält. Bevorzugt enthält die Aminosäuresequenz des kodierten Polypeptids eine Aminosäuresequenz entsprechend Position 313 bis 345 von SEQ ID NO:6 oder Position 297 bis 361 von SEQ ID NO:6 oder Position 281 bis 377 von SEQ ID NO:6 oder Position 265 bis 393 von SEQ ID NO:6 oder Position 201 bis 457 von SEQ ID NO:6 oder Position 72 bis 492 von SEQ ID NO:6 oder Position 2 bis 492 von SEQ ID NO:6. Ganz besonders bevorzugt umfasst die Länge des kodierten Proteins 492 Aminosäuren.

Gegenstand der Erfindung sind weiterhin Mutanten coryneformer Bakterien, die ein gnd-Allel enthalten, das für ein Polypeptid mit 6-Phosphogluconat Dehydrogenase Enzymaktivität kodiert, welches an Position 329 der Aminosäuresequenz jede Aminosäure ausgenommen L-Valin enthält, wobei dem Austausch gegen L-Methionin der Vorzug gegeben wird und dessen Aminosäuresequenz außerdem zu mindestens 98% oder mindestens 99% identisch ist mit der Aminosäuresequenz von SEQ ID NO:6.

Gegenstand der Erfindung sind weiterhin Mutanten coryneformer Bakterien, die ein gnd-Allel enthalten, das für ein Polypeptid mit 6-Phosphogluconat Dehydrogenase Enzymaktivität kodiert, welches an Position 329 der Aminosäuresequenz L-Methionin enthält und dessen Nukleotidsequenz außerdem zu mindestens 99% identisch ist mit der Nukleotidsequenz von SEQ ID NO:5.

Es ist bekannt, dass konservative Aminosäureaustausche die Enzymaktivität nur unwesentlich verändern. Dementsprechend kann das in den erfindungsgemäßen Mutanten enthaltene gnd-Allel, das für ein Polypeptid mit 6-Phosphogluconat Dehydrogenase Enzymaktivität kodiert, zusätzlich zu der in SEQ ID NO:6 gezeigten Aminosäuresequenz einen (1) oder mehrere konservative Aminosäureaustausch(e) enthalten. Bevorzugt enthält das Polypeptid höchstens zwei (2), höchstens drei (3), höchstens vier (4) oder höchstens fünf (5) konservative Aminosäuraustausche.

Bei den aromatischen Aminosäuren spricht man von konservativen Austauschen, wenn Phenylalanin, Tryptophan und Tyrosin gegeneinander ausgetauscht werden. Bei den hydrophoben Aminosäuren spricht man von konservativen Austauschen, wenn Leucin, Isoleucin und Valin gegeneinander ausgetauscht werden. Bei den polaren Aminosäuren spricht man von konservativen Austauschen, wenn Glutamin und Aparagin gegeneinander ausgetauscht werden. Bei den basischen Aminosäuren spricht man von konservativen Austauschen, wenn Arginin, Lysin und Histidin gegeneinander ausgetauscht werden. Bei den sauren Aminosäuren spricht man von konservativen Austauschen, wenn Asparaginsäure und Glutaminsäure gegeneinander ausgetauscht werden. Bei den Hydroxyl-Gruppen enthaltenden Aminosäuren spricht man von konservativen Austauschen, wenn Serin und Threonin gegeneinander ausgetauscht werden.

Bei der Arbeit an der vorliegenden Erfindung wurde durch Vergleich der Aminosäuresequenz mit dem Clustal-Programm (Thompson et al., Nucleic Acids Research 22, 4637-4680 (1994)) festgestellt, dass die Aminosäuresequenzen der 6-Phosphogluconat Dehydrogenase verschiedener Bakterien wie bespielsweise Escherichia coli, Corynebacterium efficiens, Mycobacterium leprae, Mycobacterium tuberculosis, Bacillus subtilis, Streptomyces coelicolor und Streptomyces avermitilis ein Sequenzmotiv bestehend aus der Abfolge Leu-Bra-Asp-Val-Ile-Val-Asp und auch ein Sequenzmotiv bestehend aus der Abfolge Ile-Aro-Arg-Ali-Gly-Cys-Ile-Ile-Arg-Ala enthalten. Die Bezeichnung "Bra" steht für die Aminosäuren Ile oder Val, "Aro" für Trp oder Phe und die Bezeichnung "Ali" für die Aminosäuren Gly oder Ala.

Dementsprechend sind solche Mutanten coryneformer Bakterien bevorzugt, die ein gnd-Allel enthalten, das für ein Polypeptid mit 6-Phosphogluconat Dehydrogenase Enzymaktivität kodiert, welches mindestens eine Aminosäuresequenz ausgewählt aus der Gruppe Leu-Bra-Asp-Val-Ile-Val-Asp und Ile-Aro-Arg-Ali-Gly-Cys-Ile-Ile-Arg-Ala umfasst und an Position 329 von SEQ ID NO:2, Position der Aminosäuresequenz jede Aminosäure ausgenommen L-Valin, bevorzugt L-Methionin, enthält.

Das Aminosäuresequenzmotiv Leu-Bra-Asp-Val-Ile-Val-Asp ist beispielsweise in der SEQ ID NO:6 von Position 262 bis 268 enthalten. Das Aminosäuresequenzmotiv Ile-Aro-Arg-Ali-Gly-Cys-Ile-Ile-Arg-Ala ist beispielsweise in der SEQ ID NO:6 von Position 376 bis 385 enthalten.

Gegenstand der Erfindung sind schließlich Mutanten coryneformer Bakterien, die ein gnd-Allel enthalten, das für ein Polypeptid mit 6-Phosphogluconat Dehydrogenase Enzymaktivität kodiert, welches die Amionosäuresequenz von SEQ ID NO:6 umfasst.

Es ist bekannt, dass durch wirtseigene Enzyme, sogenannte Aminopeptidasen, das endständige Methionin bei der Proteinsynthese entfernt wird.

Unter dem Begriff "einer zu Position 329 der Aminosäuresequenz entsprechenden Position" oder "einer zu Position 329 der Aminosäuresequenz vergleichbaren Position" versteht man die Tatsache, dass durch Insertion oder Deletion eines für eine Aminosäure kodierenden Kodons im N-terminalen Bereich (bezogen auf die Position 329 von SEQ ID NO:6) des kodierten Polypeptids die Positionsangabe und Längenangabe im Falle einer Insertion formal um eine Einheit erhöht oder im Falle einer Deletion um eine Einheit verringert wird. Beispielsweise rückt durch Deletion des für die Aminosäure L-Prolin kodierenden CCG-Kodons an Position 2 von SEQ ID NO:5 das L-Methionin von Position 329 an Position 328. In gleicher Weise wird durch Insertion oder Deletion eines für eine Aminosäure kodierenden Kodons im C-terminalen Bereich (bezogen auf die Position 329) des kodierten Polypeptids die Längenangabe im Falle einer Insertion formal um eine Einheit erhöht oder im Falle einer Deletion um eine Einheit verringert. Derartige vergleichbare Positionen lassen sich durch Vergleich der Aminosäuresequenzen in Form eines "alignments" beispielsweise mit Hilfe des Clustal-Programmes leicht identifizieren. Durch derartige Insertionen und Deletionen wird die enzymatische Aktivität im wesentlichen nicht berührt. "Im wesentlichen nicht berührt" bedeutet, dass sich die enzymatische Aktivität der genannten Varianten um maximal 20%, maximal 15%, maximal 10%, maximal 7,5%, maximal 5%, maximal 2,5% oder maximal 1% von der Aktivität des Polypeptids mit der Aminosäuresequenz von SEQ ID NO:6 unterscheidet.

Gegenstand der Erfindung sind dementsprechend auch gnd-Allele, die für Polypeptid Varianten von SEQ ID NO:6 kodieren, die eine, maximal 5, maximal 4, maximal 3 oder maximal 2 Insertionen oder Deletionen von Aminosäuren enthalten.

Die Sequenzmotive Leu-Bra-Asp-Val-Ile-Val-Asp und Ile-Aro-Arg-Ali-Gly-Cys-Ile-Ile-Arg-Ala werden durch derartige Insertionen/Deletionen vorzugsweise nicht zerrissen.

Zur Herstellung der erfindungsgemäßen Mutanten können klassische in-vivo Mutageneseverfahren mit Zellpopulationen coryneformer Bakterien unter Verwendung mutagener Stoffe wie beispielsweise N-Methyl-N'-Nitro-N-Nitrosoguanidin (MNNG) oder ultraviolettes Licht verwendet werden.

Mutagenesemethoden sind beispielsweise im Manual of Methods for General Bacteriology (Gerhard et al. (Eds.), American Society for Microbiology, Washington, DC, USA, 1981) oder bei Tosaka et al. (Agricultural and Biological Chemistry 42(4), 745-752 (1978)) oder bei Konicek et al (Folia Microbiologica 33, 337-343 (1988)) beschrieben. Typische Mutagenesen unter Verwendung von MNNG umfassen Konzentrationen 50 bis 500 mg/l oder auch höhere Konzentrationen bis zu maximal 1 g/l, eine Inkubationszeit von 1 bis 30 Minuten bei einem pH von 5,5 bis 7,5. Unter diesen Bedingungen wird die Zahl der lebensfähigen Zellen um einen Anteil von ca. 50% bis 90% oder ca. 50% bis 99% oder ca. 50% bis 99,9% oder mehr reduziert.

Aus der mutagenisierten Zellpopulation werden Mutanten beziehungsweise Zellen entnommen und vermehrt. Vorzugsweise wird anschließend deren Fähigkeit untersucht, in einer Satzkultur (batch) unter Verwendung eines geeigneten Nährmediums Aminosäuren, bevorzugt L-Lysin oder L-Tryptophan, auszuscheiden. Geeignete Nährmedien und Testbedingungen sind unter anderem in der US 6,221,636, in der US 6,632,644, in der WO 00/63388 und WO 03/014330 beschrieben. Bei Verwendungen von geeigneten Roboteranlagen, wie beispielsweise bei Zimmermann et al. (VDI Berichte Nr. 1841, VDI-Verlag, Düsseldorf, Deutschland 2004, 439-443) oder Zimmermann (Chemie Ingenenieur Technik 77 (4), 426-428 (2005) )beschrieben, können zahlreiche Mutanten in kurzer Zeit untersucht werden. Auf diese Weise werden Mutanten identifiziert, die im Vergleich zum Elternstamm beziehungsweise nicht mutagenisierten Ausgangsstamm vermehrt Aminosäuren in das Nährmedium auausscheiden. Dazu gehören beispielsweise solche Mutanten, deren Aminosäuresekretion um mindestens 0,5% erhöht ist.

Anschließend wird aus den Mutanten DNA bereitgestellt beziehungsweise isoliert und mit Hilfe der Polymerase-Kettenreaktion unter Verwendung von Primerpaaren, die die Amplifizierung des gnd-Gens beziehungsweise des erfindungsgemäßen gnd-Allels oder der erfindungsgemäßen Mutation an Position 329 der Aminosäuresequenz erlauben, das entsprechende Polynukleotid synthetisiert. Vorzugsweise wird die DNA aus solchen Mutanten isoliert, die in erhöhter Weise Aminosäuren ausscheiden.

Hierzu können beliebige Primerpaare aus der stromaufwärts und stromabwärts der erfindungsgemäßen Mutation gelegenen Nukleotidsequenz und der dazu komplementären Nukleotidsequenz ausgewählt werden. Ein Primer eines Primerpaares umfasst hierbei bevorzugt mindestens 15, mindestens 18, mindestens 20, mindestens 21 oder mindestens 24 aufeinanderfolgende Nukleotide ausgewählt aus der Nukleotidsequenz zwischen Position 1 und 1210 von SEQ ID NO:3 oder SEQ ID NO:19. Der dazugehörige zweite Primer eines Primerpaares umfasst mindestens 15, mindestens 18, mindestens 20, mindestens 21 oder mindestens 24 aufeinanderfolgende Nukleotide ausgewählt aus der komplementären Nukleotidsequenz von Position 1866 und 1214 von SEQ ID NO:3 oder SEQ ID NO:19. Ist die Amplifikation der Kodierregion gewünscht, so wird das Primerpaar vorzugsweise aus der Nukleotidsequenz zwischen Position 1 und 226 von SEQ ID NO:3 oder SEQ ID NO:19 und aus der komplementären Nukleotidsequenz zwischen Position 1866 und 1703 von SEQ ID NO:3 oder SEQ ID NO:19 ausgewählt. Ist die Amplifikation eines Teils der Kodierregion, wie beispielsweise in SEQ ID NO:11 und 13 dargestellt, erwünscht, so wird das Primerpaar vorzugsweise aus der Nukleotidsequenz zwischen Position 228 und 1210 von SEQ ID NO:3 oder SEQ ID NO:19 und aus der komplementären Nukleotidsequenz zwischen Position 1701 und 1214 von SEQ ID NO:3 oder SEQ ID NO:19 ausgewählt.

Geeignete Primerpaare sind beispielsweise das unter SEQ ID NO:7 und SEQ ID NO:8 wiedergegebene Primerpaar gnd-A1 und gnd-E1 oder das unter SEQ ID NO:9 und SEQ ID NO:10 wiedergegebene Primerpaar gnd-FS1 und gnd-FS-E.

Der Primer kann überdies mit Erkennungsstellen für Restriktionsenzyme, mit einer Biotin-Gruppe oder weiteren Accessoirs, wie sie im Stand der Technik beschrieben sind, ausgerüstet sein. Die Gesamtlänge des Primers beträgt im Allgemeinen maximal 30, 40, 50 oder 60 Nukleotide.

Für die Herstellung von Polynukleotiden durch Amplifikation ausgewählter Sequenzen wie dem erfindungsgemäßen gnd-Allel aus vorgelegter, beispielsweise chromosomaler DNA durch Amplifikation mittels PCR werden im Allgemeinen thermostabile DNA-Polymerasen eingesetzt. Beispiele derartiger DNA-Polymerasen sind die Taq-Polymerase aus Thermus aquaticus, die unter anderem von der Firma Qiagen (Hilden, Deutschland) vertrieben wird, die Vent-Polymerase aus Thermococcus litoralis, die unter anderem von der Firma New England Biolabs (Frankfurt, Deutschland) vertrieben wird oder die Pfu-Polymerase aus Pyrococcus furiosus, die unter anderem von der Firma Stratagene (La Jolla, USA) vertrieben wird. Bevorzugt werden Polymerasen mit "proof-reading"-Aktivität. "proof-reading"-Aktivität bedeutet, dass diese Polymerasen in der Lage sind, fehlerhaft eingebaute Nukleotide zu erkennen und den Fehler durch erneute Polymerisation zu beheben (Lottspeich und Zorbas, Bioanalytik, Spektrum Akademischer Verlag, Heidelberg, Deutschland (1998)). Beispiele für Polymerasen mit "proof-reading"-Aktivität sind die Vent-Polymerase und die Pfu-Polymerase.

Die Bedingungen im Reaktionsansatz werden nach Angaben des Herstellers eingestellt. Die Polymerasen werden vom Hersteller im Allgemeinen zusammen mit dem gebräuchlichen Puffer geliefert, welcher üblicherweise Konzentrationen von 10 - 100 mM Tris/HCI und 6 - 55 mM KCL bei pH 7,5 - 9,3 aufweist. Magnesiumchlorid wird in einer Konzentration von 0,5 - 10 mM zugesetzt, falls es nicht in dem vom Hersteller gelieferten Puffer enthalten ist. Dem Reaktionsansatz werden weiterhin Desoxynucleosidtriphosphate in einer Konzentration von 0,1 - 16,6 mM zugesetzt. Die Primer werden im Reaktionsansatz mit einer Endkonzentration von 0,1 - 3 µM vorgelegt und die Template-DNA im optimalen Fall mit 10² bis 10⁵ Kopien. Die entsprechende Polymerase wird dem Reaktionsansatz in einer Menge von 2-5 Units zugegeben. Ein typischer Reaktionsansatz hat ein Volumen von 20 - 100 µl.

Als weitere Zusätze können der Reaktion Rinderserumalbumin, Tween-20, Gelatin, Glycerin, Formamid oder DMSO beigesetzt werden (Dieffenbach und Dveksler, PCR Primer - A Laboratory Manual, Cold Spring Harbor Laboratory Press, USA 1995).

Ein typischer PCR-Verlauf besteht aus drei
unterschiedlichen, sich aufeinanderfolgend wiederholenden Temperaturstufen. Vorab wird die Reaktion mit einer Temperaturerhöhung auf 92°C - 98°C für 4 bis 10 Minuten gestartet, um die vorgelegte DNA zu denaturieren. Dann folgen sich wiederholend zuerst ein Schritt zur Denaturierung der vorgelegten DNA von 10 - 60 Sekunden bei ungefähr 92-98°C, dann ein Schritt zum Binden der Primer an die vorgelegte DNA von 10 - 60 Sekunden bei einer bestimmten, von den Primern abhängigen Temperatur ("Annealing-Temperatur"), die erfahrungsgemäß bei 50°C bis 60°C liegt und sich für jedes Primerpaar einzeln berechnen läßt. Genaue Informationen dazu findet der Fachmann bei Rychlik et al. (Nucleic Acids Research 18 (21): 6409-6412). Abschließend folgt ein Synthese-Schritt zur Verlängerung der vorgelegten Primer ("Extension") bei dem jeweils für die Polymerase angegebenen Aktivitätsoptimum, üblicherweise je nach Polymerase im Bereich von 73°C bis 67°C bevorzugt 72°C bis 68°C. Die Dauer dieses Extensionschrittes hängt von der Leistungsfähigkeit der Polymerase und Länge des zu amplifizierenden PCR-Produktes ab. In einer typischen PCR dauert dieser Schritt 0,5 - 8 Minuten, bevorzugt 2 - 4 Minuten. Diese drei Schritte werden 30 bis 35 mal, gegebenenfalls bis zu 50 mal wiederholt. Ein abschließender "Extension"-Schritt von 4 - 10 Minuten beendet die Reaktion. Die auf diese Weise hergestellten Polynukleotide werden auch als Amplifikate bezeichnet; die Begriffe Nukleinsäurefragment und Nukleinsäuremolekül sind ebenfalls gebräuchlich.

Weitere Anleitungen und Informationen zur PCR findet der Fachmann beispielsweise im Handbuch "PCR-Strategies" (Innis, Felfand und Sninsky, Academic Press , Inc., 1995), im Handbuch von Diefenbach und Dveksler "PCR Primer - a laboratory manual" (Cold Spring Harbor Laboratory Press,1995), im Handbuch von Gait "Oligonukleotide synthesis: A Practical Approach" (IRL Press, Oxford, UK, 1984) und bei Newton und Graham "PCR" (Spektrum Akademischer Verlag, Heidelberg, Deutschland, 1994).

Die Nukleotidsequenz wird anschließend beispielsweise nach dem Kettenabbruchverfahren von Sanger et al. (Proceedings of the National Academies of Sciences, U.S.A., 74, 5463-5467 (1977)) mit den von Zimmermann et al. (Nucleic Acids Research 18, 1067pp (1990)) angegebenen Modifikationen bestimmt und das von dieser Nukleotidsequenz kodierte Polypeptid insbesondere bezüglich der Aminosäuresequenz analysiert. Dazu wird die Nukleotidsequenz in ein Programm zur Übersetzung von DNA-Sequenz in eine Aminosäuresequenz eingegeben. Geeignete Programme sind beispielsweise das Programm "Patentin", das bei Patentämtern, beispielsweise dem US-Patentamt (USPTO) erhältlich ist, oder das "Translate Tool", das auf dem ExPASy Proteomics Server im World Wide Web (Gasteiger et al., Nucleic Acids Research 31, 3784-3788 (2003)) verfügbar ist.

Auf diese Weise werden Mutanten identifiziert, deren gnd-Allele für Proteine mit 6-Phosphogluconat Dehydrogenase Enzymaktivität kodieren, welche an Position 329 von SEQ ID NO:2 Position jede proteinogene Aminosäure ausgenommen L-Valin enthalten. Bevorzugt wird der Austausch gegen L-Methionin.

Gegenstand der Beschreibung ist dementsprechend eine Mutante eines coryneformen Bakteriums, **dadurch gekennzeichnet, dass** diese durch folgende Schritte erhältlich ist:
a) Behandlung eines coryneformen Bakteriums, das die Fähigkeit besitzt Aminosäuren auszuscheiden, mit einem mutagenen Agenz,
b) Isolierung und Vermehrung der in a) erzeugten Mutante,
c) vorzugsweise Bestimmung der Fähigkeit der Mutante in einem Medium mindestens 0,5% mehr Aminosäure auszuscheiden oder im Zellinneren anzureichern als das in a) eingesetzte coryneforme Bakterium,
d) Bereitstellung von Nukleinsäure aus der in b) erhaltenen Mutante,
e) Herstellung eines Nukleinsäuremoleküls unter Verwendung der Polymerasekettenreaktion, der Nukleinsäure aus d), und eines Primerpaares bestehend aus einem ersten Primer umfassend mindestens 15 aufeinanderfolgende Nukleotide ausgewählt aus der Nukleotidsequenz zwischen Position 1 und 1210 von SEQ ID NO:3 oder SEQ ID NO:19 und einem zweitem Primer umfassend mindestens 15 aufeinanderfolgenden Nukleotide ausgewählt aus der komplementären Nukleotidsequenz zwischen Position 1866 und 1214 von SEQ ID NO:3.
f) Bestimmung der Nukleotidsequenz des in e) erhaltenen Nukleinsäuremoleküls, und Bestimmung der kodierten Aminosäuresequenz,
g) gegebenfalls Vergleich der in f) bestimmten Aminosäuresesequenz mit SEQ ID NO:6 oder 18, und
h) Identifizierung einer Mutante, die ein Polynukleotid enthält, das für ein Polypeptid kodiert, welches an Position 329 oder an vergleichbarer Position jede proteinogene Aminosäure ausgenommen L-Valin, bevorzugt L-Methionin enthält.

Die auf diese Weise erzeugten Mutanten enthalten typischerweise eine (1) Kopie des beschriebenen gnd Allels.

In der SEQ ID NO:5 ist beispielhaft die Kodierregion eines gnd-Allels einer erfindungsgemäßen Mutante wiedergegeben. Die Kodierregion des Wildtypgens ist als SEQ ID NO:1 wiedergegeben. SEQ ID NO:1 enthält an Position 985 die Nukleobase Guanin und SEQ ID NO:5 die Nukleobase Adenin. Durch diese Guanin Adenin Transition entsteht an Position 985 bis 987 das für die Aminosäure L-Methionin kodierende ATG Kodon. Darüber hinaus kann die in SEQ ID NO: 5 dargestellte Nukleotidsequenz weitere Basenaustausche enthalten, die sich durch die Mutagenesebehandlung ergeben haben, sich jedoch nicht in einer veränderten Aminosäuresequenz äußern. Derartige Mutationen werden in der Fachwelt auch als stille oder neutrale Mutationen bezeichnet. Diese stillen Mutationen können ebenfalls in dem für Mutagenesebehandlung eingesetzten coryneformen Bakterium bereits enthalten sein. Bei den genannten Basenaustauschen handelt es sich beispielsweise um einen oder mehreren der Austausche ausgewählt aus der Gruppe: an der Position 93 Guanin anstelle Cytosin, an der Position 375 Adenin anstelle Guanin, an der Position 510 Adenin anstelle Guanin, an der Position 612 Guanin anstelle Cytosin, an der Position 786 Guanin anstelle Adenin, an der Position 813 Cytosin anstelle Thymin, an der Position 1014 Guanin anstelle Thymin, an der Position 1380 Cytosin anstelle Adenin, an der Position 1470 Guanin anstelle Thymin. In SEQ ID NO:17 ist die Nukleotidsequenz des gnd-Allels einer erfindungsgemäßen Mutante wiedergegeben, welche insgesamt sechs derartige stille Mutationen enthält.

Die Formulierung "an der Position 510 Adenin anstelle Guanin" - und vergleichbare Formulierungen - bedeutet, dass die in der Wildtypsequenz der Kodierregion an Position 510 vorhandene Nukleobase Guanin (Siehe SEQ ID NO:1) durch Adenin ersetzt worden ist (Siehe SEQ ID NO:17).

Die für die Mutagenese verwendeten coryneformen Bakterien besitzen bevorzugt bereits die Fähigkeit, die gewünschte Aminosäure in das sie umgebende Nährmedium beziehungsweise Fermentationsbrühe auszuscheiden oder im Zellinneren anzureichern.

L-Lysin produzierende coryneforme Bakterien besitzen typischerweise eine "feed back" resistente beziehungsweise desensibilisierte Aspartatkinase. Unter "feed back" resistenten Aspartatkinasen versteht man Aspartatkinasen, die im Vergleich zur Wildform eine geringere Empfindlichkeit gegenüber der Hemmung durch Mischungen von Lysin und Threonin oder Mischungen von AEC (Aminoethylcystein) und Threonin oder Lysin allein oder AEC allein aufweisen. Die für diese desensibilisierten Aspartatkinasen kodierenden Gene beziehungsweise Allele werden auch als lysC^{FBR}-Allele bezeichnet. Im Stand der Technik (Tabelle 1) sind zahlreiche lysC^{FBR}-Allele beschrieben, die für Aspartatkinase-Varianten kodieren, welche Aminosäureaustausche im Vergleich zum Wildtypprotein besitzen. Die Kodieregion des Wildtyp lysC-Gens von Corynebacterium glutamicum entsprechend der Zugangsnummer AX756575 der NCBI Datenbank ist in SEQ ID NO:15 und das von diesem Gen kodierte Protein in SEQ ID NO:16 dargestellt

**Tabelle 1**

| lysC^{FBR}-Allele kodierend für feed back resistente Aspartatkinasen | | | |
|---|---|---|---|
| Bezeichnung des Allels | Weitere Angaben | Referenz | Zugangsnummer |
| lysC^{FBR}-E05108 | | JP 1993184366-A (Sequenz 1) | E05108 |
| lysC^{FBR}-E06825 | lysC A279T | JP 1994062866-A (Sequenz 1) | E06825 |
| lysC^{FBR}-E06826 | lysC A279T | JP 1994062866-A (Sequenz 2) | E06826 |
| lysC^{FBR}-E06827 | | JP 1994062866-A (Sequenz 3) | E06827 |
| lysC^{FBR}-E08177 | | JP 1994261766-A (Sequenz 1) | E08177 |
| lysC^{FBR}-E08178 | lysC A279T | JP 1994261766-A (Sequenz 2) | E08178 |
| lysC^{FBR}-E08179 | lysC A279V | JP 1994261766-A (Sequenz 3) | E08179 |
| lysC^{FBR}-E08180 | lysC S301F | JP 1994261766-A (Sequenz 4) | E08180 |
| lysC^{FBR}-E08181 | lysC T308I | JP 1994261766-A (Sequenz 5) | E08181 |
| lysC^{FBR}-E08182 | | JP 1994261766-A (Sequenz 6) | E08182 |
| lysC^{FBR}-E12770 | | JP 1997070291-A (Sequenz 13) | E12770 |
| lysC^{FBR}-E14514 | | JP 1997322774-A (Sequenz 9) | E14514 |
| lysC^{FBR}-E16352 | | JP 1998165180-A (Sequenz 3) | E16352 |
| lysC^{FBR}-E16745 | | JP 1998215883-A (Sequenz 3) | E16745 |
| lysC^{FBR}-E16746 | | JP 1998215883-A (Sequenz 4) | E16746 |
| lysC^{FBR}-I74588 | lysC S317A | US 5688671-A (Sequenz 1) | 174588 |
| lysC^{FBR}-I74589 | lysC A279T | US 5688671-A (Sequenz 2) | I74589 |
| lysC^{FBR}-I74590 | | US 5688671-A (Sequenz 7) | I74590 |
| lysC^{FBR}-I74591 | lysC A279T | US 5688671-A (Sequenz 8) | I74591 |
| lysC^{FBR}-I74592 | | US 5688671-A (Sequenz 9) | I74592 |
| lysC^{FBR}-I74593 | lysC A279T | US 5688671-A (Sequenz 10) | I74593 |
| lysC^{FBR}-I74594 | | US 5688671-A (Sequenz 11) | I74594 |
| lysC^{FBR}-I74595 | lysC A279T | US 5688671-A (Sequenz 12) | I74595 |
| lysC^{FBR}-I74596 | | US 5688671-A (Sequenz 13) | I74596 |
| lysC^{FBR}-I74597 | lysC A279T | US 5688671-A (Sequenz 14) | I74597 |
| lysC^{FBR}-X57226 | lysC S301Y | EP0387527 Kalinowski et al., Molecular and General Genetics 224:317-324 (1990) | X57226 |
| lysC^{FBR}-L16848 | lysC G345D | Follettie and Sinskey NCBI Nucleotide Database (1990) | L16848 |
| lysC^{FBR}-L27125 | lysC R320G lysC G345D | Jetten et al., Applied Microbiology Biotechnology 43:76-82 (1995) | L27125 |
| lysC^{FBR} | lysC T311I | WO0063388 (Sequenz 17) | |
| lysC^{FBR} | lysC S301F | US3732144 | |
| lysC^{FBR} | lysC S381F | EP0435132 | |
| lysC^{FBR} | lysC S317A | US5688671 (Sequenz 1) | |
| lysC^{FBR} | lysC T380I | WO 01/49854 | |

L-Lysin ausscheidende coryneforme Bakterien besitzen typischerweise einen oder mehrere der in Tabelle 1 aufgelisteten Aminosäureaustausche.

Bevorzugt werden folgende lysC^{FBR}-Allele: lysC A279T (Austausch von Alanin an Position 279 des kodierten Aspartatkinaseproteins gemäß SEQ ID NO: 16 gegen Threonin), lysC A279V (Austausch von Alanin an Position 279 des kodierten Aspartatkinaseproteins gemäß SEQ ID NO: 16 gegen Valin), lysC S301F (Austausch von Serin an Position 301 des kodierten Aspartatkinaseproteins gemäß SEQ ID NO: 16 gegen Phenylalanin), lysC T308I (Austausch von Threonin an Position 308 des kodierten Aspartatkinaseproteins gemäß SEQ ID NO: 16 gegen Isoleucin), lysC S301Y (Austausch von Serin an Position 308 des kodierten Aspartatkinaseproteins gemäß SEQ ID NO: 16 gegen Tyrosin), lysC G345D (Austausch von Glycin an Position 345 des kodierten Aspartatkinaseproteins gemäß SEQ ID NO: 16 gegen Asparaginsäure), lysC R320G (Austausch von Arginin an Position 320 des kodierten Aspartatkinaseproteins gemäß SEQ ID NO: 16 gegen Glycin), lysC T311I (Austausch von Threonin an Position 311 des kodierten Aspartatkinaseproteins gemäß SEQ ID NO: 16 gegen Isoleucin), lysC S381F (Austausch von Serin an Position 381 des kodierten Aspartatkinaseproteins gemäß SEQ ID NO: 16 gegen Phenylalanin), lysC S317A (Austausch von Serin an Position 317 des kodierten Aspartatkinaseproteins gemäß SEQ ID NO: 16 gegen Alanin) und lysC T380I (Austausch von Threonin an Position 380 des kodierten Aspartatkinaseproteins gemäß SEQ ID NO: 22 gegen Isoleucin).

Besonders bevorzugt werden das lysC^{FBR}-Allel lysC T311I (Austausch von Threonin an Position 311 des kodierten Aspartatkinaseproteins gemäß SEQ ID NO: 16 gegen Isoleucin) und ein lysC^{FBR}-Allel enthaltend mindestens einen Austausch ausgewählt aus der Gruppe A279T (Austausch von Alanin an Position 279 des kodierten Aspartatkinaseproteins gemäß SEQ ID NO: 16 gegen Threonin) und S317A (Austausch von Serin an Position 317 des kodierten Aspartatkinaseproteins gemäß SEQ ID NO: 16 gegen Alanin).

Das lysC^{FBR}-Allel lysC T311I ist in dem bei der DSMZ hinterlegten Stamm DM1797 enthalten. DM1797 ist eine Mutante von Corynebacterium glutamicum ATCC13032.

Nach der oben beschriebenen Art und Weise wurde, ausgehend von Stamm DM1797, eine als DM1798 bezeichnete Mutante isoliert, die ein gnd-Allel enthält, das für ein Polypeptid kodiert, bei dem an Position 329 der Aminosäuresequenz L-Methionin enthalten ist. Die Nukleotidsequenz des gnd-Allels der Mutante DM1798 ist als SEQ ID NO:17 und die Aminosäuresequenz des kodierten Polypeptids als SEQ ID NO:18 dargestellt. Zusätzlich zu der zum Aminosäureaustausch an Position 329 der Aminosäuresequenz des kodierten Polypeptids führenden Guanin-Adenin Transition an Position 985 der Nukleotidsequenz gemäß SEQ ID NO:5 enthält das gnd-Allel der Mutante DM1798 folgende stille Mutationen:Guanin-Adenin Transition an Position 510, Cytosin-Guanin Transversion an Position 612, Adenin-Guanin Transition an Position 786, Thymin-Cytosin Transition an Position 813 und Thymin-Guanin Transversion an Position 1470.

In der SEQ ID NO:19 sind die stromaufwärts (upstream) und stromabwärts (downstream) von SEQ ID NO:17 gelegenen Nukleotidsequenzen mit angegeben.

Darüberhinaus können L-Lysin ausscheidende coryneforme Bakterien verwendet werden, die eine abgeschwächte Homoserin-Dehydrogenase oder Homoserinkinase aufweisen oder andere Eigenschaften besitzen, wie sie aus dem Stand der Technik bekannt sind.

L-Tryptophan produzierende coryneforme Bakterien besitzen typischerweise eine "feed back" resistente beziehungsweise desensibilisierte Anthranilatsynthase. Unter "feed back" resistenter Anthranilatsynthase versteht man Anthranilatsynthasen, die im Vergleich zur Wildform eine geringere Empfindlichkeit gegenüber der Hemmung (5 bis 10%, 10% bis 15% oder 10% bis 20%)durch Tryptophan oder 5-Fluorotryptophan (Matsui et al., Journal of Bacteriology 169 (11): 5330 - 5332(1987)) oder ähnliche Analoga. aufweisen. Die für diese desensibilisierten Anthranilatsynthasen kodierenden Gene beziehungsweise Allele werden auch als trpE^{FBR} -Allele bezeichnet. Beispiele für derartige Mutanten beziehungsweise Allele sind beispielsweise in der US 6180373 und EP0338474 beschrieben.

Die erhaltenen Mutanten zeigen eine im Vergleich zum eingesetzten Ausgangsstamm beziehungsweise Elternstamm erhöhte Ausscheidung beziehungsweise Produktion der gewünschten Aminosäure in einem Fermentationsprozess.

Gegenstand der Erfindung ist ebenfalls ein isoliertes Polynukleotid, das für ein Polypeptid mit 6-Phosphogluconat Dehydrogenase Enzymaktivität kodiert, welches an Position 329 der Aminosäuresequenz SEQ ID NO:2 jede proteinogene Aminosäure ausgenommen L-Valin enthält, wobei der Austausch gegen L-Methionin bevorzugt ist.

Das erfindungsgemäße Polynukleotid kann aus einer erfindungsgemäßen Mutante isoliert werden.

Weiterhin können für die Mutagenese des gnd-Gens in-vitro Methoden eingesetzt werden. Bei der Verwendung von in-vitro Methoden werden isolierte Polynukleotide, welche ein gnd-Gen eines coryneformen Bakteriums, vorzugsweise das im Stand der Technik beschriebene Wildtyp-Gen von Corynebacterium glutamicum, enthalten, einer mutagenen Behandlung unterzogen.

Bei den isolierten Polynukleotiden kann es sich beispielsweise um isolierte Gesamt-DNA beziehungsweise chromosomale DNA oder auch um Amplifikate des gnd-Gens handeln, die mit Hilfe der Polymerasekettenreaktion (PCR) hergestellt wurden. Derartige Amplifikate werden auch als PCR-Produkte bezeichnet; die Begriffe Nukleinsäuremolekül oder Nukleinsäurefragment sind ebenfalls gebräuchlich. Anleitungen zur Amplifikation von DNA-Sequenzen mit Hilfe der Polymerase-Kettenreaktion findet der Fachmann unter anderem im Handbuch von Gait: Oligonucleotide Synthesis: A Practical Approach (IRL Press, Oxford, UK, 1984) und bei Newton und Graham: PCR (Spektrum Akademischer Verlag, Heidelberg, Deutschland, 1994). Es ist ebenfalls möglich das zu mutagenisierende gnd-Gen zunächst in einen Vektor, beispielsweise in einen Bakteriophagen oder ein Plasmid einzubauen.

Geeignete Methoden für die in-vitro Mutagenese sind unter anderem die Behandlung mit Hydroxylamin nach Miller (Miller, J. H.: A Short Course in Bacterial Genetics. A Laboratory Manual and Handbook for Escherichia coli and Related Bacteria, Cold Spring Harbor Laboratory Press, Cold Spring Harbor, 1992), die Verwendung mutagener Oligonukleotide (T. A. Brown: Gentechnologie für Einsteiger, Spektrum Akademischer Verlag, Heidelberg, 1993 und R. M. Horton: PCR-Mediated Recombination and Mutagenesis, Molecular Biotechnology 3, 93-99 (1995)) und der Einsatz einer Polymerasekettenreaktion unter Verwendung einer DNA Polymerase, die eine hohe Fehlerquote aufweist. Eine derartige DNA-Polymerase ist beispielsweise die Mutazyme DNA Polymerase (GeneMorph PCR Mutagenesis Kit, Nr.600550) der Firma Stratagene (LaJolla, CA, USA).

Weitere Anleitungen und Übersichten zur Erzeugung von Mutationen in-vivo oder in-vitro können dem Stand der Technik und bekannten Lehrbüchern der Genetik und Molekularbiologie wie z.B. dem Lehrbuch von Knippers ("Molekulare Genetik", 6. Auflage, Georg Thieme Verlag, Stuttgart, Deutschland, 1995), dem von Winnacker ("Gene und Klone", VCH Verlagsgesellschaft, Weinheim, Deutschland, 1990) oder dem von Hagemann ("Allgemeine Genetik", Gustav Fischer Verlag, Stuttgart, 1986) entnommen werden.

Gegenstand der Erfindung ist weiterhin ein isoliertes Polynukleotid, dass für ein Polypeptid mit 6-Phosphogluconat Dehydrogenase Enzymaktivität kodiert, welches die Aminosäuresequenz von SEQ ID NO:2 umfasst, wobei an Position 329 der Aminosäuresequenz jede proteinogene Aminosäure ausgenommen L-Valin enthalten ist. Bevorzugt wird der Austausch gegen L-Methionin.

Gegenstand der Erfindung ist weiterhin ein isoliertes Polynukleotid, dass für ein Polypeptid mit 6-Phosphogluconat Dehydrogenase Enzymaktivität kodiert, welches eine Aminosäuresequenz mit einer Länge von 492 Aminoäuren umfasst und wobei an Position 329 von SEQ ID NO:2 jede proteinogene L-Aminosäure ausgenommen L-Valin, vorzugsweise L-Methionin, enthalten ist.

Gegenstand der Beschreibung ist weiterhin ein isoliertes Polynukleotid, dass für ein Polypeptid mit 6-Phosphogluconat Dehydrogenase Enzymaktivität kodiert, welches von Position 324 bis 334 der Aminosäuresequenz die Aminosäuresequenz entsprechend Position 324 bis 334 von SEQ ID NO:6 enthält. Bevorzugt enthält die Aminosäuresequenz des kodierten Polypeptids eine Aminosäuresequenz entsprechend Position 313 bis 345 von SEQ ID NO:6 oder Position 297 bis 361 von SEQ ID NO:6 oder Position 281 bis 377 von SEQ ID NO:6 oder Position 265 bis 393 von SEQ ID NO:6 oder Position 201 bis 457 von SEQ ID NO:6 oder Position 72 bis 492 von SEQ ID NO:6 oder Position 2 bis 492 von SEQ ID NO:6. Ganz besonders bevorzugt umfasst die Länge des kodierten Proteins 492 Aminosäuren.

Gegenstand der Beschreibung ist weiterhin ein isoliertes Polynukleotid, dass für ein Polypeptid mit 6-Phosphogluconat Dehydrogenase Enzymaktivität kodiert, welches an Position 329 der Aminosäuresequenz beziehungsweise einer entsprechenden oder vergleichbaren Position jede proteinogene Aminosäure ausgenommen L-Valin, bevorzugt L-Methionin, enthält und das eine Nukleotidsequenz umfasst, welche mit der Nukleotidsequenz eines Polynukleotids identisch ist, das durch eine Polymerasekettenreaktion (PCR) unter Verwendung des Primerpaars erhältlich ist, deren Nukleotidsequenzen jeweils mindestens 15 aufeinanderfolgende Nukleotide umfassen, die aus der Nukleotidsequenz zwischen Position 1 und 226 von SEQ ID NO:3 und aus der komplementären Nukleotidsequenz zwischen Position 1866 und 1703 von SEQ ID NO:3 ausgewählt werden. Zwei Beispiele für geeignete Primerpaare sind in SEQ ID NO:7 und SEQ ID NO:8 und in SEQ ID NO:9 und SEQ ID NO:10 dargestellt. Als Ausgangsmaterial ("template") wird chromosomale DNA coryneformer Bakterien bevorzugt, die insbesondere mit einem Mutagen behandelt worden sind. Besonders bevorzugt wird die chromosomale DNA der Gattung Corynebacterium und ganz besonders bevorzugt die der Art Corynebacterium glutamicum.

Gegenstand der Beschreibung ist weiterhin ein isoliertes Polynukleotid, das mit der zu SEQ ID NO:5 komplementären Nukleotidsequenz unter stringenten Bedingungen hybridisiert und für ein Polypeptid mit 6-Phosphogluconat Dehydrogenase Enzymaktivität kodiert, welches an Position 329 der Aminosäuresequenz beziehungsweise einer entsprechenden oder vergleichbaren Position jede proteinogene Aminosäure ausgenommen L-Valin, bevorzugt L-Methionin, enthält.

Anleitungen zur Hybridisierung von Nukleinsäuren beziehungsweise Polynukleotiden findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology 41: 255-260 (1991)). Die Hybridisierung findet unter stringenten Bedingungen statt, das heißt, es werden nur Hybride gebildet, bei denen die Sonde d.h. ein Polynukleotid umfassend die zu SEQ ID NO:5 komplementäre Nukleotidsequenz und die Zielsequenz, d. h. die mit der Sonde behandelten Polynukleotide, mindestens 90% identisch sind. Es ist bekannt, dass die Stringenz der Hybridisierung einschließlich der Waschschritte durch Variieren der Pufferzusammensetzung, der Temperatur und der Salzkonzentration beeinflusst bzw. bestimmt wird. Die Hybridisierungsreaktion wird im Allgemeinen bei relativ niedriger Stringenz im Vergleich zu den Waschschritten durchgeführt (Hybaid Hybridisation Guide, Hybaid Limited, Teddington, UK, 1996).

Für die Hybridisierungsreaktion kann beispielsweise ein Puffer entsprechend 5x SSC-Puffer bei einer Temperatur von ca. 50°C - 68°C eingesetzt werden. Dabei können Sonden auch mit Polynukleotiden hybridisieren, die weniger als 90% Identität zur Nukleotidequenz der eingesetzten Sonde aufweisen. Solche Hybride sind weniger stabil und werden durch Waschen unter stringenten Bedingungen entfernt. Dies kann beispielsweise durch Senken der Salzkonzentration auf 2x SSC und gegebenenfalls nachfolgend 0,5x SSC (The DIG System User's Guide for Filter Hybridisation, Boehringer Mannheim, Mannheim, Deutschland, 1995) erreicht werden, wobei eine Temperatur von ca. 50°C - 68°C, ca. 52°C - 68°C, ca. 54°C - 68°C, ca. 56°C - 68°C, ca. 58°C - 68°C, ca. 60°C - 68°C, ca. 62°C - 68°C, ca. 64°C - 68°C, ca. 66°C - 68°C eingestellt wird. Temperaturbereiche von ca. 64°C - 68°C oder ca. 66°C - 68°C werden bevorzugt. Es ist gegebenenfalls möglich, die Salzkonzentration bis auf eine Konzentration entsprechend 0,2x SSC oder 0,1x SSC zu senken. Gegebenenfalls enthält der SSC-Puffer Natriumdodecylsulfat (SDS) in einer Konzentration von 0,1%. Durch schrittweise Erhöhung der Hybridisierungstemperatur in Schritten von ca. 1 - 2°C von 50°C auf 68°C können Polynukleotidfragmente isoliert werden, die mindestens 90% oder mindestens 91%, bevorzugt mindestens 92% oder mindestens 93% oder mindestens 94% oder mindestens 95% oder mindestens 96% und ganz besonders bevorzugt mindestens 97% oder mindestens 98% oder mindestens 99% Identität zur Sequenz der eingesetzten Sonde besitzen und für ein Polypeptid mit Phosphogluconat Dehydrogenase Enzymaktivität kodieren, welches den erfindungsgemäßen Aminosäureaustausch enthält. Die Nukleotidsequenz des auf diese Weise erhaltenen Polynukleotids wird mit bekannten Methoden bestimmt. Weitere Anleitungen zur Hybridisierung sind in Form sogenannter Kits am Markt erhältlich (z.B. DIG Easy Hyb von der Firma Roche Diagnostics GmbH, Mannheim, Deutschland, Catalog No. 1603558). Die so erhaltenen Nukleotidsequenzen kodieren für Polypeptide mit Phosphogluconat Dehydrogenase Enzymaktivität, die mindestens 90% bevorzugt mindestens 92% oder mindestens 94% oder mindestens 96%, und ganz besonders bevorzugt mindestens 97% oder mindestens 98% oder mindestens 99% identisch sind mit der Aminosäuresequenz von SEQ ID NO:6 und den erfindungsgemäßen Aminosäureaustausch enthalten.

Gegenstand der Erfindung ist weiterhin ein isoliertes Polynukleotid, das für ein Polypeptid mit 6-Phosphogluconat Dehydrogenase Enzymaktivität kodiert, welches an Position 329 von SEQ ID NO:2 der Aminosäuresequenz L-Methionin enthält, und das eine Aminosäuresequenz umfasst, die außerdem zu mindestens 98% oder mindestens 99% identisch ist mit der Aminosäuresequenz von SEQ ID NO:6.

Gegenstand der Erfindung ist weiterhin ein isoliertes Polynukleotid, das für ein Polypeptid mit 6-Phosphogluconat Dehydrogenase Enzymaktivität kodiert, welches an Position 329 der Aminosäuresequenz SEQ ID NO:2 L-Methionin enthält, und das eine Nukleotidsequenz umfasst, die außerdem zu mindestens 99% identisch ist mit der Nukleotidsequenz von SEQ ID NO:5.

Darüberhinaus sind solche isolierte Polynukleotide bevorzugt, die für ein Polypeptid mit 6-Phosphogluconat Dehydrogenase Enzymaktivität kodieren, welches an Position 329 der Aminosäuresequenz von SEQ ID NO:2 jede Aminosäure ausgenommen L-Valin, bevorzugt L-Methionin, enthält und die mindestens ein Sequenzmotiv beziehungsweise eine Aminosäuresequenz ausgewählt aus der Gruppe Leu-Bra-Asp-Val-Ile-Val-Asp und Ile-Aro-Arg-Ali-Gly-Cys-Ile-Ile-Arg-Ala umfasst. Die Bezeichnung "Bra" steht für die Aminosäuren Ile oder Val, "Aro" für Trp oder Phe und die Bezeichnung "Ali" für die Aminosäuren Gly oder Ala.

Gegenstand der Erfindung ist weiterhin ein isoliertes Polynukleotid, dass für ein Polypeptid mit 6-Phosphogluconat Dehydrogenase Enzymaktivität kodiert, welches die Aminosäuresequenz von SEQ ID NO:6 umfasst. Gegebenenfalls enthält das kodierte Polypeptid einen (1), bevorzugt höchstens zwei (2), höchstens drei (3), höchstens vier (4)oder höchstens fünf (5) konservative Aminosäureaustausche enthält.

Gegenstand der Erfindung ist weiterhin ein isoliertes Polynukleotid, dass für ein Polypeptid mit 6-Phosphogluconat Dehydrogenase Enzymaktivität kodiert, welches die Aminosäuresequenz von SEQ ID NO:6 einschließlich einer Verlängerung am N- oder C-Terminus um mindestens eine (1) Aminosäure umfasst. Diese Verlängerung beträgt nicht mehr als 5, 3 oder 2 Aminosäuren beziehungsweise Aminosäurereste.

Gegenstand der Erfindung sind schließlich auch gnd-Allele, die für Polypeptid Varianten von SEQ ID NO:6 kodieren, die eine Insertion oder Deletion enthalten. Bevorzugt enthalten diese maximal 5, maximal 4, maximal 3 oder maximal 2 Insertionen oder Deletionen von Aminosäuren. Die Sequenzmotive Leu-Bra-Asp-Val-Ile-Val-Asp und Ile-Aro-Arg-Ali-Gly-Cys-Ile-Ile-Arg-Ala werden durch derartige Insertionen/Deletionen vorzugsweise nicht zerrissen.

Gegenstand der Erfindung ist weiterhin ein isoliertes Polynukleotid, das die Nukleotidsequenz gemäß SEQ ID NO:5 umfasst, wobei diese gegebenenfalls zusätzlich einen oder mehrere der Basenaustausche umfasst ausgewählt aus der Gruppe: an der Position 93 Guanin anstelle Cytosin, an der Position 375 Adenin anstelle Guanin, an der Position 510 Adenin anstelle Guanin, an der Position 612 Guanin anstelle Cytosin, an der Position 786 Guanin anstelle Adenin, an der Position 813 Cytosin anstelle Thymin, an der Position 1014 Guanin anstelle Thymin, an der Position 1380 Cytosin anstelle Adenin, an der Position 1470 Guanin anstelle Thymin.

Gegenstand der Erfindung ist schließlich ein isoliertes Polynukleotid enthaltend das gnd-Allel der Mutante DM1798. Die Nukleotidsequenz ist als SEQ ID NO:17 und die Aminosäuresequenz des kodierten Polypeptids als SEQ ID NO:18 dargestellt. Zusätzlich zu der zum Aminosäureaustausch an Position 329 der Aminosäuresequenz SEQ ID NO:2 des kodierten Polypeptids führenden Guanin-Adenin Transversion an Position 985 der Nukleotidsequenz gemäß SEQ ID NO:5 enthält das gnd-Allel der Mutante DM1798 folgende stille Mutationen:Guanin-Adenin Transition an Position 510, Cytosin-Guanin Transversion an Position 612, Adenin-Guanin Transition an Position 786, Thymin-Cytosin Transition an Position 813 und Thymin-Guanin Transversion an Position 1470.

Gegenstand der Erfindung ist außerdem ein isoliertes Polynukleotid, das einen Teil der Kodierregion eines erfindungsgemäßen gnd-Allels umfasst, wobei das isolierte Polynukleotid in jedem Fall den Teil der Kodierregion umfasst, der den Aminosäureaustausch an der Position 329 der Aminosäuresequenz des kodierten Polypeptids enthält.

Insbesondere ist ein Nukleinsäure-Molekül beziehungsweise DNA-Fragment umfasst, das für mindestens eine Aminosäuresequenz entsprechend Position 313 bis 345 von SEQ ID NO:2, oder das für mindestens eine Aminosäuresequenz entsprechend Position 297 bis 361 von SEQ ID NO:2, oder das für mindestens eine Aminosäuresequenz entsprechend Position 281 bis 377 von SEQ ID NO:2, oder das für mindestens eine Aminosäuresequenz entsprechend Position 265 bis 393 von SEQ ID NO:2, oder das für mindestens eine Aminosäuresequenz entsprechend Position 201 bis 457 von SEQ ID NO:2, oder das für mindestens eine Aminosäuresequenz entsprechend Position 102 bis 492 von SEQ ID NO:2, oder das für mindestens eine Aminosäuresequenz entsprechend Position 72 bis 492 von SEQ ID NO:2 kodiert, beziehungsweise ein entsprechendes Leseraster beinhaltet, wobei an der Position entsprechend 329 von SEQ ID NO:2 jede proteinogene Aminsäure ausgenommen L-Valin, bevorzugt L-Methionin, enthalten ist.

Ein Beispiel eines erfindungsgemäßen Leserasters umfassend ein Polynukleotid, das für mindestens die Aminosäuresequenz von Position 313 bis 345 entsprechend SEQ ID NO:2 kodiert, wobei an der Position entsprechend 329 der Aminosäuresequenz jede proteinogene Aminosäure ausgenommen L-Valin enthalten ist, ist nachfolgend aufgeführt:

Es ist ebenfalls als SEQ ID NO:11 dargestellt. Die von diesem Leseraster kodierte Aminosäuresequenz ist als SEQ ID NO:12 dargestellt.

Bevorzugt werden Nukleinsäuremoleküle, die für mindestens eine Aminosäuresequenz entsprechend Position 313 bis 345 von SEQ ID NO:6, oder mindestens entsprechend Position 297 bis 361 von SEQ ID NO:6, oder mindestens entsprechend Position 281 bis 377 von SEQ ID NO:6, oder mindestens entsprechend Position 265 bis 393 von SEQ ID NO:6, oder mindestens entsprechend Position 201 bis 457 von SEQ ID NO:6, oder mindestens entsprechend Position 102 bis 492 von SEQ ID NO:6, oder mindestens entsprechend Position 72 bis 492 von SEQ ID NO:6 kodieren.

Ein Beispiel eines erfindungsgemäßes Leserasters umfassend ein Polynukleotid, das für mindestens die Aminosäuresequenz entprechend Position 313 bis 345 von SEQ ID NO:5 kodiert, ist nachfolgend aufgeführt:

Es ist ebenfalls als SEQ ID NO:13 dargestellt. SEQ ID NO:14 zeigt die von diesem Leseraster kodierte Aminosäuresequenz.

Ganz besonders bevorzugt werden Nukleinsäuremoleküle, die mindestens eine Nukleotidsequenz entsprechend Position 937 bis 1035 von SEQ ID NO:5 oder SEQ ID NO:17, oder mindestens eine Nukleotidsequenz entsprechend Position 889 bis 1083 von SEQ ID NO:5 oder SEQ ID NO:17, oder mindestens eine Nukleotidsequenz entsprechend Position 841 bis 1131 von SEQ ID NO:5 oder SEQ ID NO:17, oder mindestens eine Nukleotidsequenz entsprechend Position 793 bis 1179 von SEQ ID NO:5 oder SEQ ID NO:17, oder mindestens eine Nukleotidsequenz entsprechend Position 601 bis 1372 von SEQ ID NO:5 oder SEQ ID NO:17, oder mindestens eine Nukleotidsequenz entsprechend Position 304 bis 1476 von SEQ ID NO:5 oder SEQ ID NO:17, oder mindestens eine Nukleotidsequenz entsprechend Position 214 bis 1476 von SEQ ID NO:5 oder SEQ ID NO:17 umfassen.

Die erfindungsgemäßen Leseraster, wie sie beispielhaft in SEQ ID NO:11 und 13 als Nukleotidsequenz und in SEQ ID NO:12 und SEQ ID NO:14 als kodierte Aminosäuresequenz gezeigt sind, können darüberhinaus eine oder mehrere Mutationen enthalten, die zu einem oder mehreren konservativen Aminosäureaustauschen führen. Bevorzugt führen die Mutationen zu maximal 4%, zu maximal 2% oder zu maximal 1% konservativen Aminosäureaustauschen. Weiterhin können die erfindungsgemäßen Leseraster eine oder mehrere stille Mutationen enthalten. Bevorzugt enthalten die erfindungsgemäßen Leseraster höchstens 4% und besonders bevorzugt höchstens 2% bis höchstens 1% stille Mutationen.

Die erfindungsgemäßen, isolierten Polynukleotide können dazu verwendet werden, um rekombinante Stämme von Mikroorganismen herzustellen, die im Vergleich zum Ausgangs- beziehungsweise Elternstamm in verbesserter Weise Aminosäuren in das sie umgebende Medium abgeben oder im Zellinneren anhäufen.

Eine verbreitete Methode zum Einbau von Mutationen in Gene coryneformer Bakterien ist die des Allelaustausches, die auch unter der Bezeichnung "gene replacement" bekannt ist. Bei diesem Verfahren wird ein DNA-Fragment, welches die interessierende Mutation enthält, in den gewünschten Stamm eines coryneformen Bakteriums überführt und die Mutation durch wenigstens zwei Rekombinationsereignisse beziehungsweise "cross over"-Ereignisse in das Chromosom des gewünschten Stammes inkorporiert beziehungsweise die im betreffenden Stamm vorhandene Sequenz eines Gens gegen die mutierte Sequenz ausgetauscht.

Von Schwarzer und Pühler (Bio/Technology 9, 84-87 (1991) wurde diese Methode verwendet um ein lysA-Allel, welches eine Deletion trug, und um ein lysA-Allel, welches eine Insertion trug in das Chromosom von C. glutamicum anstelle des Wildtypgens einzubauen. Von Schäfer et al. (Gene 145, 69-73 (1994)) wurde diese Methode eingesetzt um eine Deletion in das hom-thrB Operon von C. glutamicum einzubauen. Von Nakagawa et al. (EP 1108790) wurde diese Methode eingesetzt um verschiedene Mutationen ausgehend von den isolierten Allelen in das Chromosom von C. glutamicum einzubauen. Auf diese Weise gelang es Nakagawa et al. eine als Val59Ala bezeichnete Mutation in das Homoserin-Dehydrogenase Gen (hom), eine als Thr311Ile bezeichnete Mutation in das Aspartatkinase-Gen (lysC bzw. ask), eine als Pro458Ser bezeichnete Mutation in das Pyruvat-Carboxylase-Gen (pyc) und eine als Ala213Thr bezeichnete Mutzation in das Glucose-6-phoshat-Dehydrogenase Gen (zwf) von C. glutamicum Stämmen einzubauen.

Für ein erfindungsgemäßes Verfahren kann ein erfindungsgemäßes Polynukleotid verwendet werden, welches die gesamte Kodierregion umfasst, wie beispielsweise in SEQ ID NO:5 gezeigt, oder welches einen Teil der Kodierregion umfasst, wie beispielsweise die Nukleotidsequenz, die für mindestens die Aminosäuresequenz entsprechend Position 313 bis 345 von SEQ ID NO:6 kodiert und die als SEQ ID NO:14 dargestellt ist. Der Teil der Kodierregion entsprechend SEQ ID NO:14 hat eine Länge von 99 Nukleobasen. Bevorzugt werden solche Teile der Kodierregion, deren Länge ≥ 195 Nukleobasen beträgt, wie beispielsweise Nukleinsäuremoleküle, die für mindestens eine Aminosäuresequenz entsprechend Position von 297 bis 361 von SEQ ID NO:6 oder SEQ ID NO:18 kodieren. Besonders bevorzugt werden solche Teile der Kodierregion, deren Länge ≥ 291 Nukleobasen beträgt, wie beispielsweise Nukleinsäuremoleküle, die für mindestens eine Aminosäuresequenz entsprechend Position von 281 bis 377 von SEQ ID NO:6 oder SEQ ID NO:18 kodieren.

Das DNA-Fragment enthaltend die interessierende Mutation liegt bei dieser Methode typischerweise in einem Vektor, insbesondere einem Plasmid, vor, das vorzugsweise von dem mit der Mutation zu versehenden Stamm nicht oder nur begrenzt repliziert wird. Als Hilfs- oder Zwischenwirt, in dem der Vektor replizierbar ist, wird im Allgemeinen ein Bakterium der Gattung Escherichia bevorzugt der Spezies Escherichia coli verwendet.

Beispiele für derartige Plasmidvektoren sind die von Schäfer et al. (Gene 145, 69-73 (1994)) beschriebenen pK*mob und pK*mobsacB Vektoren, wie beispielsweise pK18mobsacB, und die in der WO 02/070685 und WO 03/014362 beschriebenen Vektoren. Diese sind in Escherichia coli aber nicht in coryneformen Bakterien replikativ. Besonders geignet sind Vektoren, die ein konditional negativ dominant wirkendes Gen wie beispielsweise das sacB-Gen (Levansucrase-Gen) von beispielsweise Bacillus oder das galK-Gen (Galaktosekinase) von beispielsweise Escherichia coli enthalten. (Unter einem konditional negativ dominant wirkenden Gen versteht man ein Gen, das unter bestimmten Bedingungen nachteilig beispielsweise toxisch für den Wirt ist, unter anderen Bedingungen aber keine negativen Auswirkungen auf den das Gen tragenden Wirt hat.) Diese ermöglichen die Selektion auf Rekombinationsereignisse, bei denen der Vektor aus dem Chromosom eliminiert wird. Weiterhin wurde von Nakamura et al. (US-A-6,303,383) ein Temperatur-sensitives Plasmid für coryneforme Bakterien beschrieben, das lediglich bei Temperaturen unterhalb von 31°C replizieren kann.

Der Vektor wird anschließend durch Konjugation beispielsweise nach der Methode von Schäfer (Journal of Bacteriology 172, 1663-1666 (1990)) oder Transformation beispielsweise nach der Methode von Dunican und Shivnan (Bio/Technology 7, 1067-1070 (1989)) oder der Methode von Thierbach et al. (Applied Microbiology and Biotechnology 29, 356-362 (1988)) in das coryneforme Bakterium überführt. Gegebenenfalls kann die Überführung der DNA auch durch Partikelbeschuss erzielt werden.

Nach homologer Rekombination mittels eines ersten, Integration bewirkenden "cross-over"-Ereignisses und eines geeigneten zweiten, eine Exzision bewirkenden "cross-over"-Ereignisses im Zielgen bzw. in der Zielsequenz erreicht man den Einbau der Mutation und erhält ein rekombinantes Bakterium.

Zur Identifizierung und Charakterisierung der erhaltenen Stämme können unter anderem die Methoden der Southern Blotting Hybridisierung, der Polymerase-Kettenreaktion, der Sequenzbestimmung, die Methode des "Fluorescence Resonance Energy Transfer" (FRET) (Lay et al. Clinical Chemistry 43, 2262-2267 (1997)) oder Methoden der Enzymologie eingesetzt werden.

Ein weiterer Gegenstand der Beschreibung ist dementsprechend ein Verfahren zur Herstellung eines coryneformen Bakteriums, bei dem man
a) ein erfindungsgemäßes Polynukleotid in ein coryneformes Bakterium überführt,
b) das im Chromosom des coryneformen Bakteriums vorhandene 6-Phosphogluconat Dehydrogenase Gen, das für eine Aminosäuresequenz SEQ ID NO:2 mit L-Valin an Position 329 kodiert, gegen das Polynukleotid aus a) austauscht, das für eine Aminosäuresequenz kodiert, die an der Position 329 der Aminosäuresequenz eine andere L-Aminosäure, vorzugsweise L-Methionin besitzt, und
c) das gemäß Schritt a) und b) erhaltene coryneforme Bakterium vermehrt.

Auf diese Weise erhält man ein rekombinantes coryneformes Bakterium, welches anstelle des Wildtyp gnd-Gens ein erfindungsgemäßes gnd-Allel enthält.

Ein weiteres beschriebenes, Verfahren zur Herstellung eines Mikroorganismus besteht darin, dass man
a) ein erfindungsgemäßes Polynukleotid, das für ein Polypeptid mit 6-Phosphogluconat Dehydrogenase Enzymaktivität kodiert, in einen Mikrorganismus überführt,
b) das Polynukleotid in dem Mikroorganismus repliziert, und
c) den gemäß Schritt a) und b) erhaltenen Mikroorganismus vermehrt.

Auf diese Weise erhält man einen rekombinanten Mikroorganismus, welcher mindestens eine (1) Kopie oder mehrere Kopien eines erfindungsgemäßen Polynukleotids enthält, das für eine 6-Phosphogluconat Dehydrogenase kodiert, die an Position 329 oder einer vergleichbaren Position der Aminosäuresequenz des kodierten Proteins, jede proteinogene Aminosäure ausgenommen L-Valin enthält, wobei der Austausch gegen L-Methionin bevorzugt wird.

Weitere Gegenstände der Erfindung sind dementsprechend Wirte beziehungsweise Wirtszellen, bevorzugt Mikroorganismen besonders bevorzugt coryneforme Bakterien und Bakterien der Gattung Escherichia, die die erfindungsgemäßen Polynukleotide enthalten. Gegenstand der Erfindung sind gleichfalls Mikrorganismen, die unter Verwendung der isolierten Polynukleotide hergestellt wurden. Derartige Mikroorganismen oder Bakterien werden auch als rekombinante Mikroorganismen oder rekombinante Bakterien bezeichnet. In gleicher Weise sind Vektoren, die die erfindungsgemäßen Polynukleotide enthalten, Gegenstand der Erfindung. Schließlich sind Wirte, die diese Vektoren enthalten, ebenfalls Gegenstand der Erfindung.

Die erfindungsgemäßen, isolierten Polynukleotide können weiterhin zur Erzielung einer Überexpression der von ihnen kodierten Polypeptide verwendet werden.

Unter Überexpression versteht man allgemein eine Erhöhung der intrazellulären Konzentration oder Aktivität einer Ribonukleinsäure, eines Proteins oder eines Enzyms. Im Falle der vorliegenden Erfindung werden gnd-Allele gemäß Anspruch 1 beziehungsweise Polynukleotide überexprimiert, die für 6-Phosphogluconat Dehydrogenasen kodieren, die an Position 329 der Aminosäuresequenz des kodierten Proteins jede proteinogene Aminosäure ausgenommen L-Valin enthalten, wobei der Austausch gegen L-Methionin bevorzugt wird.

Es ist bekannt, dass durch wirtseigene Enzyme - sogenannte Aminopeptidasen - N-terminale Aminosäuren, insbesondere das N-terminale Methionin, vom gebildeten Polypeptid abgespalten werden können.

Die genannte Erhöhung der Konzentration oder Aktivität eines Genproduktes lässt sich beispielsweise dadurch erzielen, dass man die Kopienzahl der entsprechenden Polynukleotide um mindestens eine Kopie erhöht.

Eine weit verbreitete Methode zur Erhöhung der Kopienzahl besteht darin, dass man das entsprechende Gen oder Allel in einen Vektor, bevorzugt ein Plasmid, einbaut, der von einem coryneformen Bakterium repliziert wird. Geeignete Plasmidvektoren sind beispielsweise pZ1 (Menkel et al., Applied and Environmental Microbiology (1989) 64: 549-554) oder die von Tauch et al. (Journal of Biotechnology 99, 79-91 (2002)) beschriebenen pSELF-Vektoren. Ein Übersichtsartikel zum Thema Plasmide in Corynebacterium glutamicum findet sich bei Tauch et al. (Journal of Biotechnology 104, 27-40 (2003)).

Eine andere verbreitete Methode zur Erzielung einer Überexpression ist das Verfahren der chromosomalen Genamplifikation. Bei dieser Methode wird mindestens eine zusätzliche Kopie des interessierenden Gens oder Allels in das Chromosom eines coryneformen Bakteriums eingefügt.

In einer Ausführungsform, wie sie beispielsweise bei Reinscheid et al. (Applied and Environmental Microbiology 60, 126-132) für das hom-thrB-Operon beschrieben ist, wird ein in C. glutamicum nicht-replikatives Plasmid, welches das interessierende Gen enthält,in ein coryneformes Bakterium überführt. Nach homologer Rekombination mittels eines "cross over"-Ereignisses enthält der resultierende Stamm mindestens zwei Kopien des betreffenden Gens beziehungsweise Allels.

In einer anderen Ausführungsform, die in der WO 03/040373 und US-2003-0219881-A1 beschrieben ist, wird eine oder mehrere Kopie(n) des interessierenden Gens mittels mindestens zweier Rekombinationsereignisse in einen gewünschten Ort des Chromosoms von C. glutamicum eingefügt. Auf diese Weise wurde beispielsweise eine Kopie eins lysC-Allels, das für eine L-Lysin insensitive Aspartatkinase kodiert, in das gluB-Gen von C. glutamicum eingebaut.

In einer weiteren Ausführungsform, die in der WO 03/014330 und US-2004-0043458-A1 beschrieben ist, wird mittels mindestens zweier Rekombinationsereignisse am natürlichen Ort mindestens eine weitere Kopie, vorzugsweise in tandem-Anordnung zu dem bereits vorhandenen Gen oder Allel, des interessierenden Gens eingebaut. Auf diese Weise wurde beispielsweise eine tandem-Duplikation eines lysC^{FBR}-Allels am natürlichen lysC-Genort erzielt.

Eine weitere Methode zur Erzielung einer Überexpression besteht darin, das entsprechende Gen beziehungsweise Allel in funktioneller Weise (operably linked) mit einem Promotor beziehungsweise einer Expressionskassette zu verknüpfen. Geeignete Promotoren für Corynebacterium glutamicum sind beispielsweise in dem Übersichtsartikel von Patek et al. (Journal of Biotechnology 104(1-3), 311-323 (2003) beschrieben. Weiterhin können die hinlänglich bekannten von Amann et al. (Gene 69(2), 301-315 (1988)) und Amann und Brosius (Gene 40(2-3), 183-190 (1985)) beschriebenen Promotoren T3, T7, SP6, M13, lac, tac und trc verwendet werden. Ein derartiger Promotor kann beispielsweise stromaufwärts des gnd-Allels, typischerweise im Abstand von ungefähr 1 - 500 Nukleotiden vom Startkodon, eines rekombinanten coryneformen Bakteriums eingefügt werden, welches anstelle der an Position 329 natürlicherweise vorhanden Aminosäure L-Valin eine andere proteinogene Aminsäure enthält. Ein derartiger Promotor kann naturgemäß ebenfalls stromaufwärts des gnd-Allels einer erfindungsgemäßen Mutante eingefügt werden. Es ist weiterhin möglich ein erfindungsgemäßes isoliertes Polynukleotid, das für eine erfindungsgemäße Variante der 6-Phosphogluconat Dehydrogenase kodiert, mit einem Promotor zu verknüpfen und die erhaltene Expressionseinheit in ein extrachromosomal replizierendes Plasmid oder in das Chromosom eines coryneformen Bakteriums einzubauen.

Darüberhinaus kann die Promotor- und Regulationsregion oder die Ribosomenbindungsstelle, die sich stromaufwärts des Strukturgens befindet, mutiert werden. Durch Maßnahmen zur Verlängerung der Lebensdauer der m-RNA wird ebenfalls die Expression verbessert. Weiterhin wird durch Verhinderung des Abbaus des Enzymproteins ebenfalls die Enzymaktivität verstärkt. Alternativ kann weiterhin eine Überexpression des betreffenden Gens oder Allels durch Veränderung der Medienzusammensetzung und Kulturführung erreicht werden.

Durch die Maßnahmen der Überexpression wird die Aktivität oder Konzentration des entsprechenden Proteins/Polypeptids im allgemeinen um mindestens 10%, 25%, 50%, 75%, 100%, 150%, 200%, 300%, 400% oder 500%, maximal bis 1000% oder 2000%, bezogen auf die Aktivität oder Konzentration des Proteins im Ausgangs-Mikroorganismus beziehungsweise Elternstammes erhöht. Unter einem Ausgangs-Mikroorganismus oder Elternstamm versteht man einen Mikroorganismus, an dem die Massnahmen der Erfindung durchgeführt werden.

Eine Methode zur Bestimmung der enzymatischen Aktivität der 6-Phosphogluconat Dehydrogenase ist bei Moritz et al. (European Journal of Biochemistry 267, 3442-3452 (2000)) beschrieben.

Die Konzentration des Proteins kann über 1- und 2-dimensionale Proteingelauftrennung und anschließende optische Identifizierung der Proteinkonzentration mit enstprechender Auswertesoftware im Gel bestimmt werden. Eine gebräuchliche Methode zur Präparation der Proteingele bei coryneformen Bakterien und zur Identifizierung der Proteine ist die von Hermann et al. (Electrophoresis, 22:1712-23 (2001)) beschriebene Vorgehensweise. Die Proteinkonzentration kann ebenfalls durch Western-Blot-Hybridisierung mit einem für das nachzuweisende Protein spezifischen Antikörper (Sambrook et al., Molecular cloning: a laboratory manual. 2nd Ed. Cold Spring Harbor Laboratory Press, Cold Spring Harbor, N.Y., 1989) und anschließender optischer Auswertung mit ensprechender Software zur Konzentrationsbestimmung (Lohaus und Meyer (1998) Biospektrum 5:32-39; Lottspeich, Angewandte Chemie 111: 2630-2647 (1999)) bestimmt werden.

Gegenstand der Erfindung sind dementsprechend Verfahren zur Überexpression der erfindungsgemäßen 6-Phosphogluconat Dehydrogenasen. Ein erfindungsgemässes Verfahren zur Überexpression besteht unter anderem darin, dass man die Kopienzahl eines erfindungsgemässen Polynukleotids, das für eine 6-Phosphogluconat Dehydrogenase Variante gemäß Anspruch 1 kodiert, bei der an Position 329 oder entsprechenden Position der kodierten Aminosäuresequenz jede proteinogene Aminosäure ausgenommen L-Valin enthalten ist, um mindestens eine (1) oder um mehrere Kopien erhöht. Ein weiteres erfindungsgemässes Verfahren besteht darin, dass man einen Promotor funktionell mit dem Polynukleotid verknüpft.

Gegenstand der Erfindung sind weiterhin Mikroorganismen, die eine erhöhte Konzentration oder Aktivität der erfindungsgemäßen 6-Phosphogluconat Dehydrogenase Varianten in ihrem Zellinneren aufweisen.

Zusätzlich kann es für die verbesserte Produktion von L-Aminosäuren vorteilhaft sein, in den erfindungsgemäßen Mutanten oder rekombinanten Stämme eines oder mehrere Enzyme des jeweiligen Biosyntheseweges, der Glykolyse, der Anaplerotik, des Zitronensäure-Zyklus, des Pentosephosphat-Zyklus, des Aminosäure-Exports und gegebenenfalls regulatorische Proteine überzuexprimieren. Die Verwendung endogener Gene wird im allgemeinen bevorzugt.

Unter "endogenen Genen" oder "endogenen Nukleotidsequenzen" versteht man die in der Population einer Art vorhandenen Gene beziehungsweise Nukleotidsequenzen oder Allele.

So kann für die Herstellung von L-Lysin eines oder mehrere der Gene, ausgewählt aus der Gruppe
- ein für ein Dihydrodipicolinat-Synthase kodierendes Gen dapA, wie beispielsweise das in der EP 0 197 335 beschriebene dapA-Gen des Wildtyps von Corynebacterium glutamicum,
- ein für eine Glucose-6-Phosphat Dehydrogenase kodierendes Gen zwf, wie beispielsweise das in der JP-A-09224661 und EP-A-1108790 beschriebene zwf-Gen des Wildtyps von Corynebacterium glutamicum,
- die in der US-2003-0175911-A1 beschriebenen zwf-Allele von Corynebacterium glutamicum, die für ein Protein kodieren, bei dem beispielsweise das L-Alanin an Position 243 der Aminosäuresequenz durch L-Threonin ersetzt ist oder bei dem die L-Asparaginsäure an Position 245 durch L-Serin ersetzt ist,
- ein für eine Pyruvat-Carboxylase kodierendes Gen pyc, wie beispielsweise das in der DE-A-198 31 609 und EP 1108790 beschriebene pyc-Gen des Wildtyps von Corynbebacterium glutamicum,
- das für das in der EP 1 108 790 beschriebene pyc-Allel von Corynebacterium glutamicum, das für ein Protein kodiert, bei dem L-Prolin an Position 458 der Aminosäuresequenz durch L-Serin ersetzt ist, und
- die in der WO 02/31158 beschriebene pyc-Allele von Corynebacterium glutamicum, die für Proteine kodieren, welche gemäß Aspruch 1 einen oder mehrere der Aminosäureaustausche ausgewählt aus der Gruppe L-Glutaminsäure an Position 153 ersetzt durch L-Asparaginsäure, L-Alanin an Position 182 ersetzt durch L-Serin, L-Alanin an Position 206 ersetzt durch L-Serin, L-Histidin an Position 227 ersetzt durch L-Arginin, L-Alanin an Position 452 ersetzt durch Glycin und L-Asparaginsäure an Position 1120 ersetzt durch L-Glutaminsäure tragen (Figur 2A von WO 02/31158 gibt zwei verschiedene Startpositionen für die Pyruvat-Carboxylase an, die sich um eine Länge entsprechend 17 Aminosäuren unterscheiden. Dementsprechend entspricht die Position 153 nach Anspruch 1 von WO 02/31158 der Position 170 von Fig.2A von WO 02/31158, die Position 182 nach Anspruch 1 der Position 199 von Fig.2A, die Position 206 nach Anspruch 1 der Position 223 von Fig. 2A, die Position 227 nach Anspruch 1 der Position 244 von Fig.2A, die Position 452 nach Anspruch 1 der Position 469 von Fig. 2A, die Position 1120 nach Anspruch 1 der Position 1137 von Fig.2B. In Figur 2A von WO 02/31158 ist weiterhin ein Aminosäureaustausch A (Alanin) gegen G (Glycin) an Position 472 angegeben. Die Position 472 des Proteins mit der N-terminalen Sequenz MTA entspricht der Position 455 des Proteins mit der N-terminalen Sequenz MST gemäß Fig. 2A. In Fig. 2B von WO 02/31158 ist weiterhin ein Aminosäureaustausch D (Asparaginsäure) gegen E (Glutaminsäure) an Position 1133 des Proteins mit dem N-Terminus MTA angegeben.),
- ein für eine Aspartatkinase kodierendes lysC Gen wie beispielsweise das als SEQ ID NO:281 in der EP-A-1108790 (Siehe auch Zugangsnummer AX120085 und 120365) und das als SEQ ID NO:25 in der WO 01/00843 (Siehe Zugangsnummer AX063743) beschriebene von lysC-Gen des Wildtyps von Corynebacterium glutamicum
- ein für eine feed-back resistente Aspartatkinase Variante kodierendes lysC^{FBR} Allel, insbesondere entsprechend Tabelle 1,
- ein für ein Lysin-Export-Protein kodierendes Gen lysE, wie beispielsweise das in der DE-A-195 48 222 beschriebene lysE-Gen von des Wildtyps Corynebacterium glutamicum,
- das für das Zwal-Protein kodierende Gen zwal des Wildtyps von Corynebacterium glutamicum (US 6,632,644)
   überexprimiert werden.
   Weiterhin kann es für die Produktion von L-Lysin vorteilhaft sein, neben der Verwendung der erfindungsgemäßen Allele des gnd-Gens gleichzeitig eines oder mehrere der endogenen Gene, ausgewählt aus der Gruppe
- ein für die Glucose-6-Phosphat-Isomerase kodierendes Gen pgi, wie beispielsweise das in der US 6,586,214 und US 6,465,238 beschriebene pgi-Gen von Corynebacterium glutamicum,
- ein für die Fructose-1,6-Bisphosphat Aldolase kodierendes Gen fda, wie beispielsweise das unter der Accession No. X17313 und bei von der Osten et al. (Molecular Microbiology 3 (11), 1625-1637 (1989)) beschriebene fda-Gen von Corynebacterium glutamicum,
- ein für die Homoserin-Dehydrogenase kodierendes Gen hom, wie beispielsweise das in der EP-A-0131171 beschriebene hom-Gen von Corynebacterium glutamicum,
- ein für die Homoserin-Kinase kodierendes Gen thrB, wie beispielsweise das von Peoples et al. (Molecular Microbiology 2 (1988): 63 - 72)) beschriebene thrB-Gen von Corynebacterium glutamicum und
- ein für die Phosphofruktokinase kodierendes Gen pfkB, wie beispielsweise das in der WO 01/00844 (Sequenz Nr. 57) beschriebene pfkB-Gen von Corynebacterium glutamicum,
abzuschwächen oder auszuschalten.

Der Begriff "Abschwächung" beschreibt in diesem Zusammenhang die Verringerung oder Ausschaltung der intrazellulären Aktivität eines oder mehrerer Enzyme (Proteine) in einem Mikroorganismus, die durch die entsprechende DNA kodiert werden, indem man beispielsweise einen schwachen Promotor verwendet oder ein Gen bzw. Allel verwendet, das für ein entsprechendes Enzym mit einer niedrigen Aktivität kodiert bzw. das entsprechende Gen oder Enzym (Protein) inaktiviert und gegebenenfalls diese Maßnahmen kombiniert.

Durch die Maßnahmen der Abschwächung wird die Aktivität oder Konzentration des entsprechenden Proteins im allgemeinen auf 0 bis 75%, 0 bis 50%, 0 bis 25%, 0 bis 10% oder 0 bis 5% der Aktivität oder Konzentration des Wildtyp-Proteins, beziehungsweise der Aktivität oder Konzentration des Proteins im Ausgangs-Mikroorganismus, herabgesenkt.

Als Mutationen zur Erzeugung einer Abschwächung kommen Transitionen, Transversionen, Insertionen und Deletionen von mindestens einem (1) Basenpaar bzw. Nukleotid in Betracht. In Abhängigkeit von der Wirkung des durch die Mutation hervorgerufenen Aminosäureaustausches auf die Enzymaktivität wird von Fehlsinnmutationen ("missense mutations") oder Nichtsinnmutationen ("nonsense mutations") gesprochen. Die Fehlsinnmutation führt zu einem Austausch einer gegebenen Aminosäure in einem Protein gegen eine andere, wobei es sich insbesondere um einen nichtkonservative Aminosäureaustausch handelt. Hierdurch wird die Funktionsfähigkeit bzw. Aktivität des Proteins beeinträchtigt und auf einen Wert von 0 bis 75%, 0 bis 50%, 0 bis 25%, 0 bis 10% oder 0 bis 5% reduziert. Die Nichtsinnmutation führt zu einem Stop-Kodon im Kodierbereich des Gens und damit zu einem vorzeitigen Abbruch der Translation. Insertionen oder Deletionen von mindestens einem Basenpaar in einem Gen führen zu Rasterverschiebungsmutationen ("frame shift mutations"), die dazu führen, dass falsche Aminosäuren eingebaut werden oder die Translation vorzeitig abbricht. Entsteht als Folge der Mutation ein Stop-Kodon im Kodierbereich, so führt dies ebenfalls zu einem vorzeitigen Abbruch der Translation. Deletionen von mindestens einem (1) oder mehreren Kodonen führen typischerweise ebenfalls zu einem vollständigen Ausfall der Enzymaktivität.

Anleitungen zur Erzeugung derartiger Mutationen gehören zum Stand der Technik und können bekannten Lehrbüchern der Genetik und Molekularbiologie wie z.B. dem Lehrbuch von Knippers ("Molekulare Genetik", 6. Auflage, Georg Thieme Verlag, Stuttgart, Deutschland, 1995), dem von Winnacker ("Gene und Klone", VCH Verlagsgesellschaft, Weinheim, Deutschland, 1990) oder dem von Hagemann ("Allgemeine Genetik", Gustav Fischer Verlag, Stuttgart, 1986) entnommen werden. Weitere Maßnahmen sind im Stand der Technik beschrieben.

Die durch die Massnahmen der Erfindung erhaltenen isolierten coryneformen Bakterien zeigen eine im Vergleich zum eingesetzten Ausgangsstamm beziehungsweise Elternstamm erhöhte intrazelluläre Bereitstellung von NADPH und/oder eine erhöhte Ausscheidung beziehungsweise Produktion der gewünschten Aminosäure in einem Fermentationsprozess.

Unter isolierten Bakterien sind die erfindungsgemäßen, isolierten beziehungsweise erzeugten Mutanten und rekombinanten Bakterien, insbesonders coryneformer Bakterien, zu verstehen, welche ein gnd-Allel enthalten, das für eine 6-Phosphogluconat Dehydrogenase kodiert, die den beschriebenen Aminosäureaustausch an Position 329 der Aminosäuresequenz enthält.

Die Leistung der isolierten Bakterien bzw. des Fermentationsprozesses unter Verwendung derselben bezüglich eines oder mehrerer der Parameter ausgewählt aus der Gruppe der Produkt-Konzentration (Produkt pro Volumen), der Produkt-Ausbeute (gebildetes Produkt pro verbrauchter Kohlenstoff-Quelle) und der Produkt-Bildung (gebildetes Produkt pro Volumen und Zeit) oder auch anderer Prozess-Parameter und Kombinationen davon, wird um mindestens 0,5%, mindestens 1%, mindestens 1,5% oder mindestens 2% bezogen auf den Ausgangstamm bzw. Elternstamm bzw. den Fermentationsprozess unter Verwendung derselben verbessert.

Die erfindungsgemäßen, isolierten coryneformen Bakterien können kontinuierlich - wie beispielsweise in der PCT/EP2004/008882 beschrieben- oder diskontinuierlich im batch - Verfahren (Satzkultivierung) oder im fed batch (Zulaufverfahren) oder repeated fed batch Verfahren (repetitives Zulaufverfahren) zum Zwecke der Produktion von L-Aminosäuren kultiviert werden. Eine Zusammenfassung allgemeiner Art über bekannte Kultivierungsmethoden ist im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) verfügbar.

Das zu verwendende Kulturmedium beziehungsweise Fermentationsmedium muß in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten. Die Begriffe Kulturmedium und Fermentationsmedium beziehungsweise Medium sind gegenseitig austauschbar.

Als Kohlenstoffquelle können Zucker und Kohlehydrate wie z.B. Glucose, Saccharose, Laktose, Fructose, Maltose, Melasse, Saccharose-haltige Lösungen aus der Zuckerrübenoder Zuckerrohrherstellung, Stärke, Stärkehydrolysat und Cellulose, Öle und Fette, wie zum Beispiel Sojaöl, Sonnenblumenöl, Erdnußöl und Kokosfett, Fettsäuren, wie zum Beispiel Palmitinsäure, Stearinsäure und Linolsäure, Alkohole wie zum Beispiel Glycerin, Methanol und Ethanol und organische Säuren, wie zum Beispiel Essigsäure verwendet werden. Diese Stoffe können einzeln oder als Mischung verwendet werden.

Als Stickstoffquelle können organische Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium haltigen Salze verwendet werden.

Das Kulturmedium muß weiterhin Salze beispielsweise in Form von Chloriden oder Sulfaten von Metallen wie beispielsweise Natrium, Kalium, Magnesium, Calcium und Eisen enthalten, wie zum Beispiel Magnesiumsulfat oder Eisensulfat, die für das Wachstum notwendig sind. Schließlich können essentielle Wuchsstoffe wie Aminosäuren beispielsweise Homoserin und Vitamine beispielsweise Thiamin, Biotin oder Pantothensäure zusätzlich zu den oben genannten Stoffen eingesetzt werden. Dem Kulturmedium können überdies geeignete Vorstufen der jeweiligen Aminosäure zugesetzt werden.

Die genannten Einsatzstoffe können zur Kultur in Form eines einmaligen Ansatzes hinzugegeben oder in geeigneter Weise während der Kultivierung zugefüttert werden.

Zur pH - Kontrolle der Kultur werden basische Verbindungen wie Natriumhydroxid, Kaliumhydroxid, Ammoniak beziehungsweise Ammoniakwasser oder saure Verbindungen wie Phosphorsäure oder Schwefelsäure in geeigneter Weise eingesetzt. Der pH wird im Allgemeinen auf einen Wert von 6,0 bis 9,0 vorzugsweise 6,5 bis 8 eingestellt. Zur Kontrolle der Schaumentwicklung können Antischaummittel, wie zum Beispiel Fettsäurepolyglykolester eingesetzt werden. Zur Aufrechterhaltung der Stabilität von Plasmiden können dem Medium geeignete selektiv wirkende Stoffe, wie zum Beispiel Antibiotika hinzugefügt werden. Um aerobe Bedingungen aufrechtzuerhalten, werden Sauerstoff oder Sauerstoff-haltige Gasmischungen, wie zum Beispiel Luft in die Kultur eingetragen. Die Verwendung von Flüssigkeiten, die mit Wasserstoffperoxid angereichert sind, ist ebenfalls möglich. Gegebenenfalls wird die Fermentation bei Überdruck, beispielsweise bei einem Druck von 0,03 bis 0,2 MPa, gefahren. Die Temperatur der Kultur liegt normalerweise bei 20°C bis 45°C und vorzugsweise bei 25°C bis 40°C. Bei batch-Verfahren wird die Kultivierung solange fortgesetzt, bis sich ein Maximum der gewünschten Aminosäure gebildet hat. Dieses Ziel wird normalerweise innerhalb von 10 Stunden bis 160 Stunden erreicht. Bei kontinuierlichen Verfahren sind längere Kultivierungszeiten möglich.

Beispiele für geeignete Fermentationsmedien finden sich unter anderem in den Patentschriften 5,770,409, US 5,840,551 und US 5,990,350 oder US 5,275,940.

Methoden zur Bestimmung von L-Aminosäuren sind aus dem Stand der Technik bekannt. Die Analyse kann zum Beispiel so wie bei Spackman et al. (Analytical Chemistry, 30, (1958), 1190) beschrieben durch Anionenaustausch-Chromatographie mit anschließender Ninhydrin-Derivatisierung erfolgen, oder sie kann durch reversed phase HPLC erfolgen, so wie bei Lindroth et al. (Analytical Chemistry (1979) 51: 1167-1174) beschrieben.

Gegenstand der Erfindung ist dementsprechend ein Verfahren zur Herstellung einer L-Aminosäure bei dem man
a) ein isoliertes erfindungsgemäßes coryneformes Bakterium in einem geeignetem Medium fermentiert, wobei
b) die L-Aminosäure in der Fermentationsbrühe oder in den Zellen des isolierten coryneformen Bakteriums anreichert.

Die auf diese Weise hergestellte Fermentationsbrühe wird anschließend zu einem festen oder flüssigen Produkt weiterverarbeitet.

Unter einer Fermentationsbrühe versteht man ein Fermentationsmedium, in dem ein Mikroorganismus für eine gewisse Zeit und bei einer gewissen Temperatur kultiviert wurde. Das Fermentationsmedium beziehungsweise die während der Fermentation eingesetzten Medium enthält/enthalten sämtliche Substanzen beziehungsweise Komponenten, die eine Vermehrung des Mikroorganismus und eine Bildung der gewünschten Aminosäure sicherstellt.

Bei Abschluss der Fermentation enthält die entstandene Fermentationsbrühe dementsprechend a) die infolge der Vermehrung der Zellen des Mikroorganismus entstandene Biomasse des Mikroorganismus, b) die im Laufe der Fermentation gebildete gewünschte Aminosäure, c) die im Laufe der Fermentation gebildeten organischen Nebenprodukte und d) die durch die Fermentation nicht verbrauchten Bestandteile des/der eingesetzten Fermentationsmediums/ Fermentationsmedien beziehungsweise der Einsatzstoffe wie beispielsweise Vitamine wie Biotin, Aminosäuren wie Homoserin oder Salze wie Magnesiumsulfat.

Zu den organischen Nebenprodukte gehören Stoffe, die von den bei der Fermentation eingesetzten Mikroorganismen gegebenenfalls neben der jeweiligen erwünschten L-Aminosäure erzeugt und gegebenenfalls ausgeschieden werden. Hierzu zählen L-Aminosäuren, die im Vergleich zur erwünschten Aminosäure weniger als 30%, 20% oder 10% ausmachen. Hierzu gehören weiterhin organische Säuren, die ein bis drei Carboxyl-Gruppen tragen wie zum Beispiel Essigsäure, Milchsäure, Zitronensäure, Apfelsäure oder Fumarsäure. Schließlich gehören dazu auch Zucker wie zum Beispiel Trehalose.

Typische für industrielle Zwecke geeignete Fermentationsbrühen haben einen Aminosäuregehalt von 40 g/kg bis 180 g/kg oder 50 g/kg bis 150 g/kg. Der Gehalt an Biomasse (als getrocknete Biomasse) beträgt im Allgemeinen 20 bis 50 g/kg.

Im Falle der Aminosäure L-Lysin sind im Stand der Technik im wesentlichen vier verschiedene Produktformen bekannt.

Eine Gruppe L-Lysin haltiger Produkte umfasst konzentrierte, wässrige, alkalische Lösungen von aufgereinigtem L-Lysin (EP-B-0534865). Eine weitere Gruppe, wie beispielsweise in der US 6,340,486 und US 6,465,025 beschrieben, umfasst wässrige, saure, Biomasse-haltige Konzentrate von L-Lysin-haltigen Fermentationsbrühen. Die bekannteste Gruppe fester Produkte umfasst pulverförmige oder kristalline Formen von aufgereinigtem beziehungsweise reinem L-Lysin, das typischerweise in Form eines Salzes wie zum Beispiel L-Lysin-Monohydrochlorid vorliegt. Eine weitere Gruppe fester Produktformen ist beispielsweise in der EP-B-0533039 beschrieben. Die dort beschriebene Produktform enthält neben L-Lysin den größten Teil der während der fermentativen Herstellung verwendeten und nicht verbrauchten Einsatzstoffe und gegebenfalls die Biomasse des eingesetzten Mikroorganismus mit einem Anteil von >0% - 100%.

Entsprechend der verschiedenen Produktformen kennt man verschiedenste Verfahren, bei denen die L-Aminosäure aus der Fermentationsbrühe gesammelt, isoliert oder gereinigt wird, um das L-Aminosäure haltige Produkt oder die gereinigte L-Aminosäure herzustellen.

Zur Herstellung von festen, reinen L-Aminosäuren werden im wesentlichen Methoden der Ionenaustauschchromatographie gegebenfalls unter Verwendung von Aktivkohle und Methoden der Kristallisierung angewendet. Im Falle des Lysin erhält man auf diese Weise die entsprechende Base oder ein entsprechendes Salz wie beispielsweise das Monohydrochlorid (Lys-HCl) oder das Lysinsulfat (Lys₂-H₂SO₄).

Im Falle des Lysin ist in der EP-B-0534865 ein Verfahren zur Herstellung wässriger, basischer L-Lysin haltiger Lösungen aus Fermentationsbrühen beschrieben. Bei dem dort beschriebenen Verfahren wird die Biomasse aus der Fermentationsbrühe abgetrennt und verworfen. Mittels einer Base wie beispielsweise Natrium-, Kalium- oder Ammoniumhydroxid wird ein pH-Wert zwischen 9 bis 11 eingestellt. Die mineralischen Bestandteile (anorganischen Salze) werden nach Konzentrierung und Abkühlung durch Kristallisation aus der Brühe abgetrennt und entweder als Dünger verwendet oder verworfen.

Bei Verfahren zur Herstellung von Lysin unter Verwendung der erfindungsgemäßen Bakterien werden solche Verfahren bevorzugt, bei denen Produkte erhalten werden, die Bestandteile der Fermentationsbrühe enthalten. Diese werden insbesondere als Tierfuttermitteladditive verwendet.

Je nach Anforderung kann die Biomasse ganz oder teilweise durch Separationsmethoden wie z.B. der Zentrifugation, der Filtration, dem Dekantieren oder einer Kombination hieraus aus der Fermentationsbrühe entfernt oder vollständig in ihr belassen werden. Gegebenenfalls wird die Biomasse beziehungsweise die Biomasse enthaltene Fermentationsbrühe während eines geeigneten Verfahrensschrittes inaktiviert beispielsweise durch thermische Behandlung (Erhitzen) oder durch Säurezugabe.

In einer Verfahrensweise wird die Biomasse vollständig oder nahezu vollständig entfernt, sodass keine (0 %) oder höchstens 30%, höchstens 20%, höchstens 10%, höchstens 5%, höchstens 1% oder höchstens 0,1% Biomasse im hergestellten Produkt verbleibt. In einer weiteren Verfahrensweise wird die Biomasse nicht oder nur in geringfügigen Anteilen entfernt, sodass sämtliche (100%) oder mehr als 70%, 80%, 90%, 95%, 99% oder 99,9% Biomasse im hergestellten Produkt verbleibt. In einem erfindungsgemäßen Verfahren wird dementsprechend die Biomasse in Anteilen ≥ 0% bis ≤ 100% entfernt.

Schließlich kann die nach der Fermentation erhaltene Fermentationsbrühe vor oder nach der vollständigen oder teilweisen Entfernung der Biomasse mit einer anorganischen Säure wie beispielsweise Salzsäure, Schwefelsäure oder Phosphorsäure oder organischen Säure wie beispielsweise Propionsäure auf einen sauren pH Wert gestellt werden (GB 1,439,728 oder EP 1 331 220). Es ist gleichfalls möglich die Fermentationsbrühe mit der vollständig enthaltenen Biomasse anzusäuern. Schließlich kann die Brühe auch durch Zusatz von Natriumbisulfit (NaHSO₃, GB 1,439,728) oder einem anderen Salz beispielsweise Ammonium-, Alkali- oder Erdalkalisalz der schwefligen Säure stabilisiert werden.

Bei der Abtrennung der Biomasse werden gegebenenfalls in der Fermentationsbrühe enthaltene organische oder anorganische Feststoffe teilweise oder ganz entfernt. Die in der Fermentationsbrühe gelösten organischen Nebenprodukte und die gelösten nicht verbrauchten Bestandteile des Fermentationsmediums (Einsatzstoffe) bleiben mindestens teilweise (> 0%), bevorzugt zu mindestens 25%, besonders bevorzugt zu mindestens 50% und ganz besonders bevorzugt zu mindestens 75% im Produkt. Gegebenenfalls bleiben diese auch vollständig (100%) oder nahezu vollständig das heißt > 95% oder > 98% im Produkt. In diesem Sinne bedeutet der Begriff "Fermentationsbrühebasis", dass ein Produkt mindestens einen Teil der Bestandteile der Fermentationsbrühe enthält.

Anschließend wird der Brühe mit bekannten Methoden wie z.B. mit Hilfe eines Rotationsverdampfers, Dünnschichtverdampfers, Fallfilmverdampfers, durch Umkehrosmose oder durch Nanofiltration Wasser entzogen beziehungsweise eingedickt oder konzentriert. Diese aufkonzentrierte Fermentationsbrühe kann anschließend durch Methoden der Gefriertrocknung, der Sprühtrocknung, der Sprühgranulation oder durch anderweitige Verfahren wie zum Beispiel in der zirkulierenden Wirbelschicht gemäß PCT/EP2004/006655 beschrieben, zu rieselfähigen Produkten insbesondere zu einem feinteiligen Pulver oder vorzugsweise grobkörnigem Granulat aufgearbeitet werden. Gegebenenfalls wird aus dem erhaltenen Granulat durch Sieben oder Staubabtrennung ein gewünschtes Produkt isoliert.

Es ist ebenfalls möglich, die Fermentationsbrühe direkt d. h. ohne vorherige Aufkonzentrierung durch Sprühtrocknung oder Sprühgranulation zu trocknen.

Unter "rieselfähig" versteht man Pulver, die aus einer Serie von Glasauslaufgefäßen mit verschieden großen Auslauföffnungen mindestens aus dem Gefäß mit der Öffnung 5 mm (Millimeter) ungehindert auslaufen (Klein: Seifen, Öle, Fette, Wachse 94, 12 (1968)).

Mit "feinteilig" ist ein Pulver mit überwiegendem Anteil (> 50 %) einer Korngröße von 20 bis 200 µm Durchmesser gemeint.

Mit "grobkörnig" ist ein Produkt mit einem überwiegendem Anteil (> 50 %) einer Korngröße von 200 bis 2000 µm Durchmesser gemeint.

Die Korngrößenbestimmung kann mit Methoden der Laserbeugungsspektrometrie durchgeführt werden. Die entsprechenden Methoden sind im Lehrbuch zur "Teilchengrößenmessung in der Laborpraxis" von R. H. Müller und R. Schuhmann, Wissenschaftliche Verlagsgesellschaft Stuttgart (1996) oder im Lehrbuch "Introduction to Particle Technology" von M. Rhodes, Verlag Wiley & Sons (1998) beschrieben.

Das rieselfähige, feinteilige Pulver kann wiederum durch geeignete Kompaktier- oder Granulier-Verfahren in ein grobkörniges, gut rieselfähiges, lagerbares und weitgehend staubfreies Produkt überführt werden.

Der Begriff "staubfrei" bedeutet, daß das Produkt lediglich geringe Anteile (< 5 %) an Körngrößen unter 100 µm Durchmesser enthält.

"Lagerbar", im Sinne dieser Erfindung, bedeutet ein Produkt, das mindestens ein (1) Jahr oder länger, bevorzugt mindestens 1,5 Jahre oder länger, besonders bevorzugt zwei (2) Jahre oder länger in trockener und kühler Umgebung gelagert werden kann ohne das ein wesentlicher Verlust (< 5%) der jeweiligen Aminosäure auftritt.

Ein weiterer Gegenstand der Erfindung ist dementsprechend ein Verfahren zur Herstellung eines L-Aminosäure, bevorzugt L-Lysin oder L-Tryptophan, haltigen Produktes, bevorzugt Tierfuttermittel-Additivs, aus Fermentationsbrühen, gekennzeichnet durch die Schritte
a) Kultivierung und Fermentation eines L-Aminosäure ausscheidenden erfindungsgemäßen coryneformen Bakteriums, in einem Fermentationsmedium,
b) Entfernung der während der Fermentation gebildeten Biomasse in einer Menge von 0 bis 100 Gew.-%, und
c) Trocknung der gemäß a) und/oder b) erhaltenen Fermentationsbrühe, um das Produkt in der gewünschten Pulver- oder Granulatform zu erhalten
wobei gegebenenfalls vor Schritt b) oder c) eine Säure ausgewählt aus der Gruppe Schwefelsäure, Phosphorsäure oder Salzsäure hinzugefügt wird.

Vorzugsweise wird im Anschluss an Schritt a) oder b) Wasser aus der L-Aminosäure haltigen Fermentationsbrühe entfernt (Aufkonzentration).

Vorteilhaft bei der Granulation oder Kompaktierung ist der Einsatz von üblichen organischen oder anorganischen Hilfsstoffen, beziehungsweise Trägern wie Stärke, Gelatine, Cellulosederivaten oder ähnlichen Stoffen, wie sie üblicherweise in der Lebensmittel- oder Futterverarbeitung als Binde-, Gelier-, oder Verdickungsmittel Verwendung finden, oder von weiteren Stoffen wie zum Beispiel Kieselsäuren, Silikaten (EP0743016A) Stearaten.

Weiterhin ist es vorteilhaft die Oberfläche der erhaltenen Granulate mit Ölen zu versehen so wie es in der WO 04/054381 beschrieben ist. Als Öle können Mineralöle, pflanzliche Öle oder Mischungen pflanzlicher Öle verwendet werden. Beispiele für derartige Öle sind Sojaöl, Olivenöl, Sojaöl/Lecithingemische. In gleicher Weise sind auch Silikonöle, Polyethylenglykole oder Hydroxyethycellulose geeignet. Durch die Behandlung der Oberflächen mit den genannten Ölen erzielt man eine erhöhte Abriebfestigkeit des Produktes und einen Verringerung des Staubanteils. Der Gehalt an Öl im Produkt beträgt 0,02 bis 2,0 Gew.-%, bevorzugt 0,02 bis 1,0 Gew.-%, und ganz besonders bevorzugt 0,2 bis 1,0 Gew.-% bezogen auf die Gesamtmenge des Futtermitteladditivs.

Bevorzugt sind Produkte mit einem Anteil von ≥ 97 Gew.-% einer Korngröße von 100 bis 1800 µm oder einem Anteil von ≥ 95 Gew.-% einer Korngröße von 300 bis 1800 µm Durchmesser. Der Anteil an Staub d. h. Partikeln mit einer Korngrösse < 100 µm liegt bevorzugt bei > 0 bis 1 Gew.- besonders bevorzugt bei maximal 0,5 Gew.-%.

Alternativ kann das Produkt aber auch auf einen in der Futtermittelverarbeitung bekannten und üblichen organischen oder anorganischen Trägerstoff wie zum Beispiel Kieselsäuren, Silikate, Schrote, Kleien, Mehle, Stärken Zucker oder andere aufgezogen und/oder mit üblichen Verdickungs- oder Bindemitteln vermischt und stabilisiert werden. Anwendungsbeispiele und Verfahren hierzu sind in der Literatur (Die Mühle + Mischfuttertechnik 132 (1995) 49, Seite 817) beschrieben.

Schließlich kann das Produkt auch durch Beschichtungsverfahren ("Coating") mit Filmbildnern wie beispielsweise Metallcarbonate, Kieselsäuren, Silikate, Alginate, Stearate, Stärken, Gummis und Celluloseether, wie in der DE-C-4100920 beschrieben, in einen Zustand gebracht werden, in dem es stabil gegenüber der Verdauung durch Tiermägen insbesondere dem Magen von Wiederkäuern ist.

Zur Einstellung einer gewünschten Aminosäurekonzentration im Produkt kann je nach Anforderung die entsprechende Aminosäure während des Verfahrens in Form eines Konzentrates oder gebenenfalls einer weitgehend reinen Substanz beziehungsweise dessen Salz in flüssiger oder fester Form hinzugefügt werden. Diese können einzeln oder als Mischungen zur erhaltenen oder aufkonzentrierten Fermentationsbrühe, oder auch während des Trocknungs- oder Granulationsprozesses hinzugefügt werden.

Im Falle des Lysin wird bei der Herstellung Lysin-haltiger Produkte das Verhältnis der Ionen so eingestellt, dass das Ionenverhältnis entsprechend nachstehender Formel

2x[SO₄²⁻]+[Cl⁻]-[NH₄⁺]-[Na⁺]-[K⁺]-2x[Mg⁺]-2x[Ca²⁺]/[L-Lys]

0,68 bis 0,95, bevorzugt 0,68 bis 0,90 ergibt so wie von Kushiki et al. in der US 20030152633 beschrieben.

Im Falle des Lysin hat das auf diese Weise hergestellte feste Produkt auf Fermentationsbrühebasis einen Lysingehalt (als Lysinbase) von 10 Gew.-% bis 70 Gew.-% oder 20 Gew.-% bis 70 Gew.-%, bevorzugt 30 Gew.-% bis 70 Gew.-% und ganz besonders bevorzugt von 40 Gew.-% bis 70 Gew.-% bezogen auf die Trockenmasse des Produkts. Maximale Gehalte an Lysinbase von 71 Gew.-% , 72 Gew.-%, 73 Gew.-% sind ebenfalls möglich.

Im Falle einer elektrisch neutralen Aminosäure wie dem L-Tryptophan hat das auf diese Weise hergestellte feste Produkt auf Fermentationsbrühebasis einen Aminosäuregehalt von mindestens 5 Gew.-%, 10 Gew.-%, 20 Gew.-%, 30 Gew.-% und maximal 50 Gew.-%, 60 Gew.-%, 70 Gew.-%, 80 Gew.-%, 90 Gew.-% oder bis 95 Gew.-%.

Der Wassergehalt des festen Produktes beträgt bis zu 5 Gew.-%, bevorzugt bis zu 4 Gew.-%, und besonders bevorzugt weniger als 3 Gew.-%.

Eine Mutante von Corynebacterium glutamicum mit der Bezeichnung DM1797, die den Aminosäureaustausch lysC T311I in der Aspartatkinase enthält, wurde am 28. Oktober 2004 bei der Deutschen Sammlung für Mikroorganismen und Zellkulturen (DSMZ, Braunschweig, Deutschland) als DSM 16833 hinterlegt.

Die erfindungsgemäße Mutante Corynebacterium glutamicum DM1798, die den Aminosäureaustausch gnd V329M enthält, wurde am 28. Oktober 2004 bei der Deutschen Sammlung für Mikroorganismen und Zellkulturen (DSMZ, Braunschweig, Deutschland) als DSM 16834 hinterlegt.

### Beispiel 1

### Mutagenese des L-Lysin produzierenden Stammes DM1797

Der Corynebacterium glutamicum Stamm DM1797 wurde als Ausgangsstamm für die Mutagenese mit N-Methyl-N'-Nitro-N-Nitrosoguanidin (MNNG) eingesetzt. Der Stamm DM1797 ist eine Aminoethylcystein-resistente Mutante von Corynebacterium glutamicum ATCC13032 und unter der Bezeichnung DSM16833 bei der Deutschen Sammlung für Mikroorganismen und Zellkulturen (DSMZ, Braunschweig, Deutschland) hinterlegt.

Der Stamm DM1797 wurde in 10 ml LB-Bouillon (Merck, Darmstadt, Germany), die in einem 100 ml Erlenmeyerkolben enthalten waren, für 24 Stunden bei 33°C und 200 rpm auf einem Rotations-Schüttler vom Typ Certomat BS-1 (B. Braun Biotech International, Melsungen, Deutschland) kultiviert. Anschließend wurde die Kultur abzentrifugiert, das Sediment in 10 ml 0,9% NaCl-Lösung resuspendiert, die erhaltene Suspension erneut abzentrifugiert und das erhaltene Sediment in 10 ml 0,9% NaCl-Lösung aufgenommen. 5 ml dieser Zellsuspension wurden mit 400µg/ml MNNG für 15 Minuten bei 30°C und 200 rpm auf einem Schüttler (siehe oben) behandelt. Anschließend wurde der Mutageneseansatz abzentrifugiert und das Sediment in 10 ml 2% Na-Thiosulfat in 0,9% NaCl-Puffer (pH = 6,0) aufgenommen. Die zellsuspension wurde anschließend im Verhältnis 1:1000, 1:10000 und 1:100000 mit 0,9% NaCl-Lösung verdünnt, und Aliquots auf Hirn-Herz-Agar (Merck, Darmstadt, Deutschland) ausplattiert. Auf diese Weise wurden ungefähr 2500 Mutanten isoliert.

### Beispiel 2

### Leistungstest der Mutanten des Stammes DM1797

Die in Beispiel 1 erhaltenen Mutanten wurden in einem zur Produktion von Lysin geeigneten Nährmedium kultiviert und der Lysingehalt im Kulturüberstand bestimmt.

Dazu wurden die Klone zunächst auf Hirn-Herz-Agarplatten (Merck, Darmstadt, Deutschland) für 24 Stunden bei 33°C vermehrt. Ausgehend von diesen Agarplattenkulturen wurde je eine Vorkultur angeimpft (10 ml Medium im 100 ml Erlenmeyerkolben). Als Medium für die Vorkultur wurde das Medium MM verwendet. Die Vorkultur wurde 24 Stunden bei 33°C und 240 rpm auf einem Schüttler inkubiert. Von dieser Vorkultur wurde eine Hauptkultur angeimpft, so dass die Anfangs-OD (660 nm) der Hauptkultur 0,1 OD betrug. Für die Hauptkultur wurde ebenfalls das Medium MM verwendet.

### Medium MM

| | |
|---|---|
| CSL | 5 g/l |
| MOPS | 20 g/l |
| Glucose (getrennt autoklaviert) | 50 g/l |

| Salze: | |
|---|---|
| (NH₄)₂SO₄) | 25 g/l |
| KH₂PO₄ | 0,1 g/l |
| MgSO₄ * 7 H₂O | 1,0 g/l |
| CaCl₂ * 2 H₂O | 10 mg/l |
| FeSO₄ * 7 H₂O | 10 mg/l |
| MnSO₄ * H₂O | 5,0mg/l |
| Biotin (sterilfiltriert) | 0,3 mg/l |
| Thiamin * HCl (sterilfiltriert) | 0,2 mg/l |
| CaCO₃ | 25 g/l |

CSL (Corn Steep Liquor), MOPS (Morpholinopropansulfonsäure) und die Salzlösung wurden mit Ammoniakwasser auf pH 7 eingestellt und autoklaviert. Anschließend wurden die sterilen Substrat- und Vitaminlösungen sowie das trocken autoklavierte CaCO₃ zugesetzt.

Die Kultivierung erfolgte in Volumina von 10 ml, die in 100 ml Erlenmeyerkolben mit Schikanen enthalten waren. Die Temperatur betrug bei 33°C, die Umdrehungszahl war 250 rpm und die Luftfeuchte 80%.

Nach 24 Stunden wurde die optische Dichte (OD) bei einer Meßwellenlänge von 660 nm mit dem Biomek 1000 (Beckmann Instruments GmbH, München) bestimmt. Die gebildete Lysinmenge wurde mit einem Aminosäureanalysator der Firma Eppendorf-BioTronik (Hamburg, Deutschland) durch Ionenaustauschchromatographie und Nachsäulenderivatisierung mit Ninhydrindetektion bestimmt. Eine Mutante, die sich durch eine erhöhte Lysinbildung auszeichnete, wurde als DM1798 bezeichnet.

**Tabelle 1**

| Stamm | OD(660) | Lysin-HCl (g/l) |
|---|---|---|
| DM1797 | 9,2 | 2,2 |
| DM1798 | 9,2 | 2,4 |

### Beispiel 3

### Sequenzierung des gnd-Gens der Mutante DM1798

Aus dem Klon DM1798 wurde mit der Methode von Eikmanns et al. (Microbiology 140: 1817 - 1828 (1994)) chromosomale DNA isoliert. Mit Hilfe der Polymerase-Kettenreaktion wurde ein DNA-Abschnitt amplifiziert, welcher das gnd-Gen trägt. Hierfür wurden folgende Oligonukleotide als Primer verwendet:
gnd-A1 (SEQ ID NO: 7):
   5' ccataattggcgttgttgag 3'
gnd-E1 (SEQ ID NO: 8):
   5' cgcaaggttattggaacaag 3'

Die dargestellten Primer wurden von der Firma MWG Biotech (Ebersberg, Deutschland) synthetisiert. Sie ermöglichen die Amplifizierung eines ca. 1,85 kb langen DNA-Abschnittes, welcher das gnd-Gen trägt. Der Primer gnd-A1 bindet an die Region entsprechend Position 1 bis 20 des zu SEQ ID NO: 3 komplementären Stranges. Der Primer gnd-E1 bindet an die Region ensprechend Position 1866 bis 1847 des Stranges gemäß SEQ ID NO: 3.

Die PCR-Reaktion wurde mit der Phusion High Fidelity DNA Polymerase (New England Biolabs, Frankfurt, Deutschland) durchgeführt. Der Reaktionsansatz wurde nach Herstellerangaben angesetzt und enthielt bei 50 µl GesamtVolumen 10 l des mitgelieferten 5 x Phusion HF Buffer, Desoxynucleosidtriphosphate in einer Konzentration von jeweils 200 µM, Primer in einer Konzentration von 0,5 µM, ungefähr 50 ng Template-DNA und 2 Units Phusion Polymerase. Durch Zugabe von H₂O wurde das Volumen auf 50 µl eingestellt.

Der PCR-Ansatz wurde zuerst einer einleitenden Denaturierung bei 98°C für 30 Sekunden unterzogen. Darauf folgten sich 35x wiederholend ein Denaturierungsschritt bei 98°C für 20 Sekunden, ein Schritt zum Binden der Primer an die vorgelegte DNA bei 60°C für 20 Sekunden und der Extensionsschritt zur Verlängerung der Primer bei 72°C für 60 Sekunden. Nach dem abschliessenden Extensionsschritt für 5 Minuten bei 72°C wurde der PCR-Ansatz einer Agarosegel-Elektrophorese (0,8% Agarose) unterworfen. Ein DNA-Fragment von ca. 1,85 kb Länge wurde identifiziert, aus dem Gel isoliert und unter Verwendung des QIAquick Gel Extraction Kit der Firma Qiagen, (Hilden, Deutschland) aufgereinigt.

Die Nukleotidsequenz des amplifizierten DNA-Fragmentes bzw. PCR-Produktes wurde von der Firma Agowa (Berlin, Deutschland) bestimmt. Die erhaltene Sequenz der Kodierregion des gnd-Allels ist in der SEQ ID NO: 17 dargestellt. Die sich mit Hilfe des Programmes Patentin ergebende Aminosäuresequenz des dazugehörigen 6-Phosphogluconat Dehydrogenase Proteins ist in der SEQ ID NO: 18 dargestellt.

Die Nukleotidsequenz der Kodierregion des gnd-Allels der Mutante DM1798 enthält an Position 985 die Nukleobase Adenin (Siehe SEQ ID NO: 5). Das Gen des Wildtyps (Siehe SEQ ID NO: 1) enthält an dieser Position die Nukleobase Guanin. Diese Guanin-Adenin Transition führt zu einem Aminosäureaustausch von Valin zu Methionin an Position 329 der resultierenden Aminosäuresequenz. Diese Mutation wird im Folgenden als gndV329M bezeichnet. Weiterhin enthält das gnd-Allel von DM1798 noch fünf weitere Nukleotidaustausche, die nicht zu einem Aminosäureaustausch führen ("stille Mutationen"): eine Guanin-Adenin- Transition an Position 510, eine Cytosin-Guanin-Transversion an Position 612, eine Adenin-Guanin-Transition an Position 786, eine Thymin-Cytosin-Transition an Position 813 und eine Thymin-Guanin-Transversion an Position 1470.

### Beispiel 4

### Konstruktion des Austauschvektors pK18mobsacB_gndV329M

Mit Hilfe der Polymerase-Kettenreaktion wurde ein Teil der Kodierregion, das heisst ein sogenanntes internes Fragment bzw. interner Bereich, des gnd-Allels amplifiziert, der die Mutation gndV329M trägt. Als Template wurde die in Beispiel 3 gewonnene chromosomale DNA verwendet. Folgende Oligonukleotide wurden als Primer für die PCR ausgewählt:
gnd-int1-eco (SEQ ID NO: 20):
   5' ctag-gaattc-ttcgtcggtgctggtatctc 3'
gnd-int2-eco (SEQ ID NO: 21):
   5' ctag-gaattc-gtcgatgcgcttgtaggtgt 3'

Sie wurden von der Firma MWG Biotech (Ebersberg, Deutschland) synthetisiert und ermöglichen die Amplifizierung eines ca. 1 kb langen DNA-Abschnittes der Kodierregion. Die Nukleotide 11 bis 30 des Primers gnd-int1-eco binden an die Region entsprechend Position 629 bis 648 des zu SEQ ID NO:3 koplementären Stranges. Die Positionen 629 und 648 von SEQ ID NO:3 entsprechen den Positionen 403 und 422 in SEQ ID NO:1. Die Nukleotide 11 bis 30 des Primers gnd-int2-eco binden an die Region entsprechend Position 1648 bis 1629 des Stranges gemäß SEQ ID No. 3. Die Position 1648 und 1629 von SEQ ID NO:3 ensprechen den Positionen 1422 und 1403 von SEQ ID NO:1. Außerdem enthalten die Primer die Sequenzen für Schnittstellen der Restriktionsendonuklease EcoRI, die in der oben dargestellten Nukleotidabfolge durch Unterstreichen markiert sind.

Die PCR-Reaktion wurde mit der Phusion High-Fidelity DNA Polymerase (New England Biolabs, Frankfurt, Deutschland) durchgeführt. Der Reaktionsansatz hatte die oben beschriebene Zusammensetzung. Die PCR wurde mit einer Ausnahme wie oben durchgeführt: der 72°C-Extensionsschritt in der 35-fachen Wiederholung wurde jeweils nur für 30 Sekunden durchgeführt.

Das ca. 1 kb lange Amplifikat wurde mit der Restriktionsendonuklease EcoRI behandelt und durch Elektrophorese in einem 0,8%igen Agarosegel identifiziert. Es wurde anschließend aus dem Gel isoliert und mit dem QIAquick Gel Extraction Kit der Firma Qiagen aufgereinigt.

Das dergestalt gereinigte DNA-Fragment enthält die beschrieben gndV329M-Mutation und besitzt EcoRI kompatible Enden (gndV329M-Fragment). Es wurde anschließend in den von Schäfer et al. (Gene, 145, 69-73 (1994) beschriebenen, mobilisierbaren Vektor pK18mobsacB eingebaut, um einen Allel- beziehungsweise Mutationsaustausch zu ermöglichen. Hierzu wurde pK18mobsacB mit dem Restriktionsenzym EcoRI verdaut und die Enden mit alkalischer Phosphatase (Alkaline Phosphatase, Boehringer Mannheim, Deutschland) dephosphoryliert. Der so vorbereitete Vektor wurde mit dem gndV329M-Fragment gemischt und der Ansatz mit dem Ready-To-Go T4 DNA Ligase Kit (Amersham-Pharmacia, Freiburg, Deutschland) behandelt.

Anschließend wurde der E.coli Stamm S17-1 (Simon et al.,Bio/Technologie 1: 784-791, 1993) mit dem Ligationsansatz transformiert (Hanahan, In. DNA cloning. A practical approach. Vol.1. ILR-Press, Cold Spring Habor, New York, 1989). Die Selektion auf Plasmid-tragende Zellen erfolgte durch Ausplattieren des Tansformationsansatzes auf LB-Agar (Sambrock et al., Molecular Cloning: a laboratory manual. 2^{nd} Ed. Cold Spring Habor, New York, 1989), der mit 25 mg/l Kanamycin supplementiert worden war.

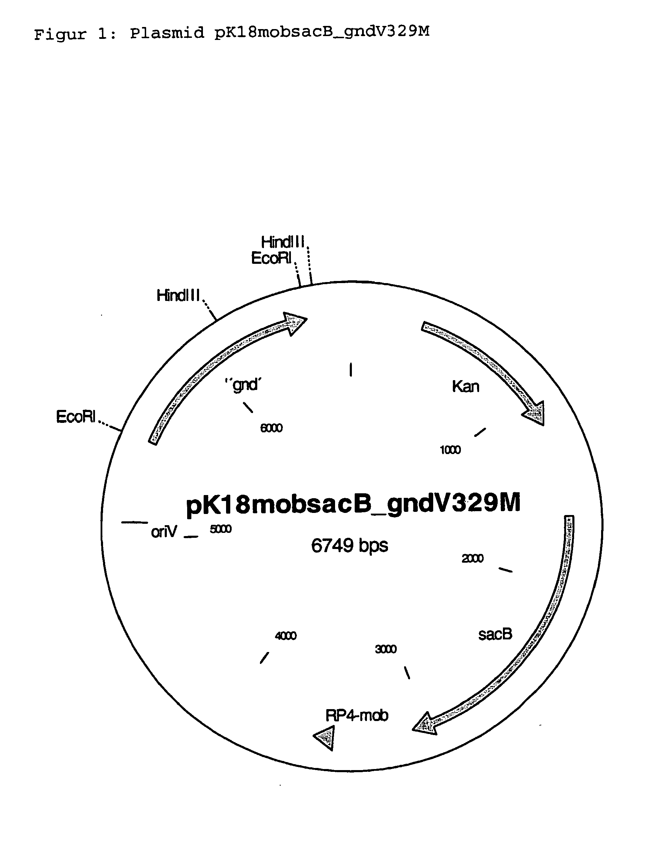

Plasmid-DNA wurde aus einer Transformante mit Hilfe des QIAprep Spin Miniprep Kit der Firma Qiagen isoliert und durch Restriktionsspaltung mit dem Enzym HindIII und anschließender Agarosegel-Elektrophorese überprüft. Das Plasmid wurde pK18mobsacB_gndV329M genannt und ist in Figur 1 dargestellt.

### Beispiel 5

### Einbau der Mutation gndV329M in den Stamm DM1797

Der in Beispiel 4 beschriebene Vektor pK18mobsacB_gndV329M wurde nach dem Protokoll von Schäfer et al. (Journal of Microbiology 172: 1663-1666 (1990)) in den C. glutamicum Stamm DM1797 durch Konjugation transferiert. Der Vektor kann in DM1797 nicht selbständig replizieren und bleibt nur dann in der Zelle erhalten, wenn er als Folge eines Rekombinationsereignisses im Chromosom integriert vorliegt. Die Selektion von Transkonjuganten, d. h. von Klonen mit integriertem pK18mobsacB_gndV329M erfolgte durch Ausplattieren des Konjugationsansatzes auf LB-Agar, der mit 25 mg/l Kanamycin und 50 mg/l Nalidixinsäure supplementiert worden war. Kanamycin-resistente Transkonjuganten wurden anschließend auf mit Kanamycin (25 mg/l) supplementierten LB-Agarplatten ausgestrichen und für 24 Stunden bei 33°C inkubiert. Zur Selektion von Mutanten, bei denen als Folge eines zweiten Rekombinationsereignisses die Exzision des Plasmides stattgefunden hatte, wurden die Klone 30 Stunden unselektiv in LB-Flüssigmedium kultiviert, anschließend auf LB-Agar, der mit 10% Sucrose supplementiert worden war ausgestrichen und 24 Stunden bei 33°C bebrütet.

Das Plasmid pK18mobsacB_gndV329M enthält ebenso wie das Ausgangsplasmid pK18mobsacB neben dem Kanamycin-Resistenzgen eine Kopie des für die Levan-Sucrase aus Bacillus subtilis kodierenden sacB-Gens. Die durch Sucrose induzierbare Expression des sacB-Gens führt zur Bildung der Levan-Sucrase, die die Synthese des für C. glutamicum toxischen Produktes Levan katalysiert. Auf mit Sucrose supplementiertem LB-Agar wachsen daher nur solche Klone, bei denen das integrierte pK18mobsacB_gndV329M als Folge eines zweiten Rekombinationsereignisses exzisiert hat. In Abhängigkeit von der Lage des zweiten Rekombinationsereignisses in bezug auf den Mutationsort findet bei der Exzision der Allelaustausch bzw. der Einbau der Mutation statt oder es verbleibt die ursprüngliche Kopie im Chromosom des Wirtes.

Anschließend wurde ein Klon gesucht, bei dem der gewünschte Austausch, d. h. der Einbau der Mutation gndV329M, erfolgt war. Hierzu wurde von 10 Klonen mit dem Phänotyp "Wachstum in Gegenwart von Sucrose" und "Nicht-Wachstum in Gegenwart von Kanamycin" die Sequenz des gnd-Gens bestimmt. Auf diese Weise wurde ein Klon identifiziert, der die Mutation gndV329M trägt. Dieser Stamm wurde als C. glutamicum DM1797_gndV329M bezeichnet.

### Beispiel 6

### Vergleich der Leistung des Stammes DM1797_gndV329M mit der des Ausgangsstammes DM1797

Der Leistungtest wurde wie in Beispiel 2 beschrieben durchgeführt. Das Ergebnis des Versuchs ist in Tabelle 2 dargestellt.

**Tabelle 2**

| Stamm | OD(660) | Lysin-HCl (g/l) |
|---|---|---|
| DM1797 | 9,3 | 2,1 |
| DM1797_gndV329M | 9,3 | 2,3 |

Kurze Beschreibung der Figur:
Figur 1: Karte des Plasmids pK18mobsacB_gndV329M

Die verwendeten Abkürzungen und Bezeichnungen haben folgende Bedeutung. Bei der Angabe der Basenpaarzahl (bps) handelt es sich um Näherungswerte, die im Rahmen der Reproduzierbarkeit von Messungen erhalten werden.
- Kan:: Kanamycin Resistenz-Gen
- EcoRI:: Schnittstelle des Restriktionsenzyms EcoRI
- HindIII:: Schnittstelle des Restriktionsenzyms HindIII
- 'gnd':: kloniertes DNA-Fragment enthaltend einen internen Bereich des gnd-Allels der Mutante DM1798
- sacB:: sacB-Gen
- RP4-mob:: mob-Region mit dem Replikationsursprung für den Transfer (oriT)
- oriV:: Replikationsursprung V

## Patentansprüche

1. Isolierte Mutanten coryneformer Bakterien, welche ein Gen enthalten, das für ein Polypeptid mit 6-Phosphogluconat Dehydrogenase Aktivität kodiert, **dadurch gekennzeichnet, dass** das Polypeptid eine Aminosäuresequenz umfasst, ausgewählt aus der Gruppe:
a) Aminosäuresequenz, die durch Austausch der Aminosäure an Position 329 von SEQ ID NO: 2 gegen jede proteinogene Aminosäure, ausgenommen L-Valin, entstanden ist, oder
b) Aminosäuresequenz SEQ ID NO: 6, die durch Austausch von L-Valin gegen L-Methionin an der Position 329 in SEQ ID NO: 2 entstanden ist oder
c) Aminosäuresequenz, die zu mindestens 98% identisch ist mit der Aminosäuresequenz gemäß b) und an Position 329 L-Methionin enthält.

2. Mutanten coryneformer Bakterien gemäß Anspruch 1, **dadurch gekennzeichnet, dass** es sich bei den coryneformen Bakterien um ein Bakterium, ausgewählt aus der Gruppe Corynebacterium efficiens, Corynebacterium glutamicum, Corynebacterium thermoaminogenes und Corynebacterium aminogenes handelt.

3. Mutanten coryneformer Bakterien gemäß Anspruch 2, **dadurch gekennzeichnet, dass** es sich um Corynebacterium glutamicum handelt.

4. Mutanten coryneformer Bakterien gemäß Anspruch 1, 2, oder 3, **dadurch gekennzeichnet, dass** es sich um L-Aminosäure ausscheidende Bakterien handelt.

5. Mutanten coryneformer Bakterien gemäß Anspruch 4, **dadurch gekennzeichnet, dass** es sich um L-Lysin oder um L-Tryptophan ausscheidende Bakterien handelt.

6. Mutanten coryneformer Bakterien gemäß Anspruch 1 bis 5 **dadurch gekennzeichnet, dass** das Polypeptid eine Aminosäuresequenz mit einer Länge von 492 L-Aminosäuren umfasst.

7. Mutanten coryneformer Bakterien gemäß Anspruch 1, **dadurch gekennzeichnet, dass** das Gen die Nukleotidsequenz von SEQ ID NO:5 umfasst, oder SEQ ID NO:5, wobei diese mindestens einen oder mehrere der im folgenden aufgelisteten Austausche aufweist: an der Position 93 Guanin anstelle Cytosin, an der Position 375 Adenin anstelle Guanin, an der Position 510 Adenin anstelle Guanin, an der Position 612 Guanin anstelle Cytosin, an der Position 786 Guanin anstelle Adenin, an der Position 813 Cytosin anstelle Thymin, an der Position 1014 Guanin anstelle Thymin, an der Position 1380 Cytosin anstelle Adenin, an der Position 1470 Guanin anstelle Thymin.

8. Mutanten coryneformer Bakterien gemäß Anspruch 6, **dadurch gekennzeichnet, dass** das Gen die Nukleotidsequenz von SEQ ID NO:17 umfasst.

9. Isoliertes Polynukleotid, das für ein Polypeptid mit 6-Phosphogluconat Dehydrogenase Aktivität kodiert, **dadurch gekennzeichnet, dass** das Polypeptid eine Aminosäuresequenz umfasst, ausgewählt aus der Gruppe:
a) Aminosäuresequenz, die durch Austausch der Aminosäure an Position 329 von SEQ ID NO: 2 gegen jede proteinogene Aminosäure, ausgenommen L-Valin, entstanden ist, oder
b) Aminosäuresequenz SEQ ID NO: 6, die durch Austausch von L-Valin gegen L-Methionin an der Postion 329 in SEQ ID NO: 2 entstanden ist, oder
c) Aminosäuresequenz, die zu mindestens 98% identisch ist mit der Aminosäuresequenz gemäß b) und an Position 329 L-Methionin enthält.

10. Isoliertes Polynukleotid gemäß Anspruch 9, **dadurch gekennzeichnet, dass** es das kodierte Polypeptid einer Aminosäuresequenz mit einer Länge von 492 L-Aminosäuren umfasst.

11. Isoliertes Polynukleotid gemäß Anspruch 9, **dadurch gekennzeichnet, dass** die Nukleotidsequenz SEQ ID NO:5 umfasst, oder SEQ ID NO:5, wobei diese einen oder mehreren der Basenaustausche umfasst, ausgewählt aus der Gruppe: an der Position 93 Guanin anstelle Cytosin, an der Position 375 Adenin anstelle Guanin, an der Position 510 Adenin anstelle Guanin, an der Position 612 Guanin anstelle Cytosin, an der Position 786 Guanin anstelle Adenin, an der Position 813 Cytosin anstelle Thymin, an der Position 1014 Guanin anstelle Thymin, an der Position 1380 Cytosin anstelle Adenin, an der Position 1470 Guanin anstelle Thymin.

12. Isoliertes Polynukleotid gemäß Anspruch 10, **dadurch gekennzeichnet, dass** die Nukleotidsequenz SEQ ID NO:17 umfasst.

13. Ein isoliertes Polynukleotid, welches ein Nukleinsäure-Molekül umfasst, das mindestens für ein Leseraster mit einer Aminosäuresequenz entsprechend Position 313 bis 345 von SEQ ID NO:2 kodiert, wobei an der Position entsprechend 329 von SEQ ID NO:2 jede proteinogene Aminosäure ausgenommen L-Valin enthalten ist.

14. Isoliertes Polynukleotid gemäß Anspruch 13, **dadurch gekennzeichnet, dass** es mindestens für ein Leseraster mit einer Aminosäuresequenz entsprechend Position 313 bis 345 von SEQ ID NO:6 kodiert.

15. Isoliertes Polynukleotid gemäß Anspruch 14, **dadurch gekennzeichnet, dass** es mindestens die Nukleotidsequenz entsprechend Position 937 bis 1035 von SEQ ID NO:5 oder von SEQ ID NO:17 umfasst.

16. Ein rekombinanter Mikroorganismus, der das isolierte Polynukleotid gemäß Anspruch 9 bis 12 oder 13 bis 15 enthält.

17. Rekombinanter Mikroorganismus gemäß Anspruch 16, **dadurch gekennzeichnet, dass** es sich um ein coryneformes Bakterium oder um ein Bakterium der Gattung Escherichia handelt.

18. Rekombinanter Mikroorganismus gemäß Anspruch 17, **dadurch gekennzeichnet, dass** es sich bei dem coryneformen Bakterium um die Gattung Corynebacterium handelt.

19. Rekombinanter Mikroorganismus gemäß Anspruch 18, **dadurch gekennzeichnet, dass** es sich bei dem Bakterium der Gattung Corynebacterium um die Art Corynebacterium glutamicum handelt.

20. Ein Vektor, der das isolierte Polynukleotid gemäß Anspruch 9 bis 12 oder 13 bis 15 enthält.

21. Ein rekombinanter Mikroorganismus gemäß Anspruch 19, der den Vektor gemäß Anspruch 20 enthält.

22. Rekombinanter Mikroorganismus, gemäß Anspruch 21, **dadurch gekennzeichnet, dass** es sich um ein coryneformes Bakterium oder um ein Bakterium der Gattung Escherichia handelt.

23. Rekombinanter Mikroorganismus gemäß Anspruch 22, **dadurch gekennzeichnet, dass** es sich bei dem coryneformen Bakterium um die Gattung Corynebacterium handelt.

24. Rekombinanter Mikroorganismus gemäß Anspruch 23, **dadurch gekennzeichnet, dass** es sich bei dem Bakterium der Gattung Corynebacterium um die Art Corynebacterium glutamicum handelt.

25. Mikroorganismus gemäß den Ansprüchen 1-8, 16-19 und 21-24, in denen das Gen, das für ein Polypeptid mit 6-Phosphogluconat Dehydrogenase Aktivität kodiert und durch die Ansprüche 9-12 definiert ist, überexprimiert vorliegt, wobei zur Erzielung der Überexpression dieses Gen in funktioneller Weise mit einem Promoter verknüpft ist.

26. Verfahren zur Herstellung einer L-Aminosäure **dadurch gekennzeichnet, dass** man
a) ein isoliertes coryneformes Bakterium ausgewählt aus der Gruppe gemäß den Ansprüchen 1-8, 16-19 und 21-25 in einem geeignetem Medium fermentiert, und
b) die L-Aminosäure in der Fermentationsbrühe oder in den Zellen des Bakteriums anreichert.

27. Verfahren nach Anspruch 26, **dadurch gekennzeichnet, dass** man die L-Aminosäure isoliert oder sammelt

28. Verfahren nach Anspruch 26, **dadurch gekennzeichnet, dass** man die L-Aminosäure reinigt.

29. Verfahren nach Anspruch 26, **dadurch gekennzeichnet, dass** man die L-Aminosäure gemeinsam mit Bestandteilen aus der Fermentationsbrühe und/oder > 0 bis 100 % der Biomasse isoliert oder sammelt.

30. Verfahren nach den Ansprüchen 26 und 29, **dadurch gekennzeichnet, dass** man
a) aus der in Schritt b) von Anspruch 26 erhaltenen Fermentationsbrühe die gebildete Biomasse in einer Menge von 0 bis 100 % entfernt, und
b) aus der in Schritt a) erhaltenen Brühe ein im wesentlichen trockenes und geformtes Produkt durch eine Methode ausgewählt aus der Gruppe Granulation, Kompaktierung, Sprühtrocknung und Extrusion herstellt.

31. Verfahren nach Anspruch 30, **dadurch gekennzeichnet, dass** man L-Lysin herstellt und der Fermentationsbrühe vor oder nach Schritt a) eine Säure ausgewählt aus der Gruppe Schwefelsäure, Salzsäure und Phosphorsäure hinzufügt.

32. Verfahren nach Anspruch 30, **dadurch gekennzeichnet, dass** man aus der vor oder nach in Schritt a) erhaltenen Brühe Wasser entfernt.

33. Verfahren gemäß Schritt 30, **dadurch gekennzeichnet, dass** man das in oder während Schritt b) erhaltene geformte Produkt mit einem Öl besprüht.

## Claims

1. Isolated mutant coryneform bacteria which contain a gene that encodes a polypeptide having 6-phosphogluconate dehydrogenase activity, **characterized in that** the polypeptide comprises an amino acid sequence selected from the group:
a) amino acid sequence which has resulted by exchanging the amino acid at position 329 in SEQ ID NO:2 for any proteinogenic amino acid, except for L-valine, or
b) amino acid sequence SEQ ID NO: 6, which has resulted by exchanging L-valine for L-methionine at position 329 in SEQ ID NO:2, or
c) amino acid sequence which is at least 98% identical to the amino acid sequence according to b) and contains L-methionine at position 329.

2. Mutant coryneform bacteria according to Claim 1, **characterized in that** the coryneform bacteria are a bacterium selected from the group Corynebacterium efficiens, Corynebacterium glutamicum, Corynebacterium thermoaminogenes and Corynebacterium aminogenes.

3. Mutant coryneform bacteria according to Claim 2, **characterized in that** they are Corynebacterium glutamicum.

4. Mutant coryneform bacteria according to Claim 1, 2, or 3, **characterized in that** they are L-amino acid-excreting bacteria.

5. Mutant coryneform bacteria according to Claim 4, **characterized in that** they are L-lysine- or L-tryptophan-excreting bacteria.

6. Mutant coryneform bacteria according to Claim 1 to 5, **characterized in that** the polypeptide comprises an amino acid sequence having a length of 492 L-amino acids.

7. Mutant coryneform bacteria according to Claim 1, **characterized in that** the gene comprises the nucleotide sequence of SEQ ID NO:5, or SEQ ID NO:5 comprising at least one or more of the following listed exchanges: at position 93, guanine instead of cytosine, at position 375, adenine instead of guanine, at position 510 adenine instead of guanine, at position 612, guanine instead of cytosine, at position 786, guanine instead of adenine, at position 813, cytosine instead of thymine, at position 1014, guanine instead of thymine, at position 1380, cytosine instead of adenine, at position 1470, guanine instead of thymine.

8. Mutant coryneform bacteria according to Claim 6, **characterized in that** the gene comprises the nucleotide sequence of SEQ ID No:17.

9. Isolated polynucleotide that encodes a polypeptide having 6-phosphogluconate dehydrogenase activity, **characterized in that** the polypeptide comprises an amino acid sequence selected from the group:
a) amino acid sequence which has resulted by exchanging the amino acid at position 329 in SEQ ID NO:2 for any proteinogenic amino acid, except for L-valine, or
b) amino acid sequence SEQ ID NO: 6, which has resulted by exchanging L-valine for L-methionine at position 329 in SEQ ID NO:2, or
c) amino acid sequence which is at least 98% identical to the amino acid sequence according to b) and contains L-methionine at position 329.

10. Isolated polynucleotide according to Claim 9, **characterized in that** it comprises the encoded polypeptide of an amino acid sequence having a length of 492 L-amino acids.

11. Isolated polynucleotide according to Claim 9, **characterized in that** it comprises the nucleotide sequence SEQ ID NO:5, or SEQ ID NO:5 comprising one or more of the base exchanges selected from the group: at position 93, guanine instead of cytosine, at position 375, adenine instead of guanine, at position 510 adenine instead of guanine, at position 612, guanine instead of cytosine, at position 786, guanine instead of adenine, at position 813, cytosine instead of thymine, at position 1014, guanine instead of thymine, at position 1380, cytosine instead of adenine, at position 1470, guanine instead of thymine.

12. Isolated polynucleotide according to Claim 10, **characterized in that** it comprises the nucleotide sequence SEQ ID NO:17.

13. Isolated polynucleotide which comprises a nucleic acid molecule that encodes at least one reading frame having an amino acid sequence corresponding to position 313 to 345 of SEQ ID NO:2, wherein at the position corresponding to 329 of SEQ ID NO:2, any proteinogenic amino acid except for L-valine is present.

14. Isolated polynucleotide according to Claim 13, **characterized in that** it encodes at least one reading frame having an amino acid sequence corresponding to position 313 to 345 of SEQ ID NO:6.

15. Isolated polynucleotide according to Claim 14, **characterized in that** it comprises at least the nucleotide sequence corresponding to position 937 to 1035 of SEQ ID NO:5 or of SEQ ID NO:17.

16. A recombinant microorganism which contains the isolated polynucleotide according to Claim 9 to 12 or 13 to 15.

17. Recombinant microorganism according to Claim 16, **characterized in that** it is a coryneform bacterium or a bacterium of the genus Escherichia.

18. Recombinant microorganism according to Claim 17, **characterized in that** the coryneform bacterium is of the genus Corynebacterium.

19. Recombinant microorganism according to Claim 18, **characterized in that** the bacterium of the genus Corynebacterium is the species Corynebacterium glutamicum.

20. Vector which contains the isolated polynucleotide according to Claim 9 to 12 or 13 to 15.

21. Recombinant microorganism according to Claim 19, which contains the vector according to Claim 20.

22. Recombinant microorganism according to Claim 21, **characterized in that** it is a coryneform bacterium or a bacterium of the genus Escherichia.

23. Recombinant microorganism according to Claim 22, **characterized in that** the coryneform bacterium is of the genus Corynebacterium.

24. Recombinant microorganism according to Claim 23, **characterized in that** the bacterium of the genus Corynebacterium is of the species Corynebacterium glutamicum.

25. Microorganism according to Claims 1-8, 16-19 and 21-24, in which the gene which encodes a polypeptide having 6-phosphogluconate dehydrogenase activity and is defined by Claims 9-12 is present in overexpressed form, wherein this gene is functionally linked to a promoter to achieve the overexpression.

26. Method for producing an L-amino acid, **characterized in that**
a) an isolated coryneform bacterium selected from the group according to Claims 1-8, 16-19 and 21-25 is fermented in a suitable medium, and
b) the L-amino acid is enriched in the fermentation broth or in the cells of the bacterium.

27. Method according to Claim 26, **characterized in that** the L-amino acid is isolated or collected.

28. Method according to Claim 26, **characterized in that** the L-amino acid is purified.

29. Method according to Claim 26, **characterized in that** the L-amino acid is isolated or collected together with components of the fermentation broth and/or > 0 to 100% of the biomass.

30. Method according to Claims 26 and 29, **characterized in that**
a) from the fermentation broth obtained in step b) of Claim 26, the biomass formed is removed in an amount of 0 to 100%, and
b) from the broth obtained in step a), an essentially dry and shaped product is produced by a method selected from the group granulation, compacting, spray drying and extrusion.

31. Method according to Claim 30, **characterized in that** L-lysine is produced and, to the fermentation broth before or after step a), an acid selected from the group sulphuric acid, hydrochloric acid and phosphoric acid is added.

32. Method according to Claim 30, **characterized in that** water is removed from the broth obtained before or after in step a).

33. Method according to Claim 30, **characterized in that** the shaped product obtained in or during step b) is sprayed with an oil.

## Revendications

1. Mutants isolés de bactéries corynéformes, qui contiennent un gène qui code pour un polypeptide ayant une activité de 6-phosphogluconate déshydrogénase, **caractérisés en ce que** le polypeptide comprend une séquence d'acides aminés choisie dans le groupe :
a) séquence d'acides aminés qui a été obtenue par remplacement de l'acide aminé en position 329 de SEQ ID N° 2 par chaque acide aminé protéinogène, à l'exception de la L-valine, ou
b) séquence d'acides aminés SEQ ID N° 6, qui a été obtenue par remplacement de la L-valine par la L-méthionine en position 329 de SEQ ID N° 2, ou
c) séquence d'acides aminés qui a une identité d'au moins 98 % avec la séquence d'acides aminés selon b), et contient une L-méthionine en position 329.

2. Mutants de bactéries corynéformes selon la revendication 1, **caractérisés en ce que**, pour ce qui concerne les bactéries corynéformes, il s'agit d'une bactérie choisie dans le groupe consistant en *Corynebacterium efficiens, Corynebacterium glutamicum, Corynebacterium thermoaminogenes* et *Corynebacterium aminogenes.*

3. Mutants de bactéries corynéformes selon la revendication 2, **caractérisés en ce qu'**il s'agit de *Corynebacterium glutamicum.*

4. Mutants de bactéries corynéformes selon la revendication 1, 2 ou 3, **caractérisés en ce qu'**il s'agit de bactéries qui secrètent un acide L-aminé.

5. Mutants de bactéries corynéformes selon la revendication 4, **caractérisés en ce qu'**il s'agit de bactéries qui secrètent de la L-lysine ou du L-tryptophane.

6. Mutants de bactéries corynéformes selon les revendications 1 à 5, **caractérisés en ce que** le polypeptide comprend une séquence d'acides aminés ayant une longueur de 492 acides L-aminés.

7. Mutants de bactéries corynéformes selon la revendication 1, **caractérisés en ce que** le gène comprend la séquence nucléotidique de SEQ ID N° 5 ou SEQ ID N° 5, cette dernière comprenant au moins un ou plusieurs des remplacements mentionnés ci-après : en position 93, une guanine au lieu d'une cytosine ; en position 375, une adénine au lieu d'une guanine ; en position 510, une adénine au lieu d'une guanine ; en position 612, une guanine au lieu d'une cytosine ; en position 786, une guanine au lieu d'une adénine ; en position 813, une cytosine au lieu d'une thymine ; en position 1014, une guanine au lieu d'une thymine ; en position 1380, une cytosine au lieu d'une adénine ; en position 1470, une guanine au lieu d'une thymine.

8. Mutants de bactéries corynéformes selon la revendication 6, **caractérisés en ce que** le gène comprend la séquence nucléotidique de SEQ ID N° 17.

9. Polynucléotide isolé, qui code pour un polypeptide ayant une activité de 6-phosphogluconate déshydrogénase, **caractérisé en ce que** le polypeptide comprend une séquence d'acides aminés choisie dans le groupe :
a) séquence d'acides aminés qui a été obtenue par remplacement de l'acide aminé en position 329 de SEQ ID N° 2 par chaque acide aminé protéinogène, à l'exception de la L-valine, ou
b) séquence d'acides aminés SEQ ID N° 6, qui a été obtenue par remplacement de la L-valine par la L-méthionine en position 329 de SEQ ID N° 2, ou
c) séquence d'acides aminés qui présente une identité d'au moins 98 % avec la séquence d'acides aminés selon b), et contient en position 329 une L-méthionine.

10. Polynucléotide isolé selon la revendication 9, **caractérisé en ce qu'**il comprend le polypeptide codé d'une séquence d'acides aminés ayant une longueur de 492 acides L-aminés.

11. Polynucléotide isolé selon la revendication 9, **caractérisé en ce qu'**il comprend la séquence nucléotidique SEQ ID N° 5, ou SEQ ID N° 5, cette dernière comprenant un ou plusieurs remplacements de bases choisies dans le groupe : en position 93, une guanine au lieu d'une cytosine ; en position 375, une adénine au lieu d'une guanine ; en position 510, une adénine au lieu d'une guanine ; en position 612, une guanine au lieu d'une cytosine ; en position 786, une guanine au lieu d'une adénine ; en position 813, une cytosine au lieu d'une thymine ; en position 1014, une guanine au lieu d'une thymine ; en position 1380, une cytosine au lieu d'une adénine ; en position 1470, une guanine au lieu d'une thymine.

12. Polynucléotide isolé selon la revendication 10, **caractérisé en ce qu'**il comprend la séquence nucléotidique SEQ ID N° 17.

13. Polynucléotide isolé, qui comprend une molécule d'acide nucléique qui code pour au moins un cadre de lecture ayant une séquence d'acides aminés correspondant aux positions 313 à 345 de SEQ ID N° 2, chaque acide aminé protéinogène, à l'exception de la L-valine, étant présent sur la position correspondant à 329 de SEQ ID N° 2.

14. Polynucléotide isolé selon la revendication 13, **caractérisé en ce qu'**il code pour au moins un cadre de lecture ayant une séquence d'acides aminés correspondant aux positions 313 à 345 de SEQ ID N° 6.

15. Polynucléotide isolé selon la revendication 14, **caractérisé en ce qu'**il comprend au moins la séquence nucléotidique correspondant aux positions 937 à 1035 de SEQ ID N° 5 ou de SEQ ID N° 17.

16. Micro-organisme recombinant, qui contient le polynucléotide isolé selon les revendications 9 à 12 ou 13 à 15.

17. Micro-organisme recombinant selon la revendication 16, **caractérisé en ce que**, pour ce qui concerne une bactérie corynéforme, il s'agit d'une bactérie du genre Escherichia.

18. Micro-organisme recombinant selon la revendication 17, **caractérisé en ce que,** pour ce qui concerne la bactérie corynéforme, il s'agit du genre Corynebacterium.

19. Micro-organisme recombinant selon la revendication 18, **caractérisé en ce que**, pour ce qui concerne la bactérie du genre Corynebacterium, il s'agit de l'espèce *Corynebacterium glutamicum*.

20. Vecteur qui contient le polynucléotide isolé selon les revendications 9 à 12 ou 13 à 15.

21. Micro-organisme recombinant selon la revendication 19, qui contient le vecteur selon la revendication 20.

22. Micro-organisme recombinant selon la revendication 21, **caractérisé en ce que**, pour ce qui concerne une bactérie corynéforme, il s'agit d'une bactérie du genre Escherichia.

23. Micro-organisme recombinant selon la revendication 22, **caractérisé en ce que**, pour ce qui concerne la bactérie corynéforme, il s'agit du genre Corynebacterium.

24. Micro-organisme recombinant selon la revendication 23, **caractérisé en ce que**, pour ce qui concerne la bactérie du genre Corynebacterium, il s'agit de l'espèce *Corynebacterium glutamicum.*

25. Micro-organisme selon les revendications 1-8, 16-19 et 21-24, dans lequel le gène qui code pour un polypeptide ayant une activité de 6-phosphogluconate déshydrogénase et qui est défini par les revendications 9 à 12, est présent à l'état surexprimé, ce gène étant, pour atteindre la surexpression, lié d'une manière fonctionnelle à un promoteur.

26. Procédé de préparation d'un acide L-aminé, **caractérisé en ce que**
a) on fermente dans un milieu approprié une bactérie corynéforme isolée choisie dans le groupe selon les revendications 1-8, 16-19 et 21-25, et
b) on enrichit l'acide L-aminé, dans le bouillon de fermentation ou dans les cellules de la bactérie.

27. Procédé selon la revendication 26, **caractérisé en ce qu'**on isole ou recueille l'acide L-aminé.

28. Procédé selon la revendication 26, **caractérisé en ce qu'**on purifie l'acide L-aminé.

29. Procédé selon la revendication 26, **caractérisé en ce qu'**on isole ou recueille l'acide L-aminé en même temps que des constituants du bouillon de fermentation et/ou > 0 à 100 % de la biomasse.

30. Procédé selon les revendications 26 et 29, **caractérisé en ce que**
a) on élimine du bouillon de fermentation obtenu dans l'étape b) de la revendication 26 la biomasse formée, en une quantité de 0 à 100 %, et
b) à partir du bouillon obtenu dans l'étape a), on prépare un produit pour l'essentiel sec et façonné par un procédé choisi dans le groupe consistant en une granulation, un compactage, une lyophilisation et une extrusion.

31. Procédé selon la revendication 30, **caractérisé en ce qu'**on prépare de la L-lysine, et qu'on ajoute au bouillon de fermentation, avant ou après l'étape a), un acide choisi dans le groupe consistant en l'acide sulfurique, l'acide chlorhydrique et l'acide phosphorique.

32. Procédé selon la revendication 30, **caractérisé en ce qu'**on élimine l'eau du bouillon obtenu avant ou après l'étape a).

33. Procédé selon la revendication 30, **caractérisé en ce qu'**on pulvérise une huile sur le produit façonné obtenu dans ou pendant l'étape b).
